# EUROPEAN PATENT APPLICATION

(11) **EP 2 628 727 A2**
(43) Date of publication of application: **21.08.2013**
(21) Application number: 13000709.9
(22) Date of filing: 20.11.2008
(51) Int. Cl.: C07D 207/16, C07D 213/64, C07D 213/79, C07D 231/12, C07D 239/42, C07D 401/06, C07D 471/04, C07D 473/18, C07D 473/40, C07D 285/135, C07D 213/06, C07D 213/74, C07D 213/75, C07D 213/84, C07D 401/04, C07D 233/90, C07D 275/06

(54) **Biaryl PDE4 inhibitors for treating pulmonary and cardiovascular disorders**

(30) Priority: 21.11.2007 US 989551 P
(62) Divisional of application: 08852110.9
(71) Applicant: Decode Genetics EHF, 101 Reykjavik (IS)
(72) Inventor: Singh, Jasbir, 60564 Naperville, IL (US); Gurney, Mark, 49506 Grand Rapids, MI (US); Burgin, Alex, 98346 Kingston, WA (US); Kiselyov, Alexander, 92130 San Diego (US); Rao, Munagala, 60559 Westmont, IL (US); Hagen, Timothy, 60532 Lisle, IL (US)
(74) Representative: Graf von Stosch, Andreas

(57) **Abstract**

The present invention relates to a genus of biaryl compounds containing at least one further ring. The compounds are PDE4 inhibitors useful for the treatment and prevention of stroke, myocardial infarct and cardiovascular inflammatory diseases and disorders. The compounds have general formula Ia, Ib, Ic or Id: 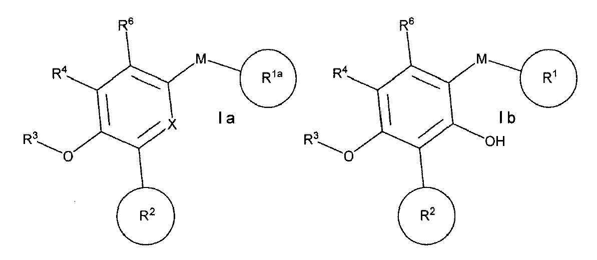 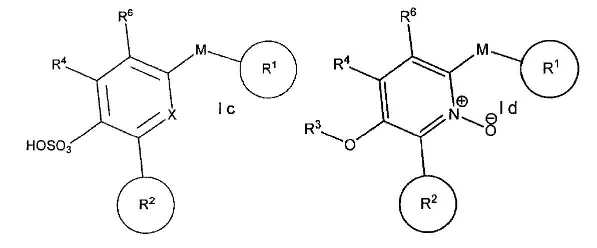 or salt thereof, in particular a pharmaceutically acceptable salt thereof.

## Description

### Field of the Invention

The present invention relates to a chemical genus of biaryl inhibitors of phosphodiesterase-4 (PDE4) useful for the treatment and prevention of stroke, myocardial infarct and cardiovascular inflammatory diseases and disorders.

### Background of the Invention

PDE4 is the major cAMP-metabolizing enzyme found in inflammatory and immune cells. PDE4 inhibitors have proven potential as anti-inflammatory drugs, especially in inflammatory pulmonary diseases such as asthma, COPD and rhinitis. They suppress the release of cytokines and other inflammatory signals and inhibit the production of reactive oxygen species. A large number of PDE4 inhibitors have been developed for a variety of clinical indications (Torphy and Page. 2000. TIPS 21, 157-159; Burnouf and Pruniaux. 2002. Curr. Pharm. Design 8, 1255-1296; Lipworth. 2005. Lancet 365, 167-175). To quote from a recent article in the British Journal of Pharmacology, "PDE4 inhibitors have been in development as a novel anti-inflammatory therapy since the 1980s with asthma and chronic obstructive pulmonary disease (COPD) being primary indications. Despite initial optimism, none have yet reached the market. In most cases, the development of PDE4 inhibitors of various structural classes, including cilomilast, filaminast, lirimilast, piclamilast, tofimilast..... has been discontinued due to lack of efficacy. A primary problem is the low therapeutic ratio of these compounds, which severely limits the dose that can be given. Indeed, for many of these compounds it is likely that the maximum tolerated dose is either sub-therapeutic or at the very bottom of the efficacy dose-response curve. Therefore, the challenge is to overcome this limitation." [Giembycz, Brit.J.Pharmacol. 155, 288-290 (2008)]. Many of the PDE4 inhibitors of the prior art have not reached the market because of the adverse side effect of emesis (Giembycz 2005. Curr. Opin. Pharm. 5, 238-244). Analysis of all known PDE4 inhibitors suggests that they are competitive with cAMP and bind within the active site (Houslay et al. 2005. DDT 10, 1503-1519); this may explain their narrow therapeutic ratio. The compounds of the present invention are non-competitive inhibitors of cAMP while being gene-specific inhibitors (PDE4D), and, based on the target rationale and in vitro potency, a person of skill in the art would expect the compounds to be useful as anti-inflammatory agents for the treatment, amelioration or prevention of inflammatory diseases and of complications arising therefrom.

### Summary of the Invention

The present invention relates to compounds exhibiting PDE4 enzyme inhibition, having the general formula Ia, Ib, Ic or Id:

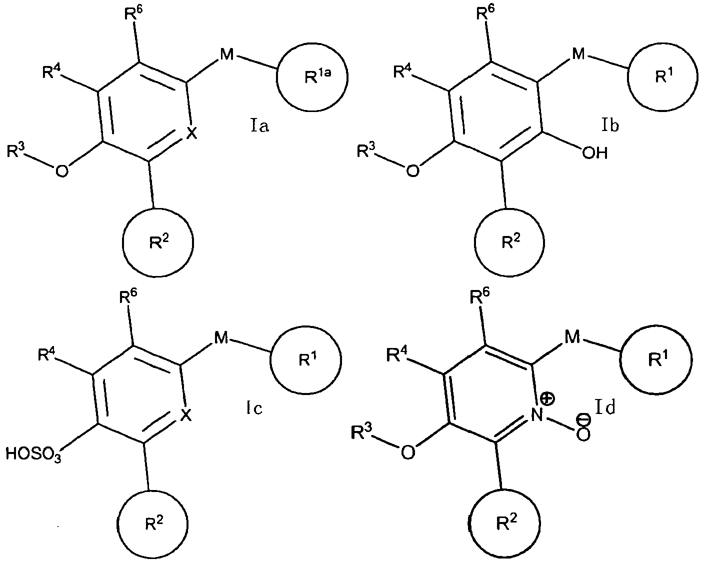

In these compounds,
R¹ is an optionally substituted carbocycle or optionally substituted heterocycle of three or fewer rings;
R^{1a} is is chosen from
   (a) a residue chosen from 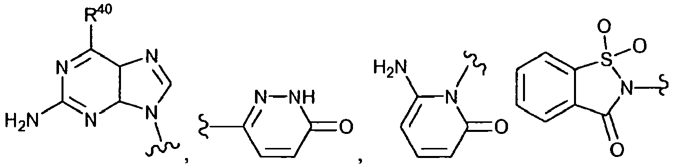 and 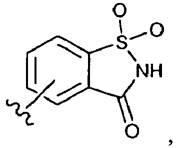 wherein R⁴⁰ is chosen from H, halogen, OH, NH₂ and CH₃;
   (b) a substituted heterocycle of three or fewer rings or substituted carbocycle of three or fewer rings; and
   (c) a heterocycle that is itself substituted with a heterocycle carrying a further substituent;
wherein substituents on the heterocycle or carbocycle are chosen from hydroxy, carboxy, alkoxycarbonyl, carboxyalkylcarbonylamino, carboxyalkylaminocarbonylamino, guanidino, 5-tetrazolyl, the residue of an amino acid and the residue of an N-methylated amino acid;
R² is an optionally substituted carbocycle or optionally substituted heterocycle of two or fewer rings;
R³ is chosen from H, -C(=O)NH₂, -(C₁-C₆)atkyl, halo(C₁-C₆)alkyl, -(C₁-C₆)alkyl-R³⁰, -(C₂-C₆)alkyl-R³¹, and saturated 4- or 5-membered heterocycle optionally substituted with methyl;
R³⁰ is chosen from -C(=O)NH₂ and 4- or 5-membered heterocycle optionally substituted with methyl;
R³¹ is chosen from (C₁-C₄)alkoxy, amino, hydroxy, (C₁-C₆)alkylamino and di(C₁-C₆)alkylamino;
R⁴ is chosen from H and F;
R⁶ is chosen from H, (C₁-C₆)alkyl and halogen;
X is N, N→O, or C-R⁵;
R⁵ is chosen from H, halogen, OH, (C₁-C₆)allcyl, (C₁-C₆)alkoxy, CF₃, CN, NH₂, CH₂OH CH₂NH₂ and C≡CH; and
M is chosen from direct bond, -C(R²⁰)(R²¹)-, -O-, -NR²²-, -S(O)ₙ-, -C(=O)-, -C(R²⁰)(R²¹)C(R²⁰)(R²¹)-, -C(R²⁰)=C(R²¹)-, -C(R²⁰)(R²¹)-O-, -C(R²⁰)(R²¹)-NR²²-, - C(R²⁰)(R²¹)-S(O)ₙ-, -C(R²⁰)(R²¹)-C(=O)-, -O-C(R²⁰)(R²¹)-, -NR²²-C(R²⁰)(R²¹)-, -S(O)ₙ-C(R²⁰)(R²¹)-, -C(=O)-C(R²⁰)(R²¹)- and 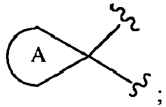 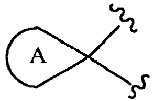 is a five or six-membered ring optionally substituted with methyl; n is zero, one or two; and R²⁰, R²¹ and R²² are selected independently in each occurrence from H and (C₁-C₄)alkyl.

The present invention also relates to pharmaceutical compositions comprising a pharmaceutically acceptable carrier and a therapeutically effective amount of at least one compound of the general formula I (i.e. Ia, Ib, Ic or Id) described above. When the compound is present as a salt, the salt should be a pharmaceutically acceptable salt.

In a third aspect, the invention relates to methods for the treatment or prophylaxis of a disease or condition mediated by peripheral (i.e. outside the CNS) phosphodiesterase-4. The methods comprise administering to a mammal a therapeutically effective amount of a compound having the general formula I. The compounds of the invention do not appear to penetrate the blood-brain barrier and are therefore of particular utility in treating peripherally PDE4 mediated diseases without the liability of central side effects, such as emesis. The disease or condition may be related to allergic, acute or chronic inflammation. The disease may be, for example, atherosclerosis, thrombosis, stroke, acute coronary syndrome, stable angina, peripheral vascular disease, critical leg ischemia, intermittent claudication, abdominal aortic aneurysm or myocardial infarction.

Selective PDE4 inhibitors of the invention are also useful for treating asthma and Chronic Obstructive Pulmonary Disease (COPD). Compounds of the invention, which inhibit tumor growth and metastases, also find utility in the treatment and prevention of cancer, including esophageal cancer, brain cancer, pancreatic cancer, and colon cancer. Compounds of the invention may also find utility in the treatment and prevention of bone loss, bladder inflammation, bladder overactivity or pain arising from bladder inflammation.

These and other embodiments of the present invention will become apparent in conjunction with the description and claims that follow.

### Detailed Description of the Invention

Throughout this specification the substituents are defined when introduced and retain their definitions.

Unless otherwise specified, alkyl is intended to include linear, branched, or cyclic hydrocarbon structures and combinations thereof. A combination would be, for example, cyclopropylmethyl. Lower alkyl refers to alkyl groups of from I to 6 carbon atoms. Examples of lower alkyl groups include methyl, ethyl, propyl, isopropyl, butyl, sand t-butyl and the like. Preferred alkyl groups are those of C₂₀ or below; C₁ to C₈ are more preferred. Cycloalkyl is a subset of alkyl and includes cyclic hydrocarbon groups of from 3 to 8 carbon atoms. Examples of cycloalkyl groups include c-propyl, c-butyl, c-pentyl, norbornyl and the like.

C₁ to C₂₀ hydrocarbon includes alkyl, cycloalkyl, polycycloalkyl, alkenyl, alkynyl, aryl and combinations thereof. Examples include benzyl, phenethyl, cyclohexylmethyl, camphoryl and naphthylethyl. Hydrocarbon refers to any substituent comprised of hydrogen and carbon as the only elemental constituents.

Unless otherwise specified, the term "carbocycle" is intended to include ring systems in which the ring atoms are all carbon but of any oxidation state. Thus (C₃-C₁₀) carbocycle refers to both non-aromatic and aromatic systems, including such systems as cyclopropane, benzene, cyclopentene and cyclohexene; (C₈-C₁₂) carbopolycycle refers to such systems as norbornane, decalin, indane and naphthalene. Carbocycle, if not otherwise limited, refers to monocycles, bicycles and polycycles.

Alkoxy or alkoxyl refers to groups of from I to 8 carbon atoms of a straight, branched or cyclic configuration and combinations thereof attached to the parent structure through an oxygen. Examples include methoxy, ethoxy, propoxy, isopropoxy, cyclopropyloxy, cyclohexyloxy and the like. Lower-alkoxy refers to groups containing one to four carbons. For the purpose of this application, alkoxy and lower alkoxy include methylenedioxy and ethylenedioxy. Alkoxyalkyl refers to ether groups of from 3 to 8 atoms of a straight, branched, cyclic configuration and combinations thereof attached to the parent structure through an alkyl. Examples include methoxymethyl, methoxyethyl, ethoxypropyl, and the like. Alkoxyaryl refers to alkoxy substituents attached to an aryl, wherein the aryl is attached to the parent structure. Arylalkoxy refers to aryl substituents attached to an oxygen, wherein the oxygen is attached to the parent structure. Substituted arylalkoxy refers to a substituted aryl substituent attached to an oxygen, wherein the oxygen is attached to the parent structure.

Oxaalkyl refers to alkyl residues in which one or more carbons (and their associated hydrogens) have been replaced by oxygen. Examples include methoxypropoxy; 3,6,9-trioxadecyl; 2,6,7-trioxabicyclo[2.2.2]octane and the like. The term oxaalkyl is intended as it is understood in the art [sec Naming and Indexing of Chemical Substances for Chemical Abstracts, published by the American Chemical Society, 196, but without the restriction of 127(a)], i.e. it refers to compounds in which the oxygen is bonded via a single bond to its adjacent atoms (forming ether bonds); it docs not refer to doubly bonded oxygen, as would be found in carbonyl groups. Similarly, thiaalkyl and azaalkyl refer to alkyl residues in which one or more carbons has been replaced by sulfur or nitrogen, respectively. Examples include ethylaminoethyl and methylthiopropyl.

Unless otherwise specified, acyl refers to formyl and to groups of 1, 2, 3, 4, 5, 6, 7 and 8 carbon atoms of a straight, branched, cyclic configuration, saturated, unsaturated and aromatic and combinations thereof, attached to the parent structure through a carbonyl functionality. One or more carbons in the acyl residue may be replaced by nitrogen, oxygen or sulfur as long as the point of attachment to the parent remains at the carbonyl. Examples include acetyl, benzoyl, propionyl, isobutyryl, t-butoxycarbonyl, benzyloxycarbonyl and the like. Lower-acyl refers to groups containing one to four carbons. The double bonded oxygen, when referred to as a substituent itself is called "oxo".

Aryl and heteroaryl mean (i) a phenyl group (or benzene) or a monocyclic 5- or 6-membered heteroaromatic ring containing 1-4 heteroatoms selected from O, N, or S; (ii) a bicyclic 9- or 10-membered aromatic or heteroaromatic ring system containing 0-4 heteroatoms selected from O, N, or S; or (iii) a tricyclic 13- or 14-membered aromatic or heteroaromatic ring system containing 0-5 heteroatoms selected from O, N, or S. Aryl, as understood herein, includes residues in which one or more rings are aromatic, but not all need be. Thus aromatic 6- to 14-membered carbocyclic rings include, e.g., benzene, naphthalene, indane, tetralin, and fluorene and the 5- to 10-membered aromatic heterocyclic rings include, e.g., imidazole, pyridine, indole, thiophene, benzopyranone, thiazole, furan, benzimidazole, quinoline, isoquinoline, quinoxaline, pyrimidine, pyrazinc, tetrazole and pyrazole.

Arylalkyl refers to a substituent in which an aryl residue is attached to the parent structure through alkyl. Examples are benzyl, phenethyl and the like. Heteroarylalkyl refers to a substituent in which a heteroaryl residue is attached to the parent structure through alkyl. In one embodiment, the alkyl group of an arylalkyl or a heteroarylalkyl is an alkyl group of from I to 6 carbons. Examples include, e.g., pyridinylmethyl, pyrimidinylethyl and the like.

Heterocycle means a cycloalkyl or aryl carbocycle residue in which from one to three carbons is replaced by a heteroatom selected from the group consisting of N, O and S. The nitrogen and sulfur heteroatoms may optionally be oxidized, and the nitrogen heteroatom may optionally be quaternized. Unless otherwise specified, a heterocycle may be non-aromatic or aromatic. It is to be noted that heteroaryl is a subset of heterocycle in which the heterocycle is aromatic. Examples of heterocyclic residues that fall within the scope of the invention include pyrazole, pyrrole, indole, quinoline, isoquinoline, tetrahydroisoquinoline, benzofuran, benzodioxan, benzodioxole (commonly referred to as methylenedioxyphenyl, when occurring as a substituent), morpholine, thiazole, pyridine (including 2-oxopyridine), pyridine N-oxide, pyrimidine, thiophene (i.e. thiene), furan, oxazole, oxazoline, oxazolidine, isoxazolidine, isoxazole, dioxane, azetidine, piperazine, piperidine, pyrrolidine, pyridazine, azepine, pyrazolidine, imidazole, imidazoline, imidazolidine, imidazolopyridine, pyrazine, thiazolidine, isothiazole, 1,2-thiazine-1,1-dioxide, quinuclidine, isothiazolidine, benzimidazole, thiadiazole, benzopyran, benzothiazole, benzotriazole, benzoxazole, tetrahydrofuran, tetrahydropyran, benzothiene, thiamorpholine, thiamorpholine sulfoxide, thiamorpholine sulfone, oxadiazole, triazole, tetrazole, isatin (dioxoindole), phthalimide (dioxoisoindole), pyrrolopyridine, triazolopyridine and the dihydro and tetrahydro congeners of the fully unsaturated ring systems among the foregoing.

An oxygen heterocycle is a heterocycle containing at least one oxygen in the ring; it may contain additional oxygens, as well as other heteroatoms. Oxygen heterocycles found in the examples of the invention include tetrahydrofuran, benzodioxole, morpholine, isoxazole and 2,6,7-trioxabicyclo[2.2.2]octane. A sulphur heterocycle is a heterocycle containing at least one sulphur in the ring; it may contain additional sulphurs, as well as other heteroatoms. A nitrogen heterocycle is a heterocycle containing at least one nitrogen in the ring; it may contain additional nitrogens, as well as other heteroatoms.

As used herein, the term "optionally substituted" may be used interchangeably with "unsubstituted or substituted". The term "substituted" refers to the replacement of one or more hydrogen atoms in a specified group with a specified radical. For example, substituted alkyl, aryl, cycloalkyl, heterocyclyl etc. refer to alkyl, aryl, cycloalkyl, or heterocyclyl wherein up to three H atoms in each residue are replaced with halogen, haloalkyl, alkyl, acyl, alkoxyalkyl, hydroxyalkyl, carbonyl (i.e. oxo), phenyl, heteroaryl, benzenesulfonyl, hydroxy, alkoxy, haloalkoxy, oxaalkyl, carboxy, carboxyalkyl, carboxyalkoxy, carboxyalkylthio, alkoxycarbonyl [-C(=O)O-alkyl], alkoxycarbonylamino [ -NHC(=O)O-alkyl], alkoxycarbonylaminoalkyl [ -alkyl-NHC(=O)O-alkyl], carboxyalkylcarbonylamino [ -NHC(=O)-alkyl-COOH], carboxamido [-C(=O)NH_{2]}, aminocarbonyloxy [-OC(=O)NH₂], alkylaminocarbonyl [-C(=O)NH-alkyl], dialkylaminocarbonyl [-C(=O)N(alkyl)₂], aminocarbonylalkyl [-alkyl-C(=O)NH₂], cyano, acetoxy, nitro, amino, alkylamino, dialkylamino, aminoalkyl, (alkyl)(aryl)aminoalkyl, alkylaminoalkyl (including cycloalkylaminoalkyl), dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, and benzyloxy. Although oxo is included among the substituents referred to in "optionally substituted", it will be appreciated by persons of skill in the art that, because oxo is a divalent radical, there are circumstances in which it will not be appropriate as a substituent (e.g. on phenyl). Additional substituents that are considered within the scope of the term, particularly for R¹, are the are the residues of amino acids, amino acid amides, protected residues of aminoacids and their amides, and N-methylated (mono- or di-, as appropriate) amino acids and amino acid amides.

For the purpose of R¹, the substituents alkyl, acyl, alkoxyalkyl, hydroxyloweralkyl, phenyl, heteroaryl, benzenesulfonyl, loweralkoxy, haloalkoxy, oxaalkyl, alkoxycarbonyl, alkoxycarbonylamino, carboxamido, alkylaminocarbonyl, amino, alkylamino, (alkyl)(aryl)aminoalkyl, alkylaminoalkyl, heterocyclylalkoxy, alkylthio, sulfonylamino, alkylsulfinyl, alkylsulfonyl, acylaminoalkyl, acylaminoalkoxy, acylamino, amidino, aryl, benzyl, heterocyclyl, heterocyclylalkyl, heterocyclylalkoxy, phenoxy, benzyloxy, heteroaryloxy, heterocyclylamino, oxaalkyl, aminosulfonyl, amidino, guanidino, urcido, benzyloxyphenyl, and benzyloxy may be further substituted with one or two substituents from the list of substituents above. Substituents that are considered within the scope of the term, particularly for R¹, are the are the residues of amino acids, amino acid amides and protected residues of aminoacids and their amides, as well as the following specific residues: -CH₃, -CH₂CF₃, -CF₃, -CHO, -COOH, -CN, halogen, -OH, , -OEt, -C(=O)NH₂, -C(=O)NHEt, -C(=O)NMe₂ -COOCH₃, -COOEt, -CH₂NHC(=O)NH₂, -CH(CH₃)NHC(=O)NH₂, -CH₂NHC(=O)H, -CH₂NHC(=O)CH₃, -CH₂C(=O)NH₂, -CH₂COOH, -CH₂COOEt, -CH₂NHC(=O)OEt, -CH₂NHC(=O)O-C₆H₅, -CH₂NHC(=O)C(=O)NH₂, -CH₂NHC(=O)NHEt, -C(CH₃)₂OH -CH₂NHC(=O)N(CH₃)₂, -CH₂NHC(=O)NHCH₃, -CH₂NH₂, -CH(CH₃)NH₂, -C(CH₃)₂NH₂, -CH₂OH -CH₂CH₂OH -CH₂NHSO₂CH₃ -CH₂OC(=O)NHEt, -OCH(CH₃)COOH, -SCH₂COOH, -OCH₃, -OC(=O)NH₂, -OCH₂CH₂N(CH₃)₂, -OCH₂CH₂OCH₃ -NHC(=O)NH₂, -NHC(=O)NHEt, -NHCH₃, -NHEt, -NH(tBoc), -NHCH₂COOH ("residue of glycine"), -N(CH₃)CH₂COOH ("residue of N-methylglycine"), -NHC(=O)NHCH₂CH₂Cl, -NHSO₂NH₂ -NHEt, -N(CH₃)₂, -NH₂,, -NH(CH₃)C(=O)NH₂, -NHSO₂CH₃ -N(SO₂CH₃)₂ -NHC(=O)OCH₃, -NHC(=O)OtBu, -NHC(=O)CH₃, -SO₂NH₂ -NHC(=O)CH₂CH₂COOH, -NHC(=O)NHCH₂COOH, -CH₂NHCHO, -NHC(=O)NHCH₂COOEt, -NHC(=O)NH(CH₂)₃COOEt, -N-HC(=O)NH(CH₂)₂COOEt, -N(CH₃)CH₂CH₂OH -NHC(=O)OEt, -N(Et)C(=O)OEt, -NHC(=O)NH(CH₂)₂COOH, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc), 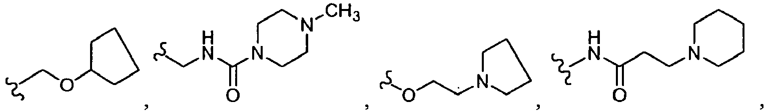 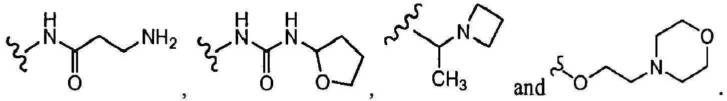 The term "a residue of an amino acid, amino acid amide", etc. refers to an amino acid etc. minus the functional groups that are considered part of the bond to the parent structure. For example, in the molecule illustrated below: 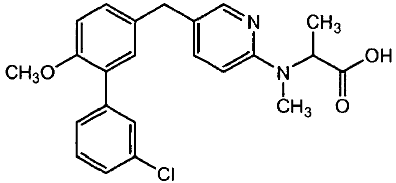 after one subtracts the hydrogen that connects N-methyl alanine to the phenyl ring, the structure of A that remains is: 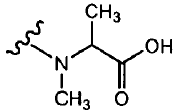 This is not *sensu stricto* an N-methyl amino acid, since it lacks the hydrogen on the amino terminus. This and similar structures that lack atoms at the points of attachment (e.g. the H of NH₂) are referred to herein as "residues" of their respective parents.

The terms "haloalkyl" and "haloalkoxy" mean alkyl or alkoxy, respectively, substituted with one or more halogen atoms. The terms "alkylcarbonyl" and "alkoxycarbonyl" mean -C(=O)alkyl or-C(O)alkoxy, respectively.

The term "halogen" means fluorine, chlorine, bromine or iodine. In one embodiment, halogen may be fluorine or chlorine.

Substituents Rⁿ are generally defined when introduced and retain that definition throughout the specification and in all independent claims.

In the characterization of some of the substituents, it is recited that certain substituents may combine to form rings. Unless stated otherwise, it is intended that such rings may exhibit various degrees of unsaturation (from fully saturated to fully unsaturated), may include heteroatoms and may be substituted with lower alkyl or alkoxy.

It will be recognized that the compounds of this invention can exist in radiolabeled form, i.e., the compounds may contain one or more atoms containing an atomic mass or mass number different from the atomic mass or mass number usually found in nature. Radioisotopes of hydrogen, carbon, phosphorous, fluorine, and chlorine include ²H, ³H, ¹³C,¹⁴C, ¹⁵N ³⁵S, ¹⁸F, and ³⁶Cl, respectively. Compounds that contain those radioisotopes and/or other radioisotopes of other atoms are within the scope of this invention. Tritiated, i.e. ³H, and carbon-14, i.e., ¹⁴C, radioisotopes are particularly preferred for their ease in preparation and detectability. Compounds that contain isotopes ¹¹C, ¹³N, ¹⁵O and ¹⁸F are well suited for positron emission tomography. Radiolabeled compounds of formula I of this invention and prodrugs thereof can generally be prepared by methods well known to those skilled in the art. Conveniently, such radiolabeled compounds can be prepared by carrying out the procedures disclosed in the Examples and Schemes by substituting a readily available radiolabeled reagent for a nonradiolabeled reagent.

As used herein (particularly in the claims), and as would be understood by the person of skill in the art, the recitation of "a compound" is intended to include salts, solvates, co-crystals and inclusion complexes of that compound as well as any stereoisomeric form, or a mixture of any such forms of that compound in any ratio. Thus, in accordance with some embodiments of the invention, a compound as described herein, including in the contexts of pharmaceutical compositions, methods of treatment, and compounds *per se*, is provided as the salt form. Thus, for example, the recitation "a compound of formula I" as depicted above, in which R¹ is imidazolyl, would include imidazolium salts. In a particular embodiment, the term "compound of formula I" refers to the compound or a pharmaceutically acceptable salt thereof.

The compounds described herein may contain asymmetric centers and may thus give rise to enantiomers, diastereomers, and other stereoisomeric forms. Each chiral center may be defined, in terms of absolute stereochemistry, as (R)- or (S)-. The present invention is meant to include all such possible isomers, in any ratio from racemic to optically pure forms. Optically active (R)- and (S)- isomers may be prepared using chiral synthons or chiral reagents, or resolved using conventional techniques. The prefix "*rac*" refers to a racemate. When the compounds described herein contain olefinic double bonds or other centers of geometric asymmetry, and unless specified otherwise, it is intended that the compounds include both E and Z geometric isomers. The representation of the configuration of any carbon-carbon double bond appearing herein is selected for convenience only, and unless explicitly stated, is not intended to designate a particular configuration. Thus a carbon-carbon double bond depicted arbitrarily as E may be *Z*, *E*, or a mixture of the two in any proportion. Likewise, all tautomeric forms are also intended to be included.

The term "solvate" refers to a compound of Formula I in the solid state, wherein molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions. Inclusion complexes are described in Remington: The Science and Practice of Pharmacy 19th Ed. (1995) volume 1, page 176-177, which is incorporated herein by reference. The most commonly employed inclusion complexes are those with cyclodextrins, and all cyclodextrin complexes, natural and synthetic, are specifically encompassed within the claims.

The term "pharmaceutically acceptable salt" refers to salts prepared from pharmaceutically acceptable non-toxic acids or bases including inorganic acids and bases and organic acids and bases. When the compounds of the present invention are basic, salts may be prepared from pharmaceutically acceptable non-toxic acids including inorganic and organic acids. Suitable pharmaceutically acceptable anions for the compounds of the present invention include acetate, benzenesulfonate (besylate), benzoate, bicarbonate, bisulfate, carbonate, camphorsulfonate, citrate, ethanesulfonate, fumarate, gluconate, glutamate, glycolatc, bromide, chloride, isethionate, lactate, maleate, malate, mandelate, methanesulfonate, mucate, nitrate, pamoate, pantothenate, phosphate, succinate, sulfate, tartrate, trifluoroacetate, p-toluenesulfonate, acetamidobenzoate, adipate, alginate, aminosalicylate, anhydromethylenecitrate, ascorbate, aspartate, calcium edetate, camphorate, camsylate, caprate, caproate, caprylate, cinnamate, cyclamate, dichloroacetate, edetate (EDTA), edisylate, embonate, estolate, esylate, fluoride, formate, gentisate, gluceptate, glucuronate, glycerophosphate, glycolate, glycollylarsanilate, hexylresorcinate, hippurate, hydroxynaphthoate, iodide, lactobionate, malonate, mesylate, napadisylate, napsylate, nicotinate, oleate, orotate, oxalate, oxoglutarate, palmitate, pectinate, pectinate polymer, phenylethylbarbiturate, picrate, pidolate, propionate, rhodanide, salicylate, sebacate, stearate, tannate, theoclate, tosylate and the like. The desired salt may be obtained by ion exchange of whatever counter ion is obtained in the synthesis of the quat. These methods arc well known to persons of skill. Although pharmaceutically acceptable counter ions will be preferred for preparing pharmaceutical formulations, other anions are quite acceptable as synthetic intermediates. When the compounds contain an acidic side chain, suitable pharmaceutically acceptable base addition salts for the compounds of the present invention include metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine.

The graphic representations of racemic, ambiscalemic and scalemic or enantiomerically pure compounds used herein are taken from Maehr J. Chem. Ed. 62, 114-120 (1985): solid and broken wedges are used to denote the absolute configuration of a chiral element; wavy lines and single thin lines indicate disavowal of any stereochemical implication which the bond it represents could generate; solid and broken bold lines are geometric descriptors indicating the relative configuration shown but denoting racemic character; and wedge outlines and dotted or broken lines denote enantiomerically pure compounds of indeterminate absolute configuration.

Terminology related to "protecting", "deprotecting" and "protected" functionalities occurs throughout this application. Such terminology is well understood by persons of skill in the art and is used in the context of processes that involve sequential treatment with a series of reagents. In that context, a protecting group refers to a group, which is used to mask a functionality during a process step in which it would otherwise react, but in which reaction is undesirable. The protecting group prevents reaction at that step, but may be subsequently removed to expose the original functionality. The removal or "deprotection" occurs after the completion of the reaction or reactions in which the functionality would interfere. Thus, when a sequence of reagents is specified, as it is in the processes of the invention, the person of ordinary skill can readily envision those groups that would be suitable as "protecting groups". Suitable groups for that purpose are discussed in standard textbooks in the field of chemistry, such as Protective Groups in Organic Synthesis by T.W. Greene [John Wiley & Sons, New York, 1991], which is incorporated herein by reference.

A comprehensive list of abbreviations utilized by organic chemists appears in the first issue of each volume of the Journal of Organic Chemistry. The list, which is typically presented in a table entitled "Standard List of Abbreviations", is incorporated herein by reference.

In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants that are in themselves known, but are not mentioned here. The starting materials, are either commercially available, synthesized as described in the examples or may be obtained by the methods well known to persons of skill in the art.

PDE4 inhibitors have been shown to be effective therapeutic agents in clinical studies. For example, administration of cilomilast and roflumilast (PDE4 inhibitors) to patients suffering from asthma and COPD showed initially excellent results, although the effect of cilomilast disappeared on long-term trial [Lipworth, Lancet 365, 167-175 (2005)]. Genetic studies have clearly demonstrated an association between PDE4D and ischemic stroke (Gretarsdottir et al. 2003. Nature Genetics. 35, 1-8). Selective PDE4 inhibitors (e.g. rolipram) are also useful for treating bone loss [Yao et al., J.Musculoskelet.Neuronal Interact. 7, 119-130 (2007)].

Additionally, a PDE4 inhibitor, YM976, was shown to ameliorate the effects of experimentally-induced interstitial cystitis in rats, resulting in a decrease in the frequency of urination and an increase in the volume of urine at each time of urination [Kitta et al., BJU Int. 102, 1472-1476 (2008)]. Another PDE4 inhibitor, IC485, was shown to be equally efficacious as tolteradine tartrate, a marketed drug for treating overactive bladder, in a rodent model of obstructive bladder [Kaiho et al. BJU Int. 101, 615-20 (2008)]. These findings suggest that PDE4 inhibitors will be useful in treating symptoms of bladder overactivity, inflammation and pain.

Furthermore, the compounds, compositions and methods of the present invention may be useful in treating cancer. Phosphodiesterase activity has been shown to be associated with hematological malignancies [Lerner ct al., Biochem.J. 393, 21-41 (2006); Ogawa et al., Blood 99, 3390-3397 (2002)].

Furthermore, the compounds, compositions and methods of the present invention, particularly when radiolabeled as described above or labeled by methods well-known in the art with florescent and spin labels, may be employed as imaging agents and in other ways for diagnosis and/or treatment. Moreover, immobilization of compounds of the invention on solid support could be of utility for affinity purification and modification of compounds of the invention with chemically active groups may be used for protein labeling.

For many of the utilities outlined above, it may be advantageous to administer compounds of the general formula 1 together with cholinesterase inhibitors (e.g. tacrine, huperzine, donepezil); NMDA antagonists (e.g. lanicemine, remacemide, neramexane, memantine); calpain inhibitors (e.g. CEP-3122); antioxidants (e.g.vitamin E, coenzyme Q10) and agents that have shown clinical efficacy but whose mechanism is unclear (e.g. dimebon).

The terms "methods of treating or preventing" mean amelioration, prevention or relief from the symptoms and/or effects associated with disorders. The term "preventing" as used herein refers to administering a medicament beforehand to forestall or obtund an acute episode. The person of ordinary skill in the medical art (to which the present method claims are directed) recognizes that the term "prevent" is not an absolute term. In the medical art it is understood to refer to the prophylactic administration of a drug to substantially diminish the likelihood or seriousness of a condition, and this is the sense intended in applicants' claims. As used herein, reference to "treatment," of a patient is intended to include prophylaxis.

The term "mammal" is used in its dictionary sense. Humans are included in the group of mammals, and humans would be the preferred subjects of the methods.

While it may be possible for compounds of formula I to be administered as the raw chemical, it will often be preferable to present them as part of a pharmaceutical composition. In accordance with an embodiment of the present invention there is provided a pharmaceutical composition comprising a compound of formula I or a pharmaceutically acceptable salt thereof, together with one or more pharmaceutically carriers thereof and optionally one or more other therapeutic ingredients. The carrier(s) must be "acceptable" in the sense of being compatible with the other ingredients of the formulation and not deleterious to the recipient thereof. Furthermore, when reference is made in an independent claim to a compound or a pharmaceutically acceptable salt thereof, it will be understood that claims which depend from that independent claim which refer to such a compound also include pharmaceutically acceptable salts of the compound, even if explicit reference is not made to the salts in the dependent claim.

The formulations include those suitable for oral, parenteral (including subcutaneous, intradermal, intramuscular, intravenous and intraarticular), rectal and topical (including dermal, buccal, sublingual and intraocular) administration. The most suitable route may depend upon the condition and disorder of the recipient. The formulations may conveniently be presented in unit dosage form and may be prepared by any of the methods well known in the art of pharmacy. Such methods include the step of bringing into association a compound of formula I or a pharmaceutically acceptable salt or solvate thereof ("active ingredient") with the carrier, which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may optionally be coated or scored and may be formulated so as to provide sustained, delayed or controlled release of the active ingredient therein. The pharmaceutical compositions may include a "pharmaceutically acceptable inert carrier", and this expression is intended to include one or more inert excipients, which include starches, polyols, granulating agents, microcrystalline cellulose, diluents, lubricants, binders, disintegrating agents, and the like. If desired, tablet dosages of the disclosed compositions may be coated by standard aqueous or nonaqueous techniques, "Pharmaceutically acceptable carrier" also encompasses controlled release means.

Pharmaceutical compositions may also optionally include other therapeutic ingredients, anti-caking agents, preservatives, sweetening agents, colorants, flavors, desiccants, plasticizers, dyes, and the like. Any such optional ingredient must be compatible with the compound of formula I to insure the stability of the formulation. The composition may contain other additives as needed, including for example lactose, glucose, fructose, galactose, trehalose, sucrose, maltose, raffinose, maltitol, melezitose, stachyose, lactitol, palatinite, starch, xylitol, mannitol, myoinositol, and the like, and hydrates thereof, and amino acids, for example alanine, glycine and betaine, and peptides and proteins, for example albumen.

Examples of excipients for use as the pharmaceutically acceptable carriers and the pharmaceutically acceptable inert carriers and the aforementioned additional ingredients include, but are not limited to binders, fillers, disintegrants, lubricants, antimicrobial agents, and coating agents.

The dose range for adult humans is generally from 0.005 mg to 10 g/day orally. Tablets or other forms of presentation provided in discrete units may conveniently contain an amount of compound of formula I which is effective at such dosage or as a multiple of the same, for instance, units containing 5 mg to 500 mg, usually around 10 mg to 200 mg. The precise amount of compound administered to a patient will be the responsibility of the attendant physician. However, the dose employed will depend on a number of factors, including the age and sex of the patient, the precise disorder being treated, and its severity.

A dosage unit (e.g. an oral dosage unit) can include from, for example, 1 to 30 mg, 1 to 40 mg, I to 100 mg, I to 300 mg, 1 to 500 mg, 2 to 500 mg, 3 to 100 mg, 5 to 20 mg, 5 to 100 mg (e.g. 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 150 mg, 200 mg, 250 mg, 300 mg, 350 mg, 400 mg, 450 mg, 500 mg) of a compound described herein.

For additional information about pharmaceutical compositions and their formulation, see, for example, Remington: The Science and Practice of Pharmacy, 20th Edition, 2000. The agents can be administered, e.g., by intravenous injection, intramuscular injection, subcutaneous injection, intraperitoneal injection, topical, sublingual, intraarticular (in the joints), intradermal, buccal, ophthalmic (including intraocular), intranasaly (including using a cannula), or by other routes. The agents can be administered orally, e.g., as a tablet or cachet containing a predetermined amount of the active ingredient, gel, pellet, paste, syrup, bolus, electuary, slurry, capsule, powder, granules, as a solution or a suspension in an aqueous liquid or a non-aqueous liquid, as an oil-in-water liquid emulsion or a water-in-oil liquid emulsion, via a micellar formulation (see, e.g. WO 97/11682) via a liposomal formulation (see, e.g., EP 736299,WO 99/59550 and WO 97/13500), via formulations described in WO 03/094886 or in some other form. The agents can also be administered transdermally (i.e. via reservoir-type or matrix-type patches, microneedles, thermal poration, hypodermic needles, iontophoresis, electroporation, ultrasound or other forms of sonophoresis, jet injection, or a combination of any of the preceding methods (Prausnitz et al. 2004, Nature Reviews Drug Discovery 3:115)). The agents can be administered locally, for example, at the site of injury to an injured blood vessel. The agents can be coated on a stent. The agents can be administered using high-velocity transdermal particle injection techniques using the hydrogel particle formulation described in U.S. 20020061336. Additional particle formulations are described in WO 00/45792, WO 00/53160, and WO 02/19989. An example of a transdermal formulation containing plaster and the absorption promoter dimethylisosorbide can be found in WO 89/04179. WO 96/11705 provides formulations suitable for transdermal administration. The agents can be administered in the form a suppository or by other vaginal or rectal means. The agents can be administered in a transmembrane formulation as described in WO 90/07923. The agents can be administered non-invasively via the dehydrated particles described in U.S. 6,485,706. The agent can be administered in an enteric-coated drug formulation as described in WO 02/49621. The agents can be administered intranasaly using the formulation described in U.S. 5,179,079. Formulations suitable for parenteral injection are described in WO 00/62759. The agents can be administered using the casein formulation described in U.S. 20030206939 and WO 00/06108. The agents can be administered using the particulate formulations described in U.S. 20020034536.

The agents, alone or in combination with other suitable components, can be administered by pulmonary route utilizing several techniques including but not limited to intratracheal instillation (delivery of solution into the lungs by syringe), intratracheal delivery of liposomes, insufflation (administration of powder formulation by syringe or any other similar device into the lungs) and aerosol inhalation. Aerosols (e.g., jet or ultrasonic nebulizers, metered-dose inhalers (MDIs), and dry-Powder inhalers (DPIs)) can also be used in intranasal applications. Aerosol formulations are stable dispersions or suspensions of solid material and liquid droplets in a gaseous medium and can be placed into pressurized acceptable propellants, such as hydrofluoroalkanes (HFAs, i.e. HFA-134a and HFA-227, or a mixture thereof), dichlorodifluoromethane (or other chlorofluorocarbon propellants such as a mixture of Propellants 11, 12, and/or 114), propane, nitrogen, and the like. Pulmonary formulations may include permeation enhancers such as fatty acids, and saccharides, chelating agents, enzyme inhibitors (e.g., protease inhibitors), adjuvants (e.g., glycocholate, surfactin, span 85, and nafamostat), preservatives (e.g., benzalkonium chloride or chlorobutanol), and ethanol (normally up to 5% but possibly up to 20%, by weight). Ethanol is commonly included in aerosol compositions as it can improve the function of the metering valve and in some cases also improve the stability of the dispersion. Pulmonary formulations may also include surfactants which include but are not limited to bile salts and those described in U.S. 6,524,557 and references therein. The surfactants described in U.S. 6,524,557, e.g., a C₈-C₁₆ fatty acid salt, a bile salt, a phospholipid, or alkyl saccharide are advantageous in that some of them also reportedly enhance absorption of the compound in the formulation. Also suitable in the invention are dry powder formulations comprising a therapeutically effective amount of active compound blended with an appropriate carrier and adapted for use in connection with a dry-Powder inhaler. Absorption enhancers which can be added to dry powder formulations of the present invention include those described in U.S. 6,632,456. WO 02/080884 describes new methods for the surface modification of powders. Aerosol formulations may include U.S. 5,230,884, U.S. 5,292,499, WO 017/8694, WO 01/78696, U.S. 2003019437, U. S. 20030165436, and WO 96/40089 (which includes vegetable oil). Sustained release formulations suitable for inhalation are described in U.S. 20010036481A1, 20030232019A1, and U.S. 20040018243A as well as in WO 01/13891, WO 02/067902, WO 03/072080, and WO 03/079885. Pulmonary formulations containing microparticles are described in WO 03/015750, U.S. 20030008013, and WO 00/00176. Pulmonary formulations containing stable glassy state powder are described in U.S. 20020141945 and U.S. 6,309,671. Other aerosol formulations are described in EP 1338272A1 WO 90/09781, U. S. 5,348,730, U.S. 6,436,367, WO 91/04011, and U.S. 6,294,153 and U.S. 6,290,987 describes a liposomal based formulation that can be administered via aerosol or other means. Powder formulations for inhalation are described in U.S. 20030053960 and WO 01/60341. The agents can be administered intranasally as described in U.S. 20010038824.

Solutions of medicament in buffered saline and similar vehicles are commonly employed to generate an aerosol in a nebulizer. Simple nebulizers operate on Bernoulli's principle and employ a stream of air or oxygen to generate the spray particles. More complex nebulizers employ ultrasound to create the spray particles. Both types are well known in the art and arc described in standard textbooks of pharmacy such as Sprowls' American Pharmacy and Remington's The Science and Practice of Pharmacy. Other devices for generating aerosols employ compressed gases, usually hydrofluorocarbons and chlorofluorocarbons, which are mixed with the medicament and any necessary excipients in a pressurized container, these devices arc likewise described in standard textbooks such as Sprowls and Remington.

The agent can be incorporated into a liposome to improve half-life. The agent can also be conjugated to polyethylene glycol (PEG) chains. Methods for pegylation and additional formulations containing PEG-conjugates (i.e. PEG-based hydrogels, PEG modified liposomes) can be found in Harris and Chess, Nature Reviews Drug Discovery 2:214-221 and the references therein. The agent can be administered via a nanocochleate or cochleate delivery vehicle (BioDelivery Sciences International). The agents can be delivered transmucosally (i.e. across a mucosal surface such as the vagina, eye or nose) using formulations such as that described in U.S. 5,204,108. The agents can be formulated in microcapsules as described in WO 88/01165. The agent can be administered intra-orally using the formulations described in U.S. 20020055496, WO 00/47203, and U.S. 6,495,120. The agent can be delivered using nanoemulsion formulations described in WO 01/91728A2.

In general, compounds of formula I may be prepared by the methods illustrated in the general reaction schemes as, for example, described below, or by modifications thereof, using readily available starting materials, reagents and conventional synthesis procedures. In these reactions, it is also possible to make use of variants that are in themselves known, but are not mentioned here.

The present invention relates to compounds exhibiting PDE4 enzyme inhibition, having the general formula Ia, Ib, Ic or Id: 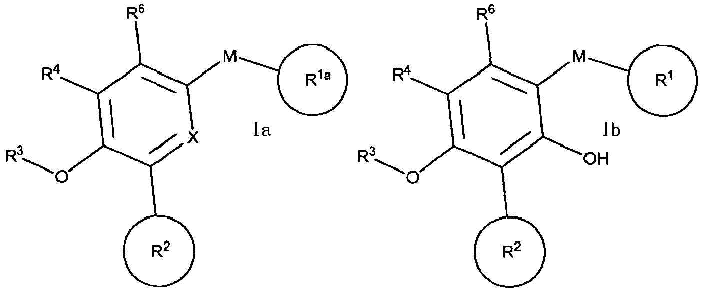 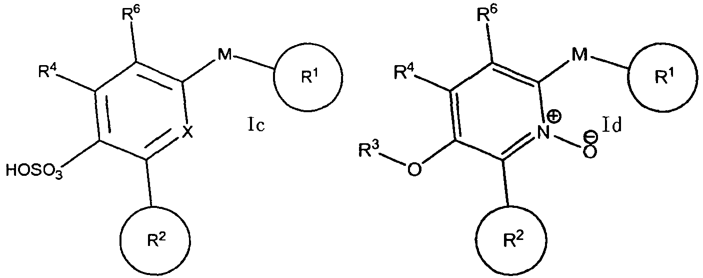 In some embodiments of Ia or Ic, X is N or N→O; in others, X is CR⁵. In another embodiment, M is chosen from direct bond, -CH₂-, -CH(OH)-, -C[(CH₃)(OH)]-, - C[(CH₃)(NH₂)]-, -C(=O)-, -O-, -NH-, -N(CH₃)-, -S(O)ₙ-, -CH₂NH-, -CH₂CH₂-, -CH=CH-, -CH₂S(O)ₙ-, -CH₂O- and

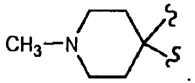

In one embodiment, R¹ or R^{1a} is a substituted phenyl. In another embodiment, R¹ is an unsubstituted heterocycle. In another embodiment, R¹ or R^{1a} is a substituted heterocycle. In both heterocycle embodiments, the heterocycle may be chosen from pyrazole, pyrrole, indole, quinoline, isoquinoline, tetrahydroisoquinoline, benzofuran, benzodioxan, benzodioxole, morpholine, thiazole, pyridine, pyridine N-oxide, pyrimidine, thiene, furan, oxazole, oxazoline, oxazolidine, isoxazolidine, isoxazole, dioxane, azetidine, piperazine, piperidine, pyrrolidine, pyridazine, azepine, pyrazolidine, imidazole, imidazoline, imidazolidine, purine, imidazolopyridinc, pyrazine, thiazolidine, isothiazole, 1,2-thiazine-1,1-dioxide, 2,6,7-trioxabicyclo[2.2.2]octane, quinuclidine, isothiazolidine, benzimidazole, thiadiazole, benzopyran, benzothiazole, benzotriazole, benzoxazole, benzoxadiazole, tetrahydrofuran, tetrahydropyran, benzothiene, thiamorpholine, thiamorpholine sulfoxide, thiamorpholine sulfone, oxadiazole, triazole, tetrazole, isoindole, pyrrolopyridine, triazolopyridine and the dihydro and tetrahydro congeners thereof, whether specifically named or not. For example, a dihydro congener of indole would be indoline or dihydroindole; a tetrahydro congener of pyridine would be piperidine. In a further embodiment, R¹ is an optionally substituted heterocycle chosen from pyrazole, benzodioxole, morpholine, thiazole, pyridine, pyridine N-oxide, pyrimidine, thiene, oxazolidine, isoxazole, azetidine, piperazine, pyrrolidine, imidazole, imidazolidine, imidazolopyridine, pyrazine, 1,2-thiazine-1,1-dioxide, benzimidazole, thiadiazole, benzotriazole, benzoxazolc, oxadiazole, triazole, tetrazole, isoindole, pyrrolopyridine, triazolopyridine and their dihydro and tetrahydro congeners.

In an embodiment, the substituted phenyl or substituted heterocycle is substituted with a substituent chosen from halogen, haloalkyl, alkyl, acyl, alkoxyalkyl, hydroxyalkyl, carbonyl, phenyl, heteroaryl, benzenesulfonyl, hydroxy, alkoxy, haloalkoxy, oxaalkyl, carboxy, alkoxycarbonyl, alkoxycarbonylalkyl, alkoxycarbonylamino, carboxyalkyl, carboxyalkoxy, carboxyalkylthio, alkoxycarbonylaminoalkyl, carboxyalkylcarbonylamino, carboxamido, aminocarbonyloxy, alkylaminocarbonyl, dialkylaminocarbonyl, aminocarbonylalkyl, cyano, acetoxy, nitro, amino, alkylamino, dialkylamino, aminoalkyl, (alkyl)(aryl)aminoalkyl, alkylaminoalkyl, dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, benzyloxy, the residues of amino acids, amino acid amides, protected residues of aminoacids, protected residues of amino acid amides, N-methylated amino acids and N-methylated amino acid amides. Exemplary amino acids are glycine, alanine and proline. Exemplary carboxyalkoxy and carboxyalkylthio are lactic acid and thioglycollic acid respectively.

In another embodiment, the substituted phenyl or substituted heterocycle is substituted with a substituent chosen from -CH₃, -CH₂CF₃, -CF₃, -CHO, -COOH, -CN, halogen, -OH, , -OEt, -C(=O)NH₂, -C(=O)NHEt, -C(=O)NMe₂ -COOCH₃, -COOEt, -CH₂NHC(=O)NH₂, -CH(CH₃)NHC(=O)NH₂, -CH₂NHC(=O)H, -CH₂NHC(=O)CH₃, -CH₂C(=O)NH₂, -CH₂COOH, -CH₂COOEt, -CH₂NHC(=O)OEt, -CH₂NHC(=O)O-C₆H₅, -CH₂NHC(=O)C(=O)NH₂, -CH₂NHC(=O)NHEt, -C(CH₃)₂OH -CH_{Z}NHC(=O)N(CH₃)₂, -CH₂NHC(=O)NHCH₃, -CH₂NH₂, -CH(CH₃)NH₂, -C(CH₃)₂NH₂, -CH₂OH -CH₂CH₂OH -CH₂NHSO₂CH₃ -CH₂OC(=O)NHEt, -OCH₃, -OC(=O)NH₂, -OCH₂CH₂N(CH₃)₂, -OCH₂CH₂OCH₃ -OCH(CH₃)COOH, -SCH₂COOH, -NHC(=O)NH₂, -NHC(=O)NHEt, -NHCH₃, -NHEt, -NH(tBoc), -NHCH₂COOH, -N(CH₃)CH₂COOH, -NHC(=O)NHCH₂CH₂Cl, -NHSO₂NH₂ -NHEt, -N(CH₃)₂, -NH₂,, -NH(CH₃)C(=O)NH₂, -NHSO₂CH₃ -N(SO₂CH₃)₂, -NHC(=O)OCH₃, -NHC(=O)OtBu, -NHC(=O)CH₃, -SO₂NH₂ -NHC(=O)CH₂CH₂COOH, -NHC(=O)NHCH₂COOH, -CH₂NHCHO, -NHC(=O)NHCH₂COOEt, -NHC(=O)NH(CH₂)₃COOEt,-NHC(=O)NH(CH₂)₂COOEt, -N(CH₃)CH₂CH₂OH -NHC(=O)OEt, -N(Et)C(=O)OEt, -NHC(=O)NH(CH₂)₂COOH, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc),

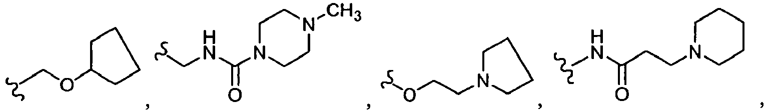

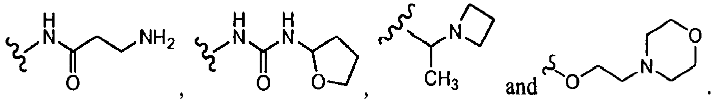

In embodiments of formula Ia, R^{1a} may be (1) a substituted heterocycle of three or fewer rings; (2) a monocyclic heterocycle attached to a substituted monocyclic heterocycle; or (3) a substituted carbocycle of three or fewer rings. The substituents on the heterocycle or carbocycle may be chosen from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂ -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH and 5-tetrazolyl. An example of the second class of embodiments, a monocyclic heterocycle attached to a monocyclic heterocycle substituted with a carboxylic acid is the R^{1a} residue found in example A053 below:

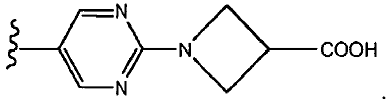

In an embodiment, R² is chosen from optionally substituted phenyl, optionally substituted monocyclic unsaturated heterocycle, unsubstituted bicyclic unsaturated heterocycle and fluoro-substituted bicyclic unsaturated heterocycle. In a further embodiment, R² is chosen from optionally substituted phenyl, indole, benzodioxole, benzoxadiazole, benzodioxan, benzimidazole, oxadiazole, pyrazole, pyridine and pyridine N-oxide. In a further embodiment, R² is chosen from meta-substituted phenyl, indole, benzodioxole, 2,2-difluorobenzodioxole, benzooxadiazole, benzimidazole, 5-(pyridin-4-yl)[1,2,4]oxadiazole, 5-(pyridin-4-yl)[1,3,4]oxadiazole, benzodioxan, 4-chloropyrazole, 4-(pyridin-4-yl)pyrazole, 6-chloropyridine, 3-(trifluoromethyl)pyrazole, and pyridine N-oxide.

In another embodiment, R² is substituted phenyl: 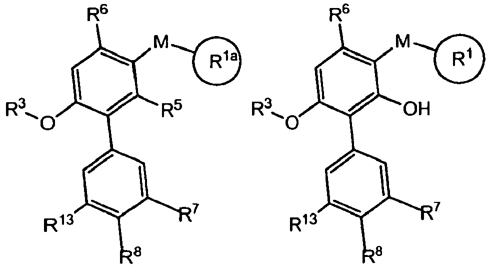 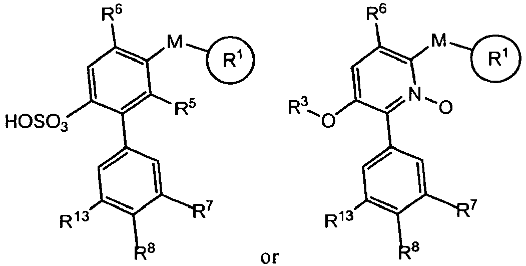 wherein R⁷ is chosen from hydrogen, halogen, nitro, cyano, halo(C₁-C₆)alkyl, hydroxy, (C₁-C₆)alkoxy, (C₁-C₆)oxaalkyl, carboxy, (C₁-C₆)alkoxycarbonyl, aminocarbonyl (-CONH₂), (C₁-C₆)alkylaminocarbonyl, acyl, hydroxy(C₁-C₆)alkyl, halo(C₁-C₆)alkoxy, amino(C₁-C₆)alkyl, ,amino, (C₁-C₆)alkylamino, di[(C₁-C₆)alkyl]amino, mercapto, (C₁-C₆)alkylthio, (C₁-C₆)alkylsulfinyl, (C₁-C₆)alkylsulfonyt, (C₁-C₆)alkylsulfonamido, acylamino, amidino, phenyl, benzyl, hetercyclyl, phenoxy, benzyloxy, and heteroaryloxy; and R⁸ and R¹³ are chosen independently from H and F. In a further embodiment, R⁸ and R¹³ are H and R⁷ is chosen from hydrogen, fluoro, chloro, bromo, nitro, cyano, acetyl, trifluoromethyl, methoxy, trifluoromethoxy, oxadiazolyl, tetrazolyl, methylthio, methanesulfinyl, methanesulfonyl, methansulfonamido, amino, methoxymethyl, hydroxyethyl, and morpholinyl.

In embodiments of formulae Ib and Ic, R¹ may be chosen from optionally substituted phenyl, optionally substituted five membered heteroaryl, optionally substituted six-membered heteroaryl, optionally substituted 4-7 membered non-aryl heterocycle, and optionally substituted fused bicycle. For example, R¹ may be chosen from optionally substituted phenyls; optionally substituted five membered heteroaryls selected from thiazoles, thiadiazoles, pyrazoles, oxadiazole, isoxazoles, triazoles, imidazoles, thiophenes, tetrazoles and oxazoles; optionally substituted six membered hereroaryls selected from pyridines, pyrimidines, pyridazinones, pyrimidinone, pyridinone, pyrazines and diazines; optionally substituted 5-and 6- membered non-aryl heterocyclics selected from tetrahydrothiophenes, piperazine, oxazolidinones, imidazolidinones, morpholines, piperidines, pyrrolidinones, pyrrolidinediones, pyrrolidines, piperidinones, piperidinediones and trioxa-bicyclo[2.2.2]octanes; and optionally substituted fused bicycles selected from benzoxazolones, indoles, isoindolinediones, 2H-pyrrolopyridinediones, purines, indolinediones, triazolopyridinones, benzimidazoles, benzoxadiazoles, quinolines and quinolones; wherein the substituents are chosen independently from hydrogen, halogen, halo(C₁-C₆)alkyl, hydroxyl, (C₁-C₆)alkoxy, carboxy, (C₁-C₆)alkoxycarbonyl, aminocarbonyl (-CONH₂), (C₁-C₆)alkylaminocarbonyl, cyano, carbonyl (oxo), acyl, hydroxy(C₁-C₆)alkyl, hato(C₁-C₆)alkoxy, amino(C₁-C₆)alkyl, (C₁-C₆)alkoxy(C₁-C₆)atkyl, nitro, amino, (C₁-C₆)alkylamino, di[(C₁-C₆)alkyl]amino, mercapto, (C₁-C₆)alkylthio, sulfoxide, sulfone, sulfonate, sulfonimide, acylamino, amidino, phenyl, benzyl, heteroaryl, phenoxy, benzyloxy, heteroaryloxy, aminocarbonyl(C₁-C₆)alkyl, (C₁-C₆)alkoxycarbonyl(C₁-C₆)alkyl, carboxy(C₁-C₆)alkyl, formylamino(C₁-C₆)alkyl, carboxy(C₁-C₆)alkylamino, - (CH₂)ₚ-NR¹²CO-(CH₂)_{q}-NR⁹R¹⁰, -NHSO₂R¹¹, -OCH₂CH₂NR⁹R¹⁰ -NHSO₂NR⁹R¹⁰, - SO₂NR⁹R¹⁰, -(CH₂)ₚ-NHCOR⁹, OCONR⁹R¹⁰ and NR¹²COOR¹¹;
R³ is chosen from -CH₃, -CH₂CH₃, -CF₃, -CHF₂ and -CH₂F;
R⁵ is chosen from H, -F, -OH, -CH₃, -OCH₃, -CF₃, -CN, -NH₂ and -C≡CH;
R² is
   (a) phenyl and R⁷ is chosen from H, halogen, nitro, acetyl, hydroxyethyl, -NH₂, - SCH₃, methoxycarbonyl, -SOCH₃, -SO₂CH₃ -OCH₃, -OCF₃, -CN, -CF₃, -CH₂OCH₃ ; or
   (b) benzoxadiazole, benzodioxole, 2,2-difluorobenzodioxole, benzoxadiazole, benzodioxan, benzimidazole, oxadiazole, pyrazole, pyridine and pyridine N-oxide;
R⁹ is chosen from H, (C₁-C₆)alkyl, halo(C₁-C₆)alkyl, (C₁-C₆)alkoxycarbonyl, carboxy(C₁-C₆)alkyl, (C₁-C₆)alkoxycarboxy(C₁-C₆)alkyl;
R¹⁰ is H, (C₁-C₆)alkyl, or taken together, or
R⁹ and R¹⁰ together form a heterocycle optionally substituted with (C₁-C₆)alkyl; p is 0 or 1,
q is 0, 1 or 2,
R¹¹ is linear (C₁-C₆)alkyl,
R¹² is H or (C₁-C₆)alkyl; or
two adjacent substituents together form an optionally substituted fused heterocyclic ring.

In embodiments of formulae Ib and Ic, R^{1a} may be chosen from substituted phenyl; substituted five membered heteroaryls selected from thiazoles, thiadiazoles, pyrazoles, oxadiazole, isoxazoles, triazoles, imidazoles, thiophenes, tetrazoles and oxazoles; substituted six membered heteroaryls selected from pyridines, pyrimidines, pyridazinones, pyrimidinone, pyridinone, pyrazines and diazines; substituted 5-and 6-membered non-aryl heterocyclics selected from tetrahydrothiophenes, piperazine, oxazolidinones, imidazolidinones, morpholines, piperidines, pyrrolidinones, pyrrolidinediones, pyrrolidines, piperidinones, piperidinediones and trioxa-bicyclo[2.2.2]octanes; and substituted fused bicycles selected from benzoxazolones, indoles, isoindolinediones, 2H-pyrrolopyridinediones, purines, indolinediones, triazolopyridinones, benzimidazoles, benzoxadiazoles, quinolines and quinolones. In these cases the substituents are chosen independently from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂ -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH and 5-tetrazolyl;
R³ is chosen from -CH₃, -CH₂CH₃, -CF₃, -CHF₂ and -CH₂F;
R⁵ is chosen from H, -F, -OH, -CH₃, -OCH₃, -CF₃, -CN, -NH₂ and -C≡CH;
R² is
   (a) phenyl and R⁷ is chosen from H, halogen, nitro, acetyl, hydroxyethyl, -NH₂, - SCH₃, methoxycarbonyl, -SOCH₃, -SO₂CH₃ -OCH₃, -OCF₃, -CN, -CF₃, -CH₂OCH₃ or
   (b) benzoxadiazole, benzodioxole, 2,2-difluorobenzodioxole, benzoxadiazole, benzodioxan, benzimidazole, oxadiazole, pyrazole, pyridine and pyridine N-oxide;
R⁹ is chosen from H, (C₁-C₆)alkyl, halo(C₁-C₆)alkyl, (C₁-C₆)alkoxycarbonyl, carboxy(C₁-C₆)alkyl, (C₁-C₆)alkoxycarboxy(C₁-C₆)alkyl;
R¹⁰ is H, (C₁-C₆)alkyl, or taken together, or
R⁹ and R¹⁰ together form a heterocycle optionally substituted with (C₁-C₆)alkyl;
p is 0 or 1,
q is 0, 1 or 2,
R¹¹ is linear (C₁-C₆)alkyl,
R¹² is H or (C₁-C₆)alkyl; or two adjacent substituents together form an optionally substituted fused heterocyclic ring.

One embodiment of compounds of the general class of Ia has the formula 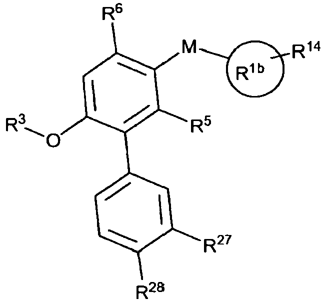 wherein
R^{1b} is phenyl, five-membered heteroaryl, six-membered heteroaryl, 4-7 membered non-aryl heterocycle or fused bicycle;
R¹⁴ is chosen from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂ -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=0)NH(CH₂)₃COOH, 5-tetrazolyl and monocyclic heterocycle substituted with any of the foregoing;
R²⁷ is chosen from hydrogen, halogen, nitro, cyano, halo(C₁-C₆)alkyl, hydroxy, (C₁-C₆)alkoxy, (C₁-C₆)oxaalkyl, carboxy, (C₁-C₆)alkoxycarbonyl, aminocarbonyl (-CONH₂), (C₁-C₆)alkylaminocarbonyl, acyl, hydroxy(C₁-C₆)alkyl, halo(C₁-C₆)alkoxy, amino(C₁-C₆)alkyl, ,amino, (C₁-C₆)alkylamino, di[(C₁-C₆)alkyl]amino, mercapto, (C₁-C₆)alkylthio, (C₁-C₆)alkylsulfinyl, (C₁-C₆)alkylsulfonyl, (C₁-C₆)alkylsulfonamido, acylamino, amidino, phenyl, benzyl, hetercyclyl, phenoxy, benzyloxy, and heteroaryloxy;
R²⁸ is chosen from H and F, or
R²⁷ together with R²⁸ forms a five-membered ring. An example of an R¹⁴ that is "a monocyclic heterocycle substituted with any of the foregoing" is example A063 below in which R¹⁴ is

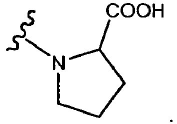

Embodiments of the general class of Ib and Ic have the formulae: 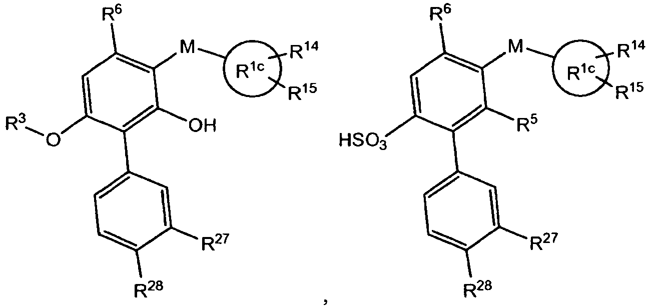 or 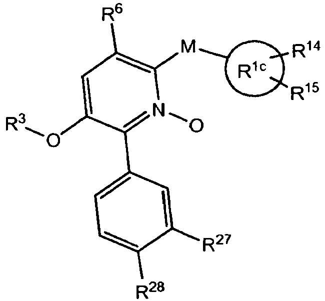 wherein
R^{1c} is phenyl, five-membered heteroaryl, six-membered heteroaryl, 4-7 membered non-aryl heterocycle or fused bicycle;
R¹⁴ is chosen from H, -CH₂NHC(=O)NH₂, -NHC(=O)NH₂, -NHC(=O)NHEt, -CH₃, -CH₂CF₃. -CH₂NHC(=O)CH₃,-NHCH₃, -NHEt, -NH(tBoc), -CHO, -NHC(=O)NHCH₂CH₂Cl, -NHSO₂NH₂ -NHEt, -N(CH₃)₂, -NH₂, -COOH, -OCH(CH₃)COOH, -SCH₂COOH, -C(=O)NH₂, -CH₂C(=O)NH₂, -CH₂COOH, -CH₂COOEt, -CN, -OCH₃, -OC(=O)NH₂, -NH(CH₃)C(=O)NH₂, halogen, -CH₂NHC(=O)OEt, -NHSO₂CH₃ -N(SO₂CH₃)₂ -NHC(=O)OCH₃, -OH, -CH₂NHC(=O)N(CH₃)₂, -CH₂NH₂, -CH₂OH -CH₂CH₂OH -SO₂NH₂, - NHC(=O)NHCH₂COOH, -CH₂NHCHO, -NHC(=O)NHCH₂COOEt, -COOCH₃, , -COOEt, -NHC(=O)NH(CH₂)₃COOEt,-NHC(=O)NH(CH₂)₂COOEt, -NH(Et)C(=O)OEt, -NHC(=O)NH(CH₂)₂COOH, -CH₂NHSO₂CH₃ -OEt, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc), -OCH₂CH₂N(CH₃)₂, -OCH₂CH₂OCH₃ 3'-nitro-6-methoxybiphenyl-3-ylmethyl, tetrahydroimidazol-2-on-1-yl, 3 -methyltetrahydroimidazol-2-one- I -yl, pyrazol- 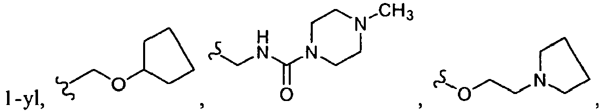 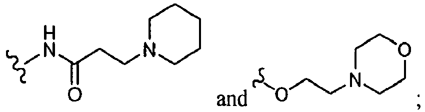
R¹⁵ is chosen from H, NO₂, OH, NH₂, and -NHSO₂NH; or
R¹⁵ together with R¹⁴ forms methylene dioxy;
R²⁷ is chosen from hydrogen, halogen, nitro, cyano, halo(C₁-C₆)alkyl, hydroxy, (C₁₋ C₆)alkoxy, (C₁-C₆)oxaalkyl, carboxy, (C₁-C₆)alkoxycarbonyl, aminocarbonyl (-CONH₂), (C₁-C₆)alkylaminocarbonyl, acyl, hydroxy(C₁-C₆)alkyl, halo(C₁₋ C₆)alkoxy, amino(C₁-C₆)alkyl, ,amino, (C₁-C₆)alkylamino, di[(C₁-C₆)alkyl]amino, mercapto, (C₁-C₆)alkylthio, (C₁-C₆)alkylsulfinyl, (C₁-C₆)alkylsulfonyl, (C₁-C₆)alkylsulfonamido, acylamino, amidino, phenyl, benzyl, hetercyclyl, phenoxy, benzyloxy, and heteroaryloxy;
R²⁸ is chosen from H and F, or
R²⁷ together with R²⁸ forms a five-membered ring.

In further embodiments R²⁷ and R²⁸ represent a fused heterocycle at 3- and 4-positions so that the residue formed from R²⁷ and R²⁸ together with the phenyl to which they are attached is chosen from:

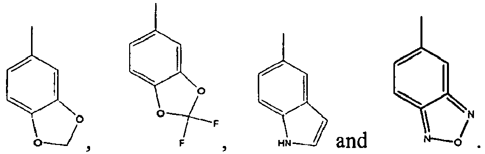

In other embodiments, R²⁷ is chosen from halogen, nitro, acetyl, hydroxyethyl, amino, methylthio, trifluoromethyl, methoxymethyl, methoxycarbonyl, trifluoromethoxy, cyano and 1,3,4-thiadiazol-2-yl, or taken together R⁷ and R⁸ are methylenedioxy or difluoromethylenedioxy. In the foregoing embodiments, R^{1a} may be chosen from a benzene ring, a triazole, a pyridine or pyridine-N-oxide, a pyrazole, a tetrahydrothiophene, an imidazole, a pyrimidine, a thiadiazole, and an imidazopyridine.

In an embodiment of the invention, R⁵ is fluoro, H, CN or OH. In other embodiments, R³ is methyl or fluoromethyl.

Another embodiment of compounds of the invention have the formula 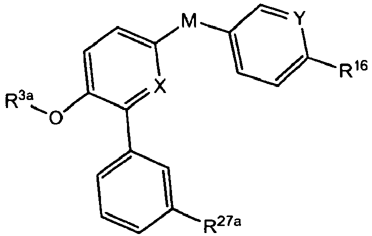 In these compounds R^{3a} is methyl, fluorinated methyl or HSO₃; Y is CH or N→O, R^{27a} is chosen from halogen, cyano, acetyl, methylthio, nitro and trifluoromethyl; and R¹⁶ is chosen from -NR¹⁷ C(=O)NR¹⁸R¹⁹ and

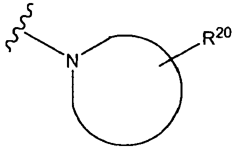

wherein 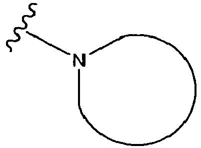 is a 4-7 membered ring heterocycle attached through its nitrogen; R¹⁷, and R¹⁸ are independently chosen from H, (C₁-C₆)alkyl and halo(C₁-C₆)alkyl; R¹⁹ is chosen from H, (C₁-C₆)alkyl, halo(C₁-C₆)alkyl, -[(C₁-C₆)alkyl]COOH, and -[(C₁-C₆)alkyl]COO(C₁-C₆)alkyl; and R²⁰ is chosen from a carboxylic acid, a carboxamide, a carboxylic ester, a primary, secondary or tertiary alcohol and a primary, secondary or tertiary amine. Examples of a carboxylic acid, a carboxamide, a carboxylic ester, a primary, secondary or tertiary alcohol and a primary, secondary or tertiary amine include -COOH, -CONH₂, -CONHCH₃, -CON(CH₃)₂, -COOCH₃, -CH₂OH -CH(CH₃)OH, -C(CH₃)₂OH-CH₂NH₂, -CH(CH₃)NH₂ and -C(CH₃)₂NH₂.

All of the compounds falling within the foregoing parent genera Ia, Ib and Ic are useful as peripheral PDE4 inhibitors. It may be found upon examination that species and genera not presently excluded arc not patentable to the inventors in this application because they have been disclosed. In this case, the exclusion of species and genera in applicants' claims are to be considered artifacts of patent prosecution and not reflective of the inventors' concept or description of their invention. The invention, in a composition aspect, is all active compounds of formulae Ia, Ib and Ic except those that are in the public's possession.

In general, the compounds of the present invention may be prepared by the methods illustrated in the general reaction schemes as, for example, described below, or by modifications thereof, using readily available starting materials, reagents and conventional synthesis procedures.

Generally compounds of the Formula I, where R² is a substituted aryl / heteroaryl and the two biaryl groups are linked by a C-C bond, may be prepared from appropriately functionalized alkoxy-aryl ether derivatives containing desirable functionalities W, where W may for example be CH, N, COH, CF, etc (Route A, Scheme A1). The biaryl portion can be constructed first, typically via Suzuki or Stille coupling (G 1 -> G2). In such case either Y = halogen or OSO2R (OTf, ONf) and the other reagent would be R2-B(OR)2 or R2-SnR3' or vise versa, where R2-halogen is coupled with G1 containing boronate/boronic acid or trialkyltin as Y. When A is a carbon derived substituent, e.g. CH3, CH2OH, CO2R", CN etc. these groups are converted to provide intermediate G3 where D is either a halogen or OTf, ONf, or OCOOR" (carbonate) such that substituent (R1) is introduced by employing a transition-metal catalyzed coupling reactions such as Suzuki, Stille or Negishi reaction. An alternative route to compounds of type G4 involves essentially reversing the order of incorporation of R1 and R2 fragments. The route B, as highlighted in Scheme A1, allows formation of G6, where the R2 fragment is introduced at later stage in the sequence, analogous to chemistries employed for G1-G2, for examples Suzuki, Stille coupling.

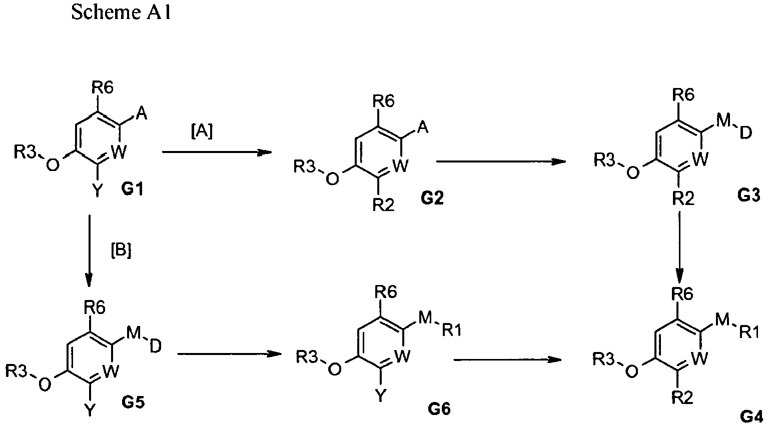

One may attach R1, which may be aryl, heterocyclic, acyclic, aliphatic, or any other desirable variety of functionality, to the central aromatic ring (Ar) by a wide rage of tether groups M. The central aromatic ring (Ar) may be a biaryl ring system with a R2 group already attached, or the R2 group may be attached subsequent to that of R1. The linker group M may be a linear chain of one or more atoms consisting of C, N, O, or S. The linker group M may also consist of functionalities including, but not limited to amide, sulfonamide, sulfone, or ketone. It is evident to one skilled in the art that many of these exemplified functional groups may be attached in more than one way, for example, Ar-CH2-O-R1 or Ar-O-CH2-R1. Considering those compounds with M groups such as S or O, the heteroatom may originally be in the Ar or the R 1 group. Some examples of how the two groups can then be joined are nucleophilic aromatic substitution, metal-promoted coupling, and nucleophilic displacement (Scheme A2). Chemical reactivity of the Ar and R1 groups should of course be considered when determining which partner contains the linker heteroatom, and which shall serve as the reaction partner. For example, it is well understood that in aromatic ring systems an electron-withdrawing group para- or ortho- to a leaving group (e.g. halogen) allows susceptibility of the aromatic halogen for nucleophilic displacement. Thus, Arl group containing NO2, CO2R, ketone, CN etc. would allow formation of aryl-M-aryl(heteroaryl) intermediates. The linker group M may also be subject to further elaboration. For example, sulfides may be oxidized to sulfoxides and sulfones and amines may be subjected to alkylation or reductive amination. Well known synthetic transformations can be used to create tether groups M such as ether amide, sulfonamide, and the like. The functional group location in the precursor Ar and R 1 groups can be used to dictate the nature and type of the linkage (e.g. alternative ethers), as mentioned above.

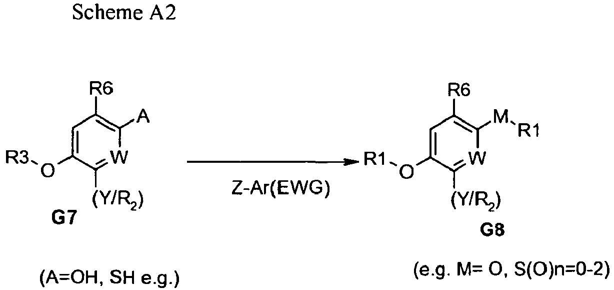

The key fragments Ar and R1 could also be joined using non transition-metal catalyzed C-C bond forming coupling reactions. When A=H, Fridele-Crafts acylation or alkylation can be employed to combine the Ar and R1 groups. Given the chemical reactivity of the aromatic para-methoxy group in the Ar ring, Friedel-Crafts acylation would typically involve a suitably elaborated R1-COC1 (acid chloride) group to form compound G10. In this case J = CO, which can be reduced to the secondary alcohol (typically with hydride-based reducing agents). If a R'MgX or other such organometallic agent is used, the J = CO group can be transformed into the tertiary alcohol with simultaneous addition of an R' group. In another variation, the J = CO group can be converted into the imine or oxime using standard procedures and addition of an R'MgX-type reagent results in the tertiary amine derivative. If desired, the J = CO group can be reduced, using a number of well established methods, to the CH2 group (M). In another variation, when A=H, an aldehyde group can be introduced using either Vilsmeier reaction or using Lewis acid (TiCl4, BF3.OEt2 etc.) mediated reaction with dichloromethylmethyl. The aldehyde functionality can subsequently be transformed into a suitable transition-metal catalyzed coupling reaction partner. Alternatively the aldehye could be used for Wittig reaction forming olefin or CH2CH2 linkage to incorporate R1. In yet another variation, substituent "A" can be various types of carbonyl (aldehyde or ketone) or imine groups. In one example, addition of a suitably elaborated organometallic R1 group (e.g. R1-MgX) to aldehyde G9 (A = CHO) would result in G10 with J = C(H)OH. Reduction of this alcohol gives rise to G11 with M = CH2. Similar types of transformations could be employed by one skilled in the art if G9 contained A = ketone or imine.

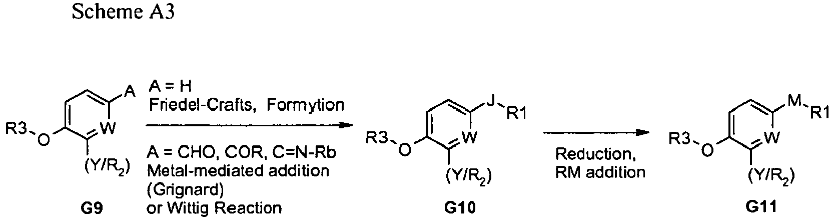

Alternatively, the C-C bond forming reaction between the Ar and R1 groups could be accomplished by displacement of a leaving group on the R1 by a nucleophile present in the tether region M (Scheme G4) of G12. The activating group could be either removed to provide Z=H (Z=CO2R - decarboxylation of or Z=CN, decyanation) or these could be further transformed to other functional groups e.g. Z = CH2)H or CH2NH2. When M-Z is CH2-halide or CH2-O-sulfonate, R1 fragment can be introduced via formation of ether linkage. This allows attachment of R1 to the central aromatic ring by spacers (M) of varying lengths and compositions. (Scheme A4)

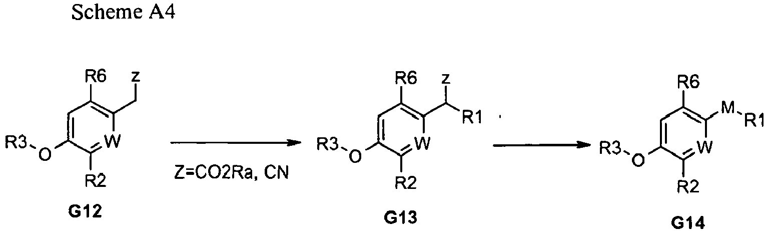

The R1 group could also be assembled form an acyclic intermediate to form a heterocylic or heteroaromatic ring. Examples of these chemistries include formation of 5-membered heteroaryls such as oxadiazolc, thiadiazole, triazole (G 17) form acyl hydrazide (G16); thiazole from 2-halo-ketone or dipolar cycloaddition reactions from olefin or acetylic group to form 5-membered heterocycles or 5-membered heteroaryls (G18) [Scheme A5]. Alternatively the 6-membered heteroaryl or heterocyclic rings could be formed using Diels-Alder or hetcro- Diels-Alder chemistries using appropriately substituted alkyl aryl ether bearing either a dienophile or a diene functionalities. The necessary acyclic precursors could be synthesized by standard methods according to previously described intermediates (e.g. aldehyde, alkyl halide) schemes.

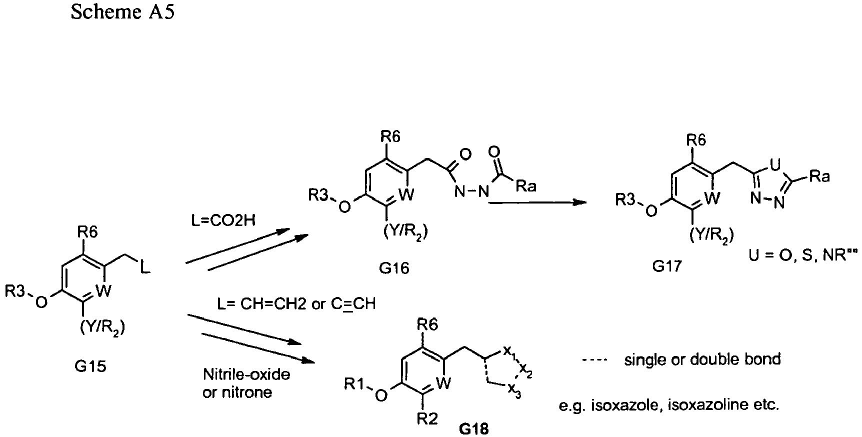

When the R2 group is linked to the Ar group thru a heteroatom (N), these biaryl systems could be prepared by organometallic mediated aza-coupling reactions or other nucleophilic aromatic substitution-based procedures (Scheme G6). The Ar-(N)R2 biaryl may be formed from intermediate G6 where R1 group is already in place. Alternatively, the (N)R2 ring can be added to the central Ar ring first, R1 can be attached through a variety of means using approaches described in previous schemes. Examples of (HN)R2 heteroaryl or heterocyclic rings include, but not limited to, imidazole, pyrrole, pyrazole, pyrrolidine, or triazole. The R2 functional group can be fully elaborated prior to addition of the (N)R2 to the Ar, or suitably elaborated after formation of the key C-N bond.

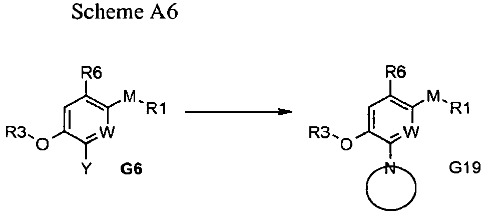

The diverse selection of substituents present in R1 and R2 could be formed by standard functional group transformations that are well know in the art. Some of these include formation of amide, sulfonamide, ureas, imidazolonc, oxazoloncs, carbamates from the R2, R3, or Ar ring fragments bearing appropriate amine, carboxylic acid, alcohol, or phenol groups. A particularly useful aromatic ring functionalization technique, in which either the R2 or R1 rings can be employed, is the nucleophilic displacement of ortho-halo N-containing aromatic rings (G20, scheme A7). Examples of ring substrates useful in this type of transformation include 2-halo-pyridine, 2-halo-pyrimidine and 2-halo-imidazole. Additionally, other leaving groups besides halogens (X) may be used such as sulfonate esters (OTf, ONf). These displacement reactions can be carried out using alkali or tertiary amine bases, or could be mediated through the use of an organometallic reagent such as palladium or aluminum reagents. Examples of nucleophiles (R") useful in this type of transformation include amines (primary, secondary, acyclic, or cyclic), alcohols, phenols, NH-containing heterocycle groups (imidazole, or pyrrazole) groups capable of performing nucleophilic displacement.

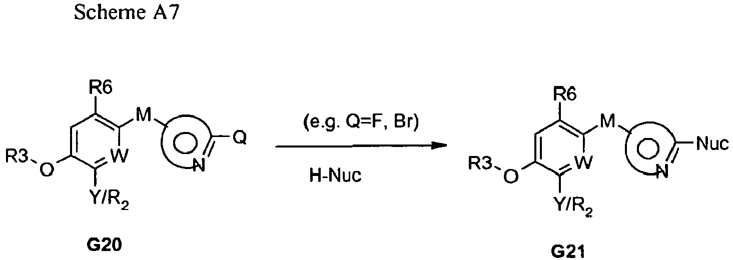

When R1 group contains additional functional groups, such as amine, ester/acid /alcohols many of which may have be masked or protected during the previous chemistries, these could be used for further functional group manipulations. A wide variety of modifications of R1 functionalities may be achieved using well established synthetic procedures including, but not limited to, alkylation, reductive amination, nucleophilic displacement, cyclization, saponification, and oxidation/reduction. Additionally, like these functional group manipulations, Arl mono-cyclic may be further transformed to a bi-cyclic ring. Examples of such ring transformations may be represented by elaboration of pyridine derivatives to imidazo[1,2-a]pyridine and imidazo[1,5-a]pyridine. These functional group manipulations and bicyclic ring elaborations may be accomplished at any chemically suitable point in the synthesis prior to or post incorporation of R2 or other synthetic transformations.

These above transformations could be carried out from alkylated phenols containing or lacking fluoro substituents in the central Ar ring. Several of these approaches are also applicable to 3-alkoxy pyridines as the Ar ring starting materials. The non-limiting specific examples described in later schemes are meant to serve as examples of the broad scope of possible reactions. Similarly, analogs where W = CH2OH, COOH, CN, CONH2 etc. (or suitably protected precursors) could be derived by following similar chemistries (schemes A1-A7) and these functional groups could be derived form an ester or amide derived starting material.

The following examples of compounds were prepared.

**Table 1.**

| | | | | | |
|---|---|---|---|---|---|
| 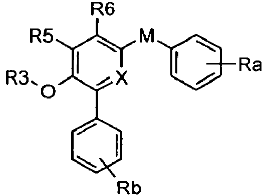 | | | | | |

| **ExNo** | **X** | **R3** | **M** | **Rb** | **Ra** |
|---|---|---|---|---|---|
| A-001 | C-H | CH3 | CH2 | 3-NO2 | 7-(Saccharine) |
| A-008 | C-F | CH3 | CH2 | 3-Cl | 4-(NHSO2NH2) phenyl |
| A-011 | C-F | CH3 | CH2 | 3-Cl | (3-OH, 4-NHSO2NH2) phenyl |
| A-016 | C-F | CH3 | CH2 | 3-COCH3 | 4-(NHSO2NE2) phenyl |
| A-018 | C-F | CH3 | CH2 | 3-COCH3 | 4-(COOCH3) phenyt |
| A-019 | C-F | CH3 | CH2 | 3-COCH3 | 4-(COOH) phenyl |
| A-022 | C-F | CH3 | CH2 | 3-Br | 4-(NHCONHCH2COOH) phenyl |
| A-023 | C-F | CH3 | CH2 | 3-Br | 4-(NHCONHCH2CH2COOH) phenyl |
| A-039 | C-F | CH3 | CH2 | 3-Cl | 6-{imidazo[1,2-a]pyridine-2-carboxylic acid ethyl ester} |
| A-043 | C-F | CH3 | CH2 | 3-Cl | 6-{imidazo[1,2-a]pyridine-2-carboxylic acid} |
| A-051 | C-F | CH3 | CH2 | 3-Cl | 6-{imidazo[1,5-a]pyridine-2-carboxylic acid} |
| A-062 | SO3-Na+ | CH3 | CH2 | CH2 | 4-(NHCONH2) phenyl |
| A-064 | C-H | CH3 | CO | 3-Cl | 4-(NHCONHCONH2) phenyl |
| A-065 | C-H | CH3 | CD(OH) | 3-Cl | 4-(NHCONHCONH2) phenyl |
| B-080 | N-O | CH3 | CH2 | 3-NO2 | 4-F-phenyl |
| B-084 | CH | CH3 | CH2 | * | 4-F-phenyl |

| | | | | | |
|---|---|---|---|---|---|
| * Rb-phenyl is replaced by 3-pyridyl N-oxide | | | | | |

**Table 2.**

| | | | | | | | | | | |
|---|---|---|---|---|---|---|---|---|---|---|
| 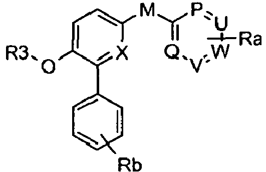 | | | | | | | | | | |

| **ExNo** | **X** | **R3** | **M** | **Rb** | **P** | **Q** | **U** | **V** | **W** | **Ra** |
|---|---|---|---|---|---|---|---|---|---|---|
| A-010 | C-F | CH3 | CH2 | 3-Cl | CH | CH | N | CH | C | (W)-NHSO2NH |
| A-012 | C-F | CH3 | CH2 | 3-Cl | CH | CH | N | N | C | (W)-NHSO2N |
| A-013 | C-F | CH3 | CH2 | 3-COCH3 | CH | CH | N | CH | C | (W)-NHSO2NH2 |
| A-014 | C-F | CH3 | CH2 | 3-Cl | CH | CH | CH | N-O | C | (W)-NHCONH2 |
| A-015 | C-F | CH3 | CH2 | 3-Cl | CH | CH | N | N-O | C | (W)-NHCONH2 |
| A-017 | C-F | CH3 | CH2 | 3-COCH3 | CH | CH | N | N | C | (W)-NHSO2NH2 |
| A-020 | C-F | CH3 | CH2 | 3-COCH3 | CH | CH | N | CH | C | (W)-CO2CH3 |
| A-021 | C-F | CH3 | CH2 | 3-COCH3 | CH | CH | N | CH | C | (W)-CO2H |
| A-028 | C-F | CH3 | CH2 | 3-Cl | CH | CH | N | CH | C | (W)-NHCONCH2CO2H |
| A-029 | C-F | CH3 | CH2 | 3-Cl | CH | CH | N | CH | C | (W)-NHCONHCH2CH2CO2Et |
| A-032 | C-F | CH3 | CH2 | 3-Cl | CH | CH | N | CH | C | (W)-NHCONHCH2CH2CO2H |
| A-033 | C-F | CH3 | CH2 | 3-Cl | CH | CH | N | CH | C | (W)-CO-N(Piperidine-4-CO2Et) |
| A-034 | C-F | CH3 | CH2 | 3-Cl | CH | CH | N | CH | C | (W)-CO-N(Piperidine-4 - COOH) |
| A-035 | C-F | CH3 | CH2 | 3-Cl | CH | CH | N | CH | C | (W)-NHCH2CO2H |
| A-036 | C-F | CH3 | CH2 | 3-Cl | CH | CH | N | CH | C | (W)-OH |
| A-037 | C-F | CH3 | CH2 | 3-Cl | CH | CH | N | CH | C | (W)-N(COCH3)CH2CO2H |
| A-038 | C-F | CH3 | CH2 | 3-Cl | CH | CH | N | CH | C | (W)-CO-IN-pyrrolidine-3-CO2H) |
| A-040 | C-F | CH3 | CH2 | 3-Cl | CH | CH | N | N | C | (W)-1N-(S')-Protine |
| A-041 | C-F | CH3 | CH2 | 3-Cl | CH | CH | N | N | C | (W)-1N-(piperidine-3-CO2H) |
| A-042 | C-F | CH3 | CH2 | 3-Cl | CH | CH | N | CH | C | (W)-1H-5-tetrazolyl |
| A-044 | C-F | CH3 | CH2 | 3-Cl | CH | CH | N | N | C | (W)-1N-(pyrrolidine-3-CO2H) |
| A-045 | C-F | CH3 | CH2 | 3-Cl | CH | CH | N | N | C | (W)-1N-(pyrrolidine-3-CO2Me |
| A-047 | C-F | CH3 | CH2 | 3-Cl | CH | CH | N | N | C | (W)-sarcosine |
| A-048 | C-F | CH3 | CH2 | 3-Cl | CH | CH | CH | N | C | (W)-CH2NHCOCO2Et |
| A-049 | c-F | CH3 | CH2 | 3-Cl | CH | CH | N | N | C | (W)-N(azetidine-(W)-COOH) |
| A-050 | C-F | CH3 | CH2 | 3-Cl | CH | CH | CH | N | C | (W)-CONHSO2CH3 |
| A-053 | C-F | CH3 | CH2 | 3-Cl | CH | CH | N | N | C | (W)- N(azetidine-4-COOH) |
| A-054 | C-F | CH3 | CH2 | 3-Cl | CH | CH | CH | N | C | (W)- N(azetidine-2-COOH) |
| A-056 | C-F | CH3 | CH2 | 3-Cl | CH | CH | CH | N | C | (W)- N(CH2-COOH)-CONHEt |
| A-057 | C-F | CH3 | CH2 | 3-Cl | CH | CH | CH | N | C | (W)- N(CH2-COOH)-CONH2 |
| A-061 | C-F | CH3 | CH2 | 3-Cl | CH2 | CH | CH | N | C | (W)- N(azetidine-(S)-2-COOH) |
| A-063 | C-F | CH3 | CH2 | 3-Cl | CH | CH | CH | N | C | (W)- N(R)-Proline |
| A-066 | C-F | CH3 | CH2 | 3-Cl | CH | CH | CH | N | C | (W)- N(azetidine-(R)-2-COOH) |
| A-067 | C-H | CH3 | CH2 | 3-Cl | CH | CH | CH | N | C | (W)- N(azetidine-2-COOH) |
| A-068 | C-H | CH3 | CH2 | 3-Cl | CH | CH | CH | N | C | (W)-N(R)-Proline |
| A-069 | C-F | CH3 | CH2 | 3-Cl | CH | CH | CH | N | C | (W)- N(R)-Proline methyl ester |
| A-070 | C-F | CH3 | CH2 | 3-Cl | CH | CH | CH | N | C | (W)-N(3-pipecolic acid) |
| A-071 | C-H | CH3 | CH2 | 3-Cl | CH | CH | CH | N | C | (W)-COOH |
| A-072 | C-F | CH3 | CH2 | 3-Cl | CH | CH | CH | N | C | (W)-N(S)-Proline |
| A-073 | C-H | CH3 | CH2 | 3-Cl | CH | CH | CH | N | C | (W)-COOCH3 |
| A-074 | C-F | CH3 | CH2 | 3-Cl | CH | CH | CH | N | C | (W)-N(CH3)-CH2COOH |
| A-075 | C-H | CF2 H | CH2 | 3-Cl | CH | CH | CH | N | C | (W)-COOCH3 |
| A-076 | C-H | CF2 H | CH2 | 3-Cl | CH | CH | CH | N | C | (W)-Tetrazole |
| A-077 | C-H | CH3 | CH2 | 3-Cl | CH | CH | CH | N | C | (W)-N(CH3)-CH2COOH |
| A-078 | C-H | CF2 H | CH2 | 3-Cl | CH | CH | CH | N | C | (W)-N(CH3)-CH2COOH |
| A-079 | C-H | CF2 H | CH2 | 3-Cl | CH | CH | CH | N | C | (W)-N(CH3)-(CH3)-COOH |
| B-081 | CF | CH3 | CH2 | 3-Cl | CH | CH | NO | CH | C | (W)-NHCONHEt |
| B-082 | CF | CH3 | CH2 | 3-Cl | CH | CH | NO | CH | C | (W)-CH2-NHCONHEt |
| B-083 | CF | CH3 | CH2 | 3-Cl | CH | CH | NO | CH | C | (W)-CH2-(1-imidazolidin-2-one) |

**Table 3.**

| | | | | | | | | | |
|---|---|---|---|---|---|---|---|---|---|
| 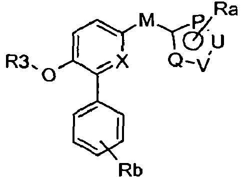 | | | | | | | | | |

| **ExNo** | **Cor e** | **R3** | **M** | **Rb** | **P** | **Q** | **U** | **V** | **Ra** |
|---|---|---|---|---|---|---|---|---|---|
| A-006 | C-H | CH3 | CH2 | 3-NO2 | C(CH 3) | C(CH 3) | N | N | (U)-CH2COOH |
| A-024 | C-F | CH3 | CH2 | 3-Cl | S | N | C | N | (U)-NHCONHCH2COOCH2CH3 |
| A-025 | C-F | CH3 | CH2 | 3-Cl | S | N | C | N | (U)-NHCONHCH2CH2COOCH2CH3 |
| A-026 | C-F | CH3 | CH2 | 3-Cl | S | N | C | N | (U)-NHCONHCH2COOH |
| A-027 | C-F | CH3 | CH2 | 3-Cl | S | N | C | N | (U)-NHCONHCH2CH2COOH |
| A-030 | C-F | CH3 | CH2 | 3-Cl | S | N | C | N | (U)-NHCONH(CH2)3COOCH2CH3 |
| A-031 | C-F | CH3 | CH2 | 3-Cl | S | N | C | N | (U)-NHCONH(CH2)3COOH |
| A-058 | C-F | CH3 | CH2 | 3-Cl | N | NH | N | N | - |

**Table 4.**

| | | | | |
|---|---|---|---|---|
| 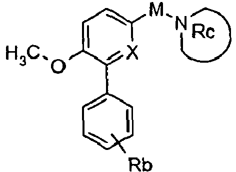 | | | | |

| **ExNo** | **X** | **M** | **Rb** | **N(Rc)** |
|---|---|---|---|---|
| A-002 | C-H | CH2 | 3-NO2 | N-Saccharin |
| A-003 | C-H | CH2 | 3-NO2 | 9N-2-Amino-6-chloropurine |
| A-004 | C-H | CH2 | 3-NO2 | 9N-Guanine |
| A-005 | C-H | CH2 | 3-NO2 | N-L-4-Hydroxyproline methyl ester |
| A-007 | C-H | CH2 | 3-NO2 | 1N-Pyridin-2-one-4-carboxylic acid |
| A-059 | C-F | CH2 | 3-Cl | 1N-Imidazole4-carboxylic acid |
| A-060 | C-F | CH2 | 3-Cl | 1N-Imidazole-5-carboxylic acid |

In-vitro assay for PDE4 enzymes. The in-vitro activity of PDE4 enzymes and the in-vitro potency of therapeutic agents described in the present invention was measured using a real-time, enzyme-coupled spectrophotometric assay. By using three different coupling enzymes, the product of the PDE4 reaction is coupled to the oxidation of the reduced form β-nicotinamide adenine dinucleotide (NADH), which dissipation can be monitored spectrophotmetrically at 340 nM.

Assay description. Buffer A containing 50 mM Tris, pH 8.0, 16 mM MgCl₂ and 80 mM KCl is prepared and stored at room temperature. Buffer B containing 50 mM Tris, pH 8.0 is prepared and stored at toom temperature. Stock solutions of the following reagents are prepared in Buffer B and stored at -20°C: Adenosine-5'-triphosphate (ATP), cyclic adenosine-5'-monophosphate (cAMP), phosphoenolpyruvate (PEP) and NADH. An assay mix is prepared by mixing Buffer A, trichloroethylphosphine (TCEP), ATP, PEP, NADH, myokinase (MK), pyruvate kinase (PK), lactate dehydroganese (LDH) and PDE4 to a final volume of 20 mL, which is enough for a single 96-well assay plate. Assay mix (180 µL) and test article (10 µL) in 1:1 DMSO/H2O mixture is preincubated at room temperature for 10 min. The enzymatic reaction is initiated by addition of cAMP (10 µL). Final concentration of all components in the assay (200 µL/well) are as follows: 10 mM MgCl₂, 50 mM KCl, 5 mM TCEP, 2.5% DMSO, 0.4 mM NADH, 1 mM PEP, 0.04 mM ATP, 5 units MK, 1 unit PK, 1 unit LDH and appropriate amount of PDE4. Reaction progress curves are monitored in a plate reader capable of measuring light absorbance at 340 nM. A decrease in light absorbance at 340 nm is due to oxidation of NADH. Positive controls containing no test article and negative controls containing no test article and no cAMP are included on every assay plate. Reaction rates are determined from the slopes of the linear portions of the progress curves. All data is percent normalized with respect to controls and presented as percent inhibition.

The results of testing of representative species are shown below in Tables A and B. The activities are designated A = < 5 µM, B = 5-20 µM, C = 20-40 µM.

**Table A.**

| **CmpdNo** | **hPDE4D** | | **CmpdNo** | **hPDE4D** | | **CmpdNo** | **hPDE4D** |
|---|---|---|---|---|---|---|---|
| A-001 | A | | A-026 | A | | A-051 | A |
| A-002 | B | | A-027 | A | | A-053 | A |
| A-003 | A | | A-028 | A | | A-054 | A |
| A-004 | B | | A-029 | A | | A-056 | A |
| A-005 | C | | A-030 | A | | A-057 | A |
| A-006 | A | | A-031 | A | | A-058 | B |
| A-007 | B | | A-032 | A | | A-059 | A |
| A-008 | A | | A-033 | A | | A-060 | A |
| A-010 | A | | A-034 | A | | A-061 | A |
| A-011 | A | | A-035 | A | | A-062 | A |
| A-012 | A | | A-036 | A | | A-063 | A |
| A-013 | A | | A-037 | A | | A-064 | B |
| A-014 | A | | A-038 | A | | A-065 | A |
| A-015 | A | | A-039 | A | | A-066 | A |
| A-016 | A | | A-040 | A | | A-067 | A |
| A-017 | A | | A-041 | A | | A-068 | A |
| A-018 | A | | A-042 | A | | A-069 | A |
| A-019 | A | | A-043 | A | | A-070 | A |
| A-020 | A | | A-044 | A | | A-071 | A |
| A-021 | B | | A-045 | A | | A-072 | A |
| A-022 | A | | A-047 | A | | A-073 | A |
| A-023 | A | | A-048 | A | | A-074 | A |
| A-024 | A | | A-049 | A | | A-075 | A |
| A-025 | A | | A-050 | A | | A-076 | A |
| A-077 | A | | A-078 | A | | A-079 | A |
| B-080 | A | | B-081 | A | | B-082 | A |
| B-083 | A | | B-084 | B | | | |

**Table B.**

| **Cmpd No** | **hPDE4B** | | **Cmpd No** | **hPDE4B** | | **Cmpd No** | **hPDE4B** |
|---|---|---|---|---|---|---|---|
| A-001 | C | | A-028 | A | | A-052 | A |
| A-003 | B | | A-030 | B | | A-053 | B |
| A-004 | C | | A-031 | B | | A-054 | A |
| A-006 | B | | A-032 | B | | A-055 | A |
| A-007 | B | | A-034 | C | | A-057 | A |
| A-008 | A | | A-035 | A | | A-058 | C |
| A-010 | A | | A-036 | A | | A-059 | B |
| A-011 | B | | A-037 | A | | A-060 | B |
| A-012 | A | | A-038 | A | | A-061 | B |
| A-013 | A | | A-040 | A | | A-063 | A |
| A-014 | B | | A-041 | B | | A-064 | B |
| A-015 | A | | A-042 | A | | A-065 | B |
| A-016 | A | | A-043 | B | | A-066 | A |
| A-017 | A | | A-044 | C | | A-067 | B |
| A-018 | C | | A-047 | A | | A-068 | A |
| A-019 | B | | A-048 | A | | A-070 | B |
| A-020 | A | | A-049 | A | | A-073 | A |
| A-021 | B | | A-050 | A | | A-074 | A |
| A-027 | C | | A-051 | A | | A-075 | A |
| A-076 | A | | B-080 | A | | B-081 | A |
| B-082 | A | | B-083 | A | | | |

The activity of PDE4 inhibitors decribed in the present invention was also measured using in an ex-vivo assay measuring leukotriene E4 (LTE4) in human whole blood after Sephadex stimulation. The anti-inflammatory activity of therapeutic agents of the present invention is demonstrated by the inhibition of eosinophil activation as measured by sephadex bead stimulated LTE4 production in whole human blood. For each sample, 356 µl of heparinized human whole blood (Vacutainer tube #6480) is added to wells of a 96 well plate. Then, 4 µl of a series of compound dilutions (in DMSO) are added in triplicatcs, suspension mixed and allowed to incubate at 37°C for 15 min with gentle shaking. After that, blood samples are stimulated by adding 40 µL of Sephadex G-15 beads (Sigma-Aldrich, Sweden). The beads are predissolved in PBS (0.16 g/ mL PBS). After mixing, the suspension is incubated at 37 °C for 90 min. Then, 8 µL of 15% EDTA/PBS is added to each sample, mixed and plate centrifuged for 5 min at 115 x g at 21°C and supernatants taken. In each plate, 10 positive controls and 10 negative controls are used, containing DMSO instead of compound solution. The positive controls are stimulated with Sephadex as described for the samples, and in the negative controls (unstimulatcd), Sephadex solution is replaced by PBS. LTE₄ levels in the resulting plasma samples are determined using a commercial enzyme-linked immunoassay (Cayman Chemical Company, Ann Arbor, MI) according to the manufacturer's instructions. Examples A-008, A-016, A-035, A-037, A-054, A-066 and B-080 showed IC50 < 1 µM in this ex-vivo assay, whereas A-028 and A-055 gave IC50 > 1 µM. Persons of skill in the art accept that positive results in PDE4 models are predictive of therapeutic utility as discussed above.

Syntheses of examples of the invention are shown below:

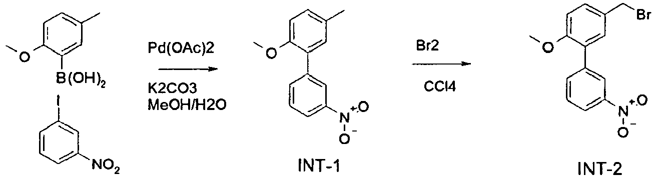

### 2-Methoxy-5-methyl-3'-nitro-biphenyl (INT-1):

A reaction mixture of 2-methoxy-5-methylphenyl boronic acid (5 g, 30.3 mmol), 3-nitro-iodobenzen (7.5 g, 30.3 mmol), K₂CO₃ (8.36 g, 60.6 mmol), Pd(OAc)₂ (339 mg, 1.52 mmol) in methanol (150 ml) and water (30 ml) was stirred at room temperature for 20 hours. The reaction mixture was diluted with ethyl acetate (600 ml) washed with diluted NaHSO₃ aq., water, brine and dried over Na₂SO₄. After removal of solvent, 6.7 g of crude product **INT-1** was obtained. Yield: 91 %.

### 5-Bromomethyl-2-methoxy-3'-nitro-biphenyl (INT-2):

To a mixture of compound **INT-1** (6.95 g, 28.5 mmol) in carbon tetrachloride (350 ml), Bromine (1.54 ml, 29.9 mmol) was added at room temperature. The reaction mixture was stirred at 80 °C under sun lamp for 2 hours with the protection of N₂. After removal of solvent, the residue was purified by 150g silica gel column chromatography with ethyl acetate /Hexane as eluent to give 5.6g of product **INT-2.** Yield: 61 %.

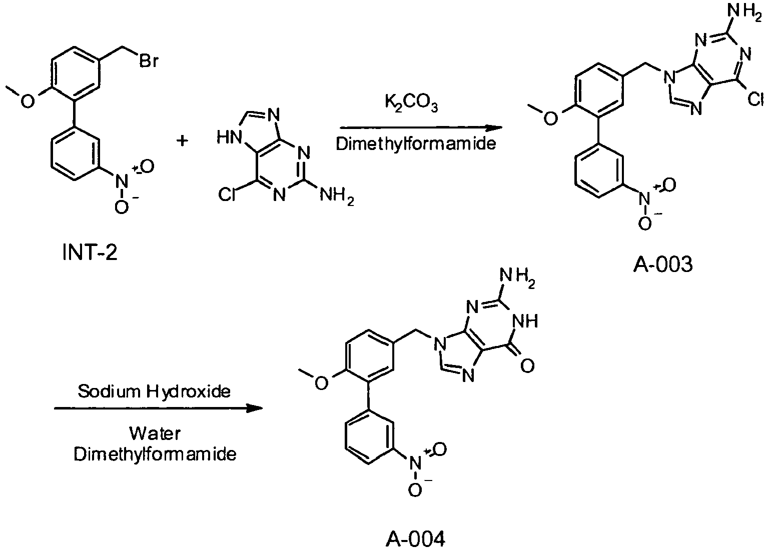

**A-003. Synthesis of 6-Chloro-9-(6-methoxy-3'-nitro-biphenyl-3-ylmethyl)-9H-purin-2-ylamine.** A suspension of **INT-2** (322 mg, 1.00 mmol), 2-amino-6-chloropurine (169 mg, 1.00 mmol), and solid potassium carbonate (276 mg, 2.00 mmol) in dimethyl formamide (10 mL) was stirred at room temperature for 18 h. The reaction mixture was poured over water (100 mL), stirrcd for 10 min, and the resulting precipitate collected. The crude material was dissolved in ethyl acetate (30 mL), dried over sodium sulfate, filtered, the solvent removed under vacuum. The crude residue was purified by trituration in hexanes/ethyl acetate (100 mL: 30 mL) several times to obtain **A-003** (58.5 mg, 14 % yield) as a yellow solid.

1H NMR (400 MHz, DMSO-d6) δppm 3.78 (s, 3 H) 5.27 (s, 2 H) 6.93 (s, 2 H) 7.15 (d, J=8.45 Hz, 1 H) 7.29 - 7.43 (m, 1 H) 7.47 (d, J=2.15 Hz, 1 H) 7.62 - 7.83 (m, 1 H) 7.92 (d, J=7.65 Hz, 1 H) 8.13 - 8.41 (m, 3 H)

**A-004. Synthesis of 2-Amino-9-(6-methoxy-3'-nitro-biphenyl-3-ylmethyl)-1,9-dihydro-purin-6-one**. A solution of **A-003** (60 mg, 0.15 mmol) and anhydrous sodium hydroxide (1.2 g, 30 mmol) in water (6 mL) and dimethyl formamide (6 mL) was stirred at room temperature for 5 min. The yellow solution was quenched with the addition of water (50 mL). The resulting precipitate was collected by filtration, dissolved in ethyl acetate (10 mL), dried over sodium sulfate, filtrered, and the solvent removed under vacuum to obtain **A-004** (29.9 mg, 51 % yield) as a yellow solid.
1H NMR (400 MHz, DMSO-d6) δ 3.77 (s, 3 H) 5.19 (s, 2 H) 5.82 (s, 2 H) 6.91 (br. s., 1 H) 7.14 (d, J=8.59 Hz, 1 H) 7.30 (dd, J=8.45, 2.01 Hz, 1 H) 7.42 (d, J=2.01 Hz, 1 H) 7.71 (t, J=7.98 Hz, 1 H) 7.83 (s, 1 H) 7.91 (d, J=7.78 Hz, 1 H) 8.16 - 8.23 (m, 1 H) 8.27 (d, J=1.61 Hz, 1 H)

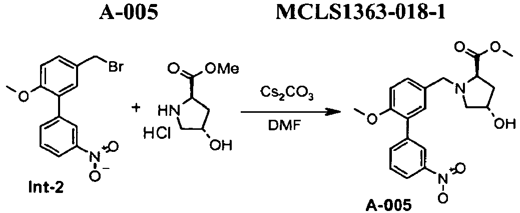

### (2R,4S)-4-Hydroxy-1-(6-methoxy-3'-nitro-biphenyl-3-ylmethyl)-pyrrolidine-2-carboxylic acid methyl ester

Into a 20 mL vial with stir bar added Int-2 (0.30 g, 0.93 mmol), (2R,4S)-4-Hydroxy-pyrrolidine-2-carboxylic acid methyl ester hydrochloride (0.62 mmol), C_{S2}CO₃ (0.20 g, 0.62 mmol), and DMF (2 mL). The reaction was stirred for 4 days at room temperature and then 10 mL water was added. The product was extracted with ethyl acetate (3 X 10 mL) and the organics were combined and conecentrated. The residue was purified by flash column chromatography using 0-5% methanol/dichloromethane to obtain 36 mg (8%) of A-005. ¹H NMR (400 MHz, CHLOROFORM-*_{d}*) δ ppm 2.03 - 2.15 (m, 1 H) 2.27 (dt, *J* =13.8, 7.1 Hz, 1 H) 2.51 (dd, *J*=10.2, 3.6 Hz, 1 H) 3.37 (dd, *J*=10.1, 5.6 Hz, 1 H) 3.57 - 3.73 (m, 5 H) 3.83 (s, 3 H) 3.90 (d, *J*=12.9 Hz, 1 H) 4.48 (br. s., 1 H) 6.96 (d, *J*=8.3 Hz, 1 H) 7.29 (d, *J*=2.0 Hz, 1 H) 7.33 (dd, *J=*8.3, 2.0 Hz, 1 H) 7.56 (t, *J=8.0* Hz, I H) 7.86 (d, *J=*7.7 Hz, 1 H) 8.17 (dd, *J=*8.2, 1.3 Hz, 1 H) 8.37 - 8.44 (m, 1 H). LC/MS = 93.7%, 387.1, (APCI+)

### A-006A [MCLS1354-035-2]

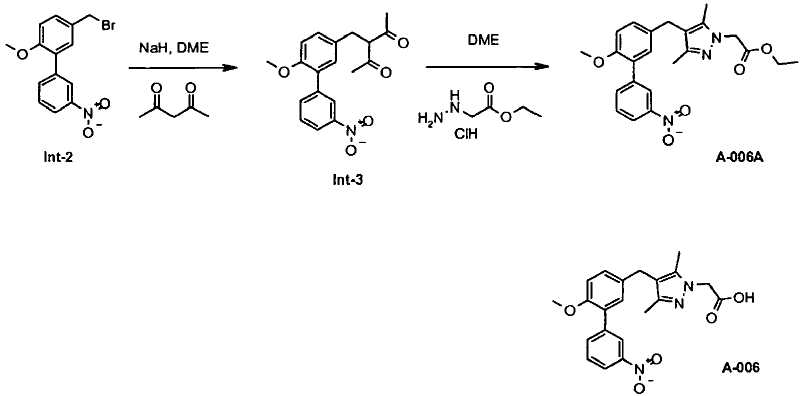

### Int-3 MCLS1354-034-2

**Synthesis of 3-(6-Methoxy-3'-nitro-biphenyl-3-ylmethyl)-pentane-2,4-dione.** To a solution of 2,4-pentadione in dimethoxyethane (5 mL) was added NaH (80 mg, 1.2 mmol). After 30 minutes at room temperature, Int-2 (322 mg, 1.0 mmol) was added and the reaction was stirred at room temperature overnight. To the reaction was added 30 mL of water and the product was extracted with 2 X 30mL EtOAc, washed with brine, dried over Na₂SO₄, and concentrated. The residue was purified by flash column chromatography eluting with 2:1 Hexanes:EtOAc to afford the title compound (106 mg, 31%).

**Synthesis of [4-(6-Methoxy-3'-nitro-biphenyl-3-ylmethyl)-3,5-dimethyl-pyrazol-1-yl]-acetic acid ethyl** ester. To a solution of **INT-3** (102 mg, 0.300 mmol) in 1,2-dimethoxyethane (5 mL), were added ethyl hydrazinoacetate hydrochloride (93 mg, 0.60 mmol) and 4 Angstrom molecular sieves (200 mg), and the reaction was stirred at reflux for 3 h. The hot suspension was filtered and the solvent removed under vacuum. The residue was dissolved in dichloromethane (10 mL) and washed with water (30 mL). The aqueous wash was extracted with dichloromethane (2 x 30 mL), and the extracts combined. The organic solution was washed with brine, dried over sodium sulfate, filtered, and the solvent removed under vacuum. The crude material was purified by silica gel column chromatography (2:1 hexanes:ethyl acetate) to give **A-006A** (97.1 mg, 76% yield).

1 H NMR (CHLOROFORM-d) d: 8.38 (t, J = 1.9 Hz, 1 H), 8.15 (dd, J = 8.2, 2.3 Hz, 1 H), 7.79 - 7.82 (m, 1 H), 7.53 - 7.56 (m, 1 H), 7.07 - 7.10 (m, 2H), 6.90 (d, J = 9.1 Hz, 1H), 4.79 (s, 2H), 4.21 (q, J = 7.1 Hz, 2H), 3.80 (s, 3H), 3.74 (s, 2H), 2.13 (s, 3H), 2.13 (s, 3H), 1.26 (t, J = 7.1 Hz, 3H). LCMS: 98.7% purity.

### A-006.

**Synthesis of [4-(6-Methoxy-3'-nitro-biphenyl-3-ylmethyl)-3,5-dimethyl-pyrazol-1-yl]-acetic acid.** To a solution **of A-006A** (105 mg, 0.25 mmol) in ethanol (1 mL) was added 2 M aqueous sodium hydroxide solution (0.625 mL, 1.25 mmol) and the reaction was stirred at 50°C for 3 h. The reaction was acidified to pH 6 with 10% aqueous hydrogen chloride solution. The suspension was extracted with diethyl ether (5 mL), dried over sodium sulfate, and concentrated en vacuo. The crude was purified by silica gel column chromatography (10% methanol in dichloromethane) to give A-006 (33.1 mg, 34% yield) as a white powder. I H NMR (CHLOROFORM-d) d: 8.32 - 8.39 (m, 1H), 8.11 - 8.19 (m, 1H), 7.80 (d, J = 7.7 Hz, 1 H), 7.49 - 7.58 (m, 1 H), 7.02 - 7.10 (m, 1H), 6.90 (d, J = 9.1 Hz, 1H), 4.77 (s, 2H), 3.80 (s, 3H), 3.73 (s, 2H), 2.16 (s, 3H), 2.15 (s, 3H). LCMS = 97.6% purity.

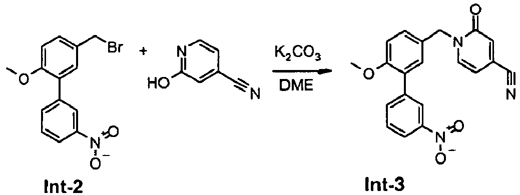

### Int-3 MCLS1363-200-1

### 1-(6-Methoxy-3'-nitro-biphenyl-3-ylmethyl)-2-oxo-1,2-dihydro-pyridine-4-carbonitrile.

Into a 100 mL RBF with stir bar was added Int-2 (2.76 g, 8.55 mmol), 2-Hydroxy-isonicotinonitrile (934 mg, 7.78 mmol), K₂CO₃ (2.36 g, 17.11 mmol), and DME (30 mL). The suspension was stirred for 18 hours at 80 °C and then RT for 2 days. The reaction was filtered, the filtrate was concentrated and the resulting solid was triturated with ether to give 2.12 g (75%) of Int-3 as a yellow solid. ¹H NMR (400MHz ,DMSO-d₆) δ = 8.29 (s, 1 H), 8.20 (dd, *J=* 1.3, 8.3 Hz, I H), 8.12 (d, *J=* 7.1 Hz, I H), 7.93 (d, J = 7.8 Hz, I H), 7.73 (t, *J=* 8.0 Hz, 1 H), 7.49 (d, J= 2.0 Hz, I H), 7.43 (dd, *J=* 1.9, 8.5 Hz, 1 H), 7.16 (d, J= 8.6 Hz, 1 H), 7.04 (d, *J= 1.3* Hz, 1 H), 6.56 (dd, *J= 1.7, 7.0* Hz, 1 H), 5.12 (s, 2 H), 3.79 (s, 3 H). LC/MS = 96.1 %, 361.0 (APCI-).

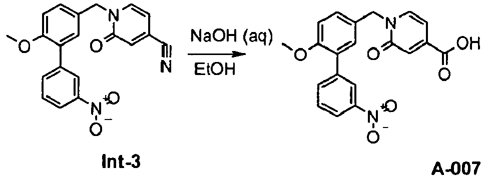

### A-007 MCLS1404-010-1

### 1-(6-Methoxy-3'-nitro-biphenyl-3-ylmethyl)-2-oxo-1,2-dihydro-pyridine-4-carboxylic acid

Into a 20 mL vial with stir bar was added Int-3 (480 mg, 1.33 mmol), 3 mL of ethanol, and 3 mL of 1 N NaOH (aq). The solution was stirred at 80 °C for 1 hour then cooled to room temperature. About half the volume of solvent was evaporated and the solution was washed twice with DCM. The remaining aqueous portion was added to a 6N HCl solution and stirred for 1 hour, after which time the solids were filtered and washed with water. The solid was dried in a vacuum dessicator for 3 days to yield 366 mg (72%) of A-007as a light-yellow solid. ¹H NMR (400MHz ,DMSO-d₆) δ= 8.28 (d, *J*= 1.6 Hz, 1 H), 8.20 (dd, *J* = 1.5, 8.1 Hz, 1 H), 7.99 (d, *J* = 7.0 Hz, 1 H), 7.93 (d, *J* = 7.8 Hz, 1 H), 7.72 *(t, J =* 7.9 Hz, 1 H), 7.48 (d, *J =* 2.0 Hz, 1 H), 7.42 (dd, *J =* 2.0, 8.5 Hz, 1 H), 7.16 (d, *J*= 8.5 Hz, 1 H), 6.87 (d, *J*= 1.6 Hz, I H), 6.57 (dd, *J*= 1.7, 7.0 Hz, 1 H), 5.13 (s, 2 H), 3.79 (s, 3 H). LC/MS = 95.9%,

### Synthesis of A-0 16

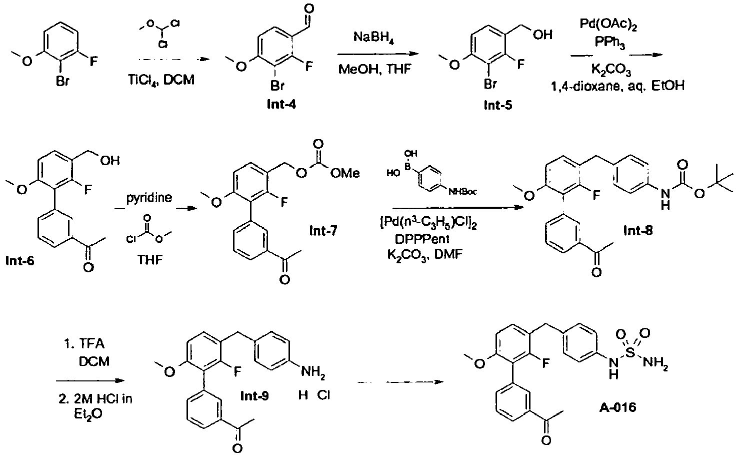

### (Int-4) [MCLS1374-115-1] Synthesis of 3-Bromo-2-fluoro-4-methoxy-benzaldehyde:

In a 3-neck 250 mL round bottomed flask equipped with nitrogen lines and a stir bar was placed 2-bromo-1-fluoro-3-methoxy-benzene (2.0 g, 9.75 mmol) and dichloromethane (48 mL). The solution was cooled in an ice water bath for 15 minutes and then titanium tetrachloride (5.02 mL, 45.8 mmol) and dichloromethyl methyl ether (1.32 mL, 14.6 mmol) were added and the reaction mixture was allowed to warm to room temperature and react for 2 hours. The reaction mixture was slowly added to ice water (250 mL) and extracted with dichloromethane (2 x 100 mL). The organic portions were combined, washed with a saturated sodium bicarbonate solution (75 mL), water (75 mL) and brine (75 mL), dried (MgSO₄) and concentrated. The crude material was triturated with hexanes (15 mL) to produce 1.67 g of Int-4 as an off-white solid in 74% yield. MS(ESI+): 233.2 (M+)

### (Int-5) [MCLS1374-119-1] Synthesis of (3-Bromo-2-fluoro-4-methoxy-phenyl)-methanol:

In a 100 mL round bottomed flask equipped with a stir bar was placed Int-4 (1.67 g, 7.17 mmol), methanol (12 mL), dichloromethane (12 mL) and sodium borohydride. The reaction mixture was allowed to stir at room temperature for 17 hours, quenched with water (10 mL) and 1M HCl (5 mL) and extracted with dichloromethane (2 x 30 mL). The organic portions were combined, washed with brine (30 mL), dried (MgSO₄) and concentrated. The crude material was triturated with hexanes (15 mL) to produce 955 mg of Int-5 as a white solid in 57% yield.

### (Int-6) MCLS1374-121-2] Synthesis of 1-(2'-Fluoro-3'-hydroxymethyl-6'-methoxy-biphenyl-3-yl)-ethanone:

In a 40 mL vial equipped with a stir bar was placed Int-5 (950 mg, 4.04 mmol), 3-acetlylphenylboronic acid (729 mg, 4.44 mmol), potassium carbonate (1.68 g, 12.1 mmol), triphenylphosphine (318 mg, 1.21 mmol), 1,4-dioxane (8 mL), 50% aqueous ethanol (8 mL) followed by palladium (II) acetate (90.7 mg, 0.404 mmol). The mixture was heated to 68 °C for 72 hours and then cooled to room temperature. The palladium catalyst was removed via filtration through Celite. To the filtrate was added 1M HCl(30 mL) and water (30 mL). The aqueous portion was extracted with ethyl acetate (2 x 30 mL), the organic portions were combined, washed with brine (30 mL), dried (MgSO₄) and concentrated. The crude material was purified by column chromatography utilizing 50% EtOAc/hexanes as the eluent to produce 614 mg of Int-6 as an off-white solid in 55% yield.

### (Int-7) [MCLS1374-123-2] Synthesis of Carbonic acid 3'-acetyl-2-fluoro-6-methoxy-biphenyl-3-ylmethyl ester methyl ester:

In an 18 mL vial equipped with a stir bar was placed Int-6 (400 mg, 1.46 mmol), anhydrous tetrahydrofuran (4.9 mL) and pyridine (154 µL, 1.90 mmol). The resulting clear solution was cooled in an ice water bath for 10 minutes and then methyl chloroformate (124 µL, 1.61 mmol) was added and reaction mixture was slowly warmed to room temperature and reacted for 17 hours. Additional pyridine (154 µL, 1.90 mmol) and methyl chloroformate (124 µL, 1.61 mmol) were introduced and the reaction mixture stirred for another 17 hours at room temperature. The reaction was acidified to pH 1 with 1M HCl, water (30 mL) was added followed by an extraction with dichlormethane (2 x 30 mL). The organic portions were combined, washed with brine (30 mL), dried (MgSO₄) and concentrated. The crude material was purified by column chromatography utilizing 40% EtOAc/hexanes as the eluent to produce 346 mg of Int-7 as a colorless viscous oil in 71% yield.

### (Int-8) [MCLS1374-131-1] Synthesis of [4-(3'-Acetyl-2-fluoro-6-methoxy-biphenyl-3-ylmethyl)-phenyl]-carbamic acid tert-butyl ester:

In an 8 mL vial equipped with a stir bar was placed Int-7 (295 mg, 0.888 mmol), 4-[(tert-butoxycarbonyl)amino]- phenylboronic acid (232 mg, 0.977 mmol), potassium carbonate (270 mg, 1.95 mmol), 1,5-bis(diphenylphosphino)pentane (39.1 mg, 0.0888 mmol), allylpalladium(II) chloride dimer (16.2 mg, 0.0444 mmol) and dimethylformamide (1.5 mL). The reaction mixture was heated to 80 °C for 17 hours. In order to consume residual Int-7, additional allylpalladium(II) chloride dimer (32.5 mg, 0.0888 mmol) and 1,5-bis(diphenylphosphino)pentane (78.2 mg, 0.178 mmol) were added and the reaction mixture was allowed to stir at 80 °C for 17 hours. The reaction mixture was filtered through Celite and to the filtrate was added water (40 mL) and a saturated ammonium chloride solution (40 mL). After an extraction with ethyl acetate (2 x 50 mL), the organic portions were combined, washed with brine (50 mL), dried (MgSO₄) and concentrated. The crude material was purified by column chromatography utilizing 30% EtOAc/hexanes as the eluent to produce 365 mg of Int-8 as a pale yellow solid in 91% yield.

### (Int-9) [MCLS1374-135-1]. Synthesis of 1-[3'-(4-Amino-benzyl)-2'-fluoro-6'-methoxy-biphenyl-3-yl]-ethanone; hydrochloride:

In an 8 mL vial equipped with a stir bar was placed Int-8 (265 mg, 0.590 mmol), dichloromethane (2.0 ml) and trifluoroacetic acid (438 µL, 5.90 mmol). The reaction mixture was stirred at room temperature for 4 hours and then quenched to pH 7 with a saturated sodium bicarbonate solution. After the addition of water (30 mL) and extraction with dichloromethane (2 x 30 mL), the organic portions were combined, washed with brine (30 mL), dried (MgSO₄) and concentrated. The crude material was treated with diethyl ether (3 mL) and 2.0 M HCl in diethyl ether (1 mL) and allowed to stir at room temperature for 2 hours. The solid was collected an washed with diethyl ether (3 x 2 mL) to produce 139 mg of Int-9 as a pale orange powder in 61 % yield.

A-016 [MCLS1374-173-2].

### Sulfamoylation of 1-[3'-(4-Amino-benzyl)-2'-fluoro-6'-methoxy-biphenyl-3-yl]-ethanone; hydrochloride:

In an 8 mL vial equipped with a stir bar was placed dichloromethane (400 µL) and chlorosulfonyl isocyanate (13.5 µL, 0.155 mmol). The solution was cooled in an ice water bath for 5 minutes and then tert-butanol (15.9 µL, 0.167 mmol) was added and the reaction mixture was warmed to room temperature for 25 minutes. Then a solution of Int-9 (46 mg, 0.119 mmol), pyridine (106 µL, 0.131 mmol) and dichloromethane (300 µL) was added and the reaction mixture was stirred at room temperature for 17 hours. The reaction was quenched with water (30 mL) and extracted with dichloromethane (2 x 30 mL). The organic portions were combined, washed with 1M HCl (30 mL) and brine (30 mL), dried (MgSO₄) and concentrated. To this Boc protected intermediate was added 1,4-dioxane (600 µL) and 4.0 M HCl in 1,4-dioxane (400 µL). The mixture was stirred at room temperature for 17 hours and then quenched with water (30 mL) and extracted with dichlormethane (2 x 30 mL). The organic portions were combined, washed with a saturated sodium bicarbonate solution (30 mL) and brine (30 mL), dried (MgSO₄) and concentrated to produce 12 mg of A-016 as an orange viscous oil in 24% yield.
¹H NMR (400 MHz, CDCl₃) δ 2.62 (s, 3 H), 3.76 (s, 3 H), 3.95 (s, 2 H), 4.63 (bs, 2 H), 6.34 (bs, I H), 6.73 (d, *J*= 8 Hz, 1 H), 7.10-7.24 (m, 5 H), 7.52 (t, *J*= 8 Hz, 1 H), 7.60 (d, *J*= 6 Hz, 1 H), 7.94 (d, *J*= 8 Hz, 1 H), 7.99 (bs, 1 H).

### Synthesis of A-017

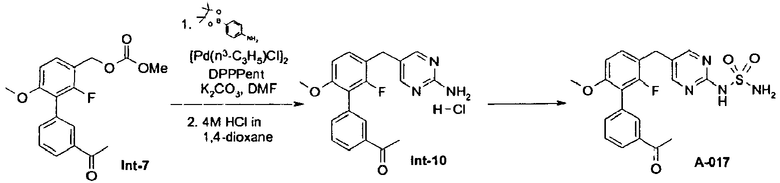

### (Int-10) [MCLS1374-155-1]. Synthesis of 1-[3'-(2-Amino-pyrimidin-5-ylmethyl)-2'-fluoro-6'-methoxy-biphenyl-3-yl]-ethanone hydrochloride:

In an 18 mL vial equipped with a stir bar was placed Int-7 (704 mg, 2.12 mmol), 5-(4,4,5,5-tetramethyl-[1,3,2]dioxaborolan-2-yl)-pyrimidin-2-ylamine (515 mg, 2.33 mmol), potassium carbonate (879 mg, 6.36 mmol), 1,5-bis(diphenylphosphino)pentane (280 mg, 0.636 mmol), allylpalladium(II) chloride dimer (116 mg, 0.318 mmol) and dimethylformamide (4.2 mL). The reaction mixture was heated to 70 °C for 65 hours. The reaction mixture was filtered through Celite and to the filtrate were added water (40 mL) and a saturated ammonium chloride solution (40 mL). After an extraction with ethyl acetate (2 x 50 mL), the organic portions were combined, washed with brine (50 mL), dried (MgSO₄) and concentrated. The crude material was purified by column chromatography utilizing 20% acetone/DCM (gradient elution increased to 30%, then 40% acetone/DCM) as the eluent to produce 341 mg of Int-10 (free base) an off-white solid in 46% yield. Then Int-10 (free base) (20 mg, 0.0569 mmol) was treated with 1,4-dioxane (1 mL) and the mixture was heated to form a solution. To this solution was added 4.0M HCl in 1,4-dioxane (1 mL) and the mixture was stirred at room temperature for 3 hours. The solvent was removed via nitrogen stream and the resulting solid was triturated with diethyl ether (1 mL), collected via suction filtration and washed with diethyl ether (3 x I mL) to produce 12 mg of Int-10 as a pale yellow solid in 55% yield.

### A-017 MCLS1374-175-3]. Sulfamoylation of 1-[3'-(2-Amino-pyrimidin-5-ylmethyl)-2'-fluoro-6'-methoxy-biphenyl-3-yl]-ethanone:

In an 8 mL vial equipped with a stir bar was placed dichloromethane (500 µL) and chlorosulfonyl isocyanate (32.2 µL, 0.371 mmol). The solution was cooled in an ice water bath for 5 minutes and then tert-butanol (38.2 µL, 0.399 mmol) was added and the reaction mixture was warmed to room temperature for 25 minutes. Then a solution of Int-10 (free base) (100 mg, 0.285 mmol), pyridine (254 µL, 3.14 mmol) and dichloromethane (500 µL) was added and the reaction mixture was stirred at room temperature for 3 hours. The reaction was quenched with water (30 mL) and extracted with dichloromethane (2 x 30 mL). The organic portions were combined, washed with 1M HCl (30 mL) and brine (30 mL), dried (MgSO₄) and concentrated. To this Boc protected intermediate was added 1,4-dioxane (1.5 mL) and 4.0 M HCl in 1,4-dioxane (1.0 mL). The mixture was stirred at 50 °C for 2 hours and then quenched with water (30 mL) and extracted with dichloromethane (2 x 30 mL). The organic portions were combined, washed with a saturated sodium bicarbonate solution (30 mL) and brine (30 mL), dried (MgSO₄) and concentrated. The crude material was triturated with 20% acetone/DCM to produce 9.5 mg of A-017 as a white solid in 8% yield.

¹H NMR (400 MHz, DMSO-*d₆*) δ 2.59 (s, 3 H), 3.73 (s, 3 H), 3.91 (bs, 2 H), 6.97 (d, J= 8 Hz, I H), 7.08 (bs, 2 H), 7.36 (t, J= 9 Hz, I H), 7.57-7.58 (m, 2 H), 7.87 (s, 15 H), 7.94-7.96 (m, 15 H), 8.46 (s, 2 H), 10.96 (bs, 1 H). MS(APCI+): 431.1 (M+1), LC-MS: 97%.

### A-018. Synthesis of 4-(3'-Acetyl-2-fluoro-6-methoxy-biphenyl-3-ylmethyl)-benzoic acid methyl ester.

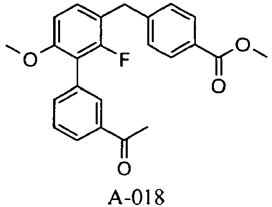

A suspension of **Int-7** (300 mg, 0.928 mmol), 4-methoxycarbonylbenzylboronic acid (184 mg, 1.02 mmol), and potassium carbonate (385 mg, 2.78 mmol) in dimethylformamide (2 mL) was purged with nitrogen and allylpalladiumchloride dimmer (50.9 mg, 0.139 mmol) and bis(diphenylphosphino)pentane (123 mg, 0.278 mmol) were added. The reaction was heated to 65 °C overnight. To this reaction was added ethyl acetate (5 mL) and water (5 mL), the layers were filtered through celite,the celite washed with ethyl acetate (15 mL) and water (15 mL), and the layers separated. The aqueous layer was extracted with ethyl acetate (2 x 50 mL), and the organic extracts combined and washed with brine (100 mL). The organic solution was dried over magnesium sulfate, filtered, and the solvent removed under vacuum to give crude product. The product was purified by flash silica gel column chromatography (25% ethyl acetate in hexanes), then on a preparatory silica gel TLC plate (eluting with 25% ethyl acetate in hexnaes) and triturated in diethyl ether (5 mL) to give **A-018** (72.4 mg, 20% yield) as a white powder.
1H NMR (400 MHz CDCl3) d: 7.991-7.939 (m, 4H), 7.586-7.583 (m, 1H), 7.517 (t, J = 7.80 Hz, 1 H), 7.299 (d, J = 8.80 Hz, 2H), 7.107 (t, J = 8.60 Hz, 1H), 6.742-6.720 (m, 1H), 4.027 (s, 2H), 3.899 (s, 3H), 3.758 (s, 3H), 2.619 (s, 3H). LCMS = 96.6% purity. MS(APCI+)= 394.1(M+2).

### A-020 (PR162)[MCLS1376-184-4]. Synthesis of 5-(3'-Acetyl-2-fluoro-6-methoxy-biphenyl-3-ylmethyl)-pyridine-2-carboxylic acid methyl ester.

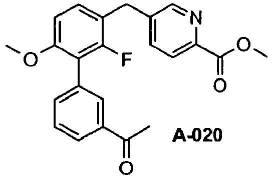

A suspension of **Int-7** (500 mg, 1.50 mmol), 2-methylcarboxypyridine-5-boronic acid pinocol ester (435 mg, 1.65 mmol), and potassium carbonate (624 mg, 4.51 mmol) in dimethylformamide (3.5 mL) was degassed under a nitrogen stream for 15 min. To this solution was added bis(diphenylphosphino)pentate (199 mg, 0.451 mmol) and allylpalladiumchloride dimer (82.7 mg, 0.226 mmol). The reaction was heated to 65 ° C for 50 h. The reaction was diluted with ethyl acetate (50 mL) and filtered through celite. To the filtrate was added water (50 mL), and the layers separated. The aqueous wash was extracted with ethyl acetate (2 x 50 mL), and all three organic extracts combined and washed with brine (100 mL). The organic solution was dried over magnesium sulfate, filtered, and the solvent removed under vacuum. The residue was purified by flash silica gel column chromatography (5% acetone in dichloromethane) and triturated in diethyl ether (15 mL), filtered, and washed with diethyl ether (5 mL) to give **A-020** (190.6 mg, 32% yield) as a white powder. 1H NMR (400 MHz CDC13) d: 8.656 (d, J = 2.00 Hz, I H), 8.062 (d, J = 8.00 Hz, 1 H), 7.973-7.940 (m, 2H), 7.665 (dd, J = 8.20 Hz, 2.20 Hz, 1 H), 7.574 (dd, J = 7.60 Hz, 1.60 Hz, H), 7.518 (t, J = 7.60 Hz, 1 H), 7.125 (t, J = 8.60 Hz, 1 H), 6.747 (d, J = 8.80 Hz, 1 H), 4.053 (s, 2H), 3.996 (s, 3H), 3.765 (s, 3H), 2.618 (s, 3H). LCMS = 100.0% purity. MS(APCI+)= 394.1 (M+1).

### Synthesis of A-019 and A-021

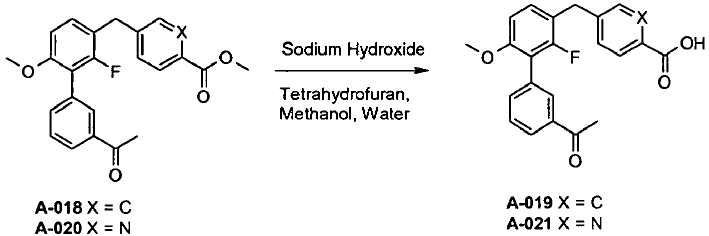

### A-019 (PR164)[MCLS1376-188-1]. Synthesis of 4-(3'-Acetyl-2-fluoro-6-methoxy-biphenyl-3-ylmethyl)-benzoic acid.

A solution of A-018 (65.0 mg, 0.166 mmol) in methanol (750 uL), water (750 uL), and I N aqueous sodium hydroxide (231 uL, 0.231 mmol) was stirred at room temperature overnight. The resultant white suspension was dissolved with tetrahydrofuran (1 mL), and stirred at room temperature for 2 h. Reaction showed completion by silica TLC (Rf = 0.07 in 50% ethyl acetate in hexanes). The tetrahydrofuran and methanol were removed under vacuum, and 1 N aqueous hydrochloric acid (2 mL) was added. The aqueous suspension was extracted with ethyl acetate (2 x 25 mL), the organic extracts combined, and washed with brine (10 mL) that had been acidified with 1 N aqueous hydrochloric acid (3 mL), dried over sodium sulfate, filtered, and the solvent removed under vacuum to give A-019 (42.6 mg, 68% yield) as a white powder. 1 HNMR (400 MHz d6-DMSO) d: 12.81 (s, 1H), 7.962-7.935 (m, 1H), 7.880-7.860 (m, 3H), 7.577 (d, J = 5.20 Hz, 2H), 7.366-7.322 (m, 3H), 6.965 (d, J = 8.80 Hz, 1H), 4.030 (s, 2H), 3.729 (s, 3H), 2.600 (s, 3H). LCMS = 100.0% purity. MS(APCI-)= 377.0 (M-1).

### A-021 (PR165)[MCLS1376-192-2]. Synthesis of 5-(3'-Acetyl-2-fluoro-6-methoxy-biphenyl-3-ylmethyl)-pyridine-2-carboxylic acid.

A solution of A-020 (150 mg, 0.381 mmol) in tetrahydrofuran (1 mL), methanol (1 mL), watrer (1 mL) and 1 N aqueous sodium hydroxide (0.763 mL) was strred at room temperature for 18 h. Approximately one half of the solvent was removed under vacuum. The remaining solution was adjusted to pH 3 by addition of glacial acetic acid. The suspension was extracted with dichloromethane (10 mL), water (5 mL) was added to the wash, and the aqueous wash was extracted with additional dichloromethane (2 x 10 mL). All three organic extracts were combined, dried over magnesium sulfate, filtered, and the solvent removed under vacuum, and the residue was dried under high vacuum for 24 h. The resultant beige syrup was impure with ethyl acetate and acetic acid, so the residue was crystallized in diethyl ether (5 mL), stirred for 30 min, filtered, and washed with hexanes (2 x 2 mL) to give A-021 (90.6 mg, 63% yield) as a white powder.
1H NMR (400 MHz d6-DMSO) d: 8.622 (d, J = 2.00 Hz, 1H), 7.989-7.936 (m, 2H), 7.869 (s, 1H), 7.779 (dd, J = 7.80 Hz, 2.20 Hz, 1H), 7.576 (d, J = 5.20 Hz, 2H), 7.387 (t, J = 8.80 Hz, 1H), 6.982 (d, J = 8.40 Hz, 1H), 4.081 (s, 2H), 3.732 (s, 3H), 2.588 (s, 3H).

LCMS = 100.0 % purity (APCI+). MS(APCI+)= 380.0 (M+1)

### Synthesis of A-022

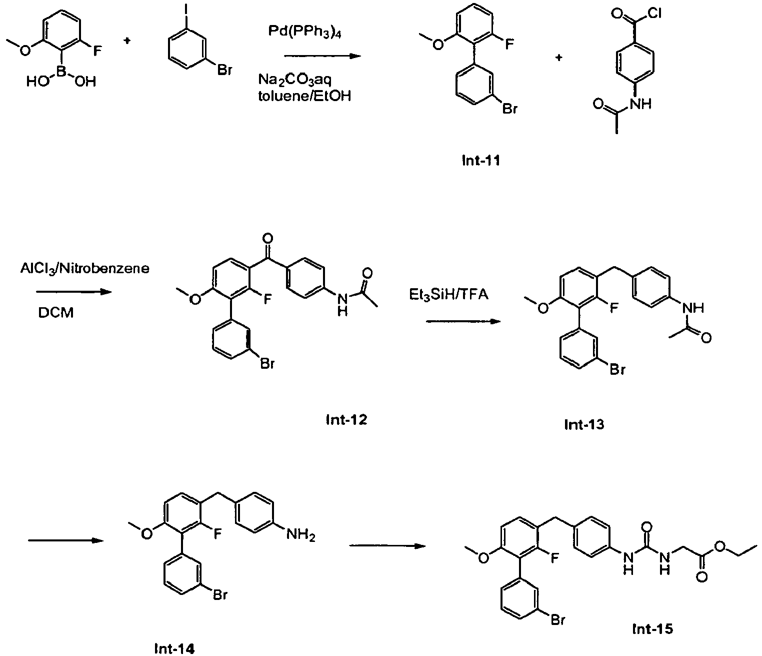

### (Int-11) [MCLS1277-195-01] Synthesis of 3'-Bromo-6-fluoro-2-methoxy-biphenyl.

To a mixture of 2-fluoro-5-methoxyphenyl boronic acid (1g, 5.9 mmol, I eq.), Pd(PPh₃)₄ (340 mg, 0.3 mmol, 0.05 eq.) was added toluene (48 ml) and EtOH (12 ml) followed by the 1-bromo-3-iodo benzene (2g, 7.1 mmol, 1.2 eq) and 2N Na₂CO₃ aq. (6 ml, 2 eq.). The mixture was stirred at 90 C for 3 hr. After cooling to room temperature, the aqueous layer was separated and the organic layer was dried over MgSO₄. Removal of solvent under reduced pressure gave a residue, which was purified by chromatography on silica gel using hexane followed by hexane/EtOAc (20:1) as eluent to give 1.37 g of desired product Int-11 as colorless oil in 83% yield.

### (Int-12) [MCLS1277-197-01] Synthesis of N-[4-(3'-Bromo-2-fluoro-6-methoxy-biphenyl-3-carbonyl)-phenyl]-acetamide.

To AlCl₃ (400 mg, 3.3 mmol, 3 eq.) was added nitrobenzene (2 ml) at rt. The mixture was stirred at rt for I hr. Then 4-Acetylamino-benzoyl chloride (200 mg, 1 mmol, 1 eq.) was added in one portion followed by a solution of Int-11 in DCM (2 ml). The resulting mixture was stirred at rt until the acid chloride was consumed in 3 hr. The mixture was added water and then extracted with ethyl acetate. The organic layer was separated and dried. Evaporation of solvent gave a residue, which was purified by chromatography on silica gel using EtOAc/hexane (1:1 then 2:1) as eluent to give 221 mg off desired product Int-12 in 50 % yield.

### (Int-13) [MCLS1277-200-01] Synthesis of N-[4-(3'-Bromo-2-fluoro-6-methoxy-biphenyl-3-ylmethyl)-phenyl]-acetamide.

To a solution of Int-12 (170 mg, 0.38 mmol, 1 eq.) in trifluoroacetic acid (1 ml) was added triethylsilane (0.5 ml). The mixture was stirred at rt for 4 hr. and then volatile material was removed under reduced pressure to give a residue, which was triturated with DCM/hexane (1:2). The solid was filtered and washed with hexane. After drying, 190 mg of Int-13 was obtained in quantitative yield.

### (Int-14) [MCLS1277-201-01] Synthesis of 4-(3'-Bromo-2-fluoro-6-methoxy-biphenyl-3-ylmethyl)-phenylamine.

In a 40 ml vial was charged with Int-13 (230 mg, 0.54 mmol, 1 eq.), conc. HCl (8 ml) and EtOH (8 ml). The mixture was stirred at 85 C for 5hr. After cooling to room temperature, water was added followed by addition of NaHCO₃ aq. The mixture was extracted with EtOAc. The organic layer was separated and dried. Removal of solvent gave 176 mg of desired product Int-14 without further purification (85% yield).

### (Int-15) [MCLS1435-055-1] Synthesis of {3-[4-(3'-Bromo-2-fluoro-6-methoxy-biphenyl-3-ylmethyl)-phenyl]-ureido}-acetic acid ethyl ester.

To an 8 mL vial containing Int-14 (108 mg, 0.280 mmol) was added a magnetic stir bar and pyridine (0.5 mL). To this stirring solution was added ethyl isocyanatoacetate (62 uL, 72 mg, 0.56 mmol) dropwise over 15 s. The solution was allowed to stir at room temperature overnight. The reaction mixture was diluted with water (5 mL) and rapidly stirred at room temperature for 2 h. The resulting solid was filtered, rinsed with water (2 x 3 mL) and dried to yield 120 mg (83%) of Int-15 as a solid. 1H NMR (400 MHz, DMSO-d6) 1.19 (t, J = 7.2 Hz, 3H), 3.71 (s, 3H), 3.84 (m, 4H), 4.10 (q, J = 7.2 Hz, 2H), 6.38 (t, J = 5.6 Hz, 1H), 6.91 (d, J = 8.0 Hz, 1H) 7.08 (m, 2H), 7.26, t, J = 8.8 Hz, 1H), 7.30 (m, 3H), 7.38 (dd, apparent triplet, J = 8.0, 7.6 Hz, 1H), 7.49 (s, 1H), 7.55 (m, 1H).

### A-022 [MCLS1435-059-1] Synthesis of {3-[4-(3'-Bromo-2-fluoro-6-methoxy-biphenyl-3-ylmethyl)-phenyl]-ureido}-acetic acid.

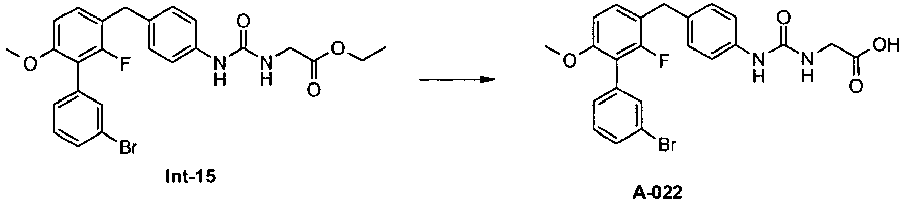

To an 8 mL vial containing **Int-15** (70 mg, 0.136 mmol) and a magnetic stir bar was added anhydrous tetrahydrofuran (2 mL) followed by 1M aqueous lithium hydroxide (408 uL, 0.408 mmol). After 1 h, the reaction mixture was concentrated under a stream of nitrogen gas to a final volume of ∼0.4 mL and then diluted with water to a total volume of 3 mL. With stirring, 2 N aqueous HCl (250 uL, 0.5 mmol) was added dropwise rover ∼1 min affording a clumpy solid. The mixture was stirred for 30 min at room temperature affording a free-flowing solid which was filtered and dried to give 50 mg (76%) yield of **A-022.**
1H NMR (400 MHz, DMSO-d6) 3.71 (s, 3H), 3.77 (d, J = 5.6 Hz, 2H), 3.84 (s, 2H) 6.30 (t, H = 5.6 Hz, 1H), 6.91 (d, J = 8.4 Hz, 1H), 7.25 (dd, apparent triplet, J = 9.2, 8.4 Hz, 1 H), 7.30 (m, 3H), 7.38 (dd, apparent triplet, J = 8.0, 7.6 Hz, 1H), 7.49 (m, 1H), 7.55 (m, 1H), 8.68 (s, 1H), 12.53 (br s, 1H). MS(APCI+): 487.0 (M⁺), 489 (M⁺+2) ; LC-MS: 98.0%.

### A-023 [MCLS1435-063-1] Synthesis of 3-{3-[4-(3'-Bromo-2-fluoro-6-methoxy-biphenyl-3-ylmethyl)-phenyl]-ureido}-propionic acid.

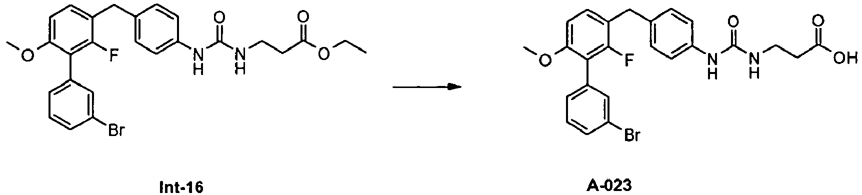

### [MCLS 1435-056-1] Synthesis of 3-{3-[4-(3'-Bromo-2-fluoro-6-methoxy-biphenyl-3-ylmethyl)-phenyl]-ureido}-propionic acid ethyl ester.

Compound Int-16 was synthesized in a manner analogous to those described for the synthesis of compound Int-15, using Int-14 (101 mg, 0.26 mmol) and ethyl isocyanatopropionate in place of ethyl isocyanatoacetate to give 117 mg (85%) of Int-16 as an off-white solid.

1H NMR (400 MHz, DMSO-d6) 1.19 (t, J = 7.6 Hz, 3H), 2.47 (t, J = 6.4 Hz, 2H), 3.29 (dd, J = 6.4, 5.6 Hz, 2H), 3.72 (s, 3H), 3.84 (s, 2H), 4.07 (q, J = 7.6 Hz, 2H), 6.17 (t, H = 5.6 Hz, 1H), 6.91 (d, J = 8.8 Hz, 1H), 7.06 (m, 2H), 7.25 (dd, apparent triplet, J = 8.4 Hz, 1H), 7.30 (m, 3H), 7.38 (dd, apparent triplet, J = 8.0, 7.6 Hz, 1H), 7.49 (m, 1H), 7.55 (m, 1H).

Compound A-023 was synthesized in a manner analogous to those described for the synthesis of compound A-022 using compound Int-16 (73 mg, 0.26 mmol) to give 46 mg (67%) of A-023 as a solid.

1 H NMR (400 MHz, DMSO-d6) δ 2.40 (t, J = 6.4 Hz, 2H), 3.26 (dd, J = 6.4, 5.6 Hz, 2H), 3.71 (s, 3H), 3.83 (s, 2H), 6.16 (t, H = 5.6 Hz, I H), 6.91 (d, J = 8.4 Hz, 1H), 7.06 (m, 2H), 7.27 (m, 3H), 7.32 (m, 1H), 7.38 (dd, apparent triplet, J = 8.0, 7.6 Hz, 1H), 7.49 (m, 1H), 7.55 (m, 1H), 8.47 (s, 1H), 12.25 (br s, 1H). MS(APCI+): 50 1.0 (M⁺), 503 (M⁺+2); LC-MS: 97.8 %.

### Synthesis of A-024, A-025, A-026, A-027:

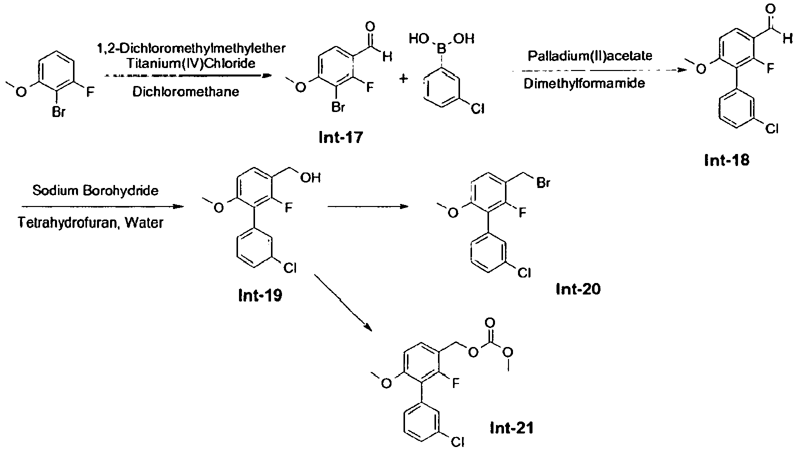

### (Int-17) [MCLS 1379-166-1] Synthesis of 3-Bromo-2-fluoro-4-methoxy-benzaldehyde using titanium(IV)chloride.

A solution of 2-bromo-3-fluoroanisol (5.00 g, 24.3 mmol) in dichloromethane (120 mL) was cooled to 0 °C in a salt-ice bath and purged with nitrogen. The reaction was allowed to stir 15 min under nitrogen. To the reaction was added titanium(IV)chloride (23.1 g, 122 mmol), followed by a,a-dichloromethyl-methylether (4.21 g, 36.6 mmol) at 0 °C under nitrogen. The reaction was allowed to warm to room temperature and stirred for 22 h. The red solution was poured into ice water (600 mL), and extracted into dichloromethane (3 x 200 mL). The organic extracts were combined, washed with saturated aqueous sodium bicarbonate (2 x 400 mL), water (2 x 400 mL), and brine (400 mL), dried over sodium sulfate, filtered, and the solvent removed under vacuum to give wet product. The product was dried in a vacuum oven at 80°C overnight to give Int-17 (5.75 g, quantitative yield).
1H NMR (400 MHz CDCl3) δ 10.22 (s, 1 H), 7.86 (dd, J = 8.8 Hz, 7.6 Hz, 1H), 6.82 (d, J = 8.4 Hz, 1H), 4.01 (s, 3H).

### (Int-18) [MCLS1489-130-1] Synthesis of 3'-Chloro-2-fluoro-6-methoxy-biphenyl-3-carbaldehyde.

A mixture of **Int-17** (2.0 g, 7.98 mmol), 3-chlorophenyl boronic acid (1.4 g, 8.76 mmol) and cesium carbonate (5.2 g, 15.9 mmol) in dimethylformamide (20 mL) was degassed with a nitrogen stream for 4 min. To the reaction was added palladium(II) acetate (89 mg, 0.398 mmol) and the reaction was stirred at 50 °C for 2 h and at room temperature for 16 h. Additional palladium(III) acetate (89 mg, 0.398 mmol) was added and the reaction stirred at 50°C for 2 h. The reaction was cooled, filtered through celite, and washed with ethyl acetate. The reaction was partitioned with ethyl acetate (100 mL) and water (100 mL), and brine was added (20 mL). The aqueous layer was extracted with ethyl acetate (2 x 75 mL) and the combined extracts washed with aqueous 5% lithium chloride (50 mL), aqueous 0.5 N hydrochloric acid (50 mL), water (2 x 50 mL), and brine (50 mL). The solution was dried over magnesium sulfate, filtered, and the solvent removed under vacuum to give crude **Int-18** (2.2 g, 98% yield) which was used in the next step without further purification.

### (Int-19) [MCLS1489-132] Synthesis of (3'-Chloro-2-fluoro-6-methoxy-biphenyl-3-yl)-methanol.

A solution of Int-18 (2.00 g, 6.8 mmol) in tetrahydrofuran (18 mL) and water (6 mL) was cooled to 0°C in an ice water bath. To the solution was added sodium borohydride (388 mg, 10 mmol). The reaction was stirred for 10 minutes, and upon completion was diluted with water (10 mL) and aqueous saturated ammonium chloride (40 mL). The reaction was extracted with ethyl acetate (50 mL, 100 mL), and the combined extracts washed with water (2 x 50 mL) and brine (50 mL). The organic extract was dried over magnesium sulfate, filtered, and the solvent removed under vacuum to give **Int-19** (2.1 g) which was used in the next step without further purification.

### (Int-20) [MCLS1426-103-1] Synthesis of 3-Bromomethyl-3'-chloro-2-fluoro-6-methoxy-biphenyl:

A solution of **Int-19** (5.44 mmol; 1.0 eq.) and triphenylphosphine (5.98 mmol; 1.1 eq.) was cooled to 0-5°C, and N-bromosuccinimide (5.98 mmol; 1.1 eq.) was added. The mixture was allowed to stir, and warm to room temperature for h hours. Additional N-bromosuccinmide (0.54 mmol; 0.1 eq.) and triphenylphosphine (0.54 mmol; 0.1 eq.) were added, and the reaction was stirred for an additional 1hour. The reaction was then diluted with water, and the layers separated. The organic portion was washed with successive portions of water and brine, dried over magnesium sulfate, and filtered through a short pad of silica gel. The filtrate was concentrated, and the residue purified via silica gel plug filtration, using 1% acetone/hexanes as eluent to afford the title compound as white needles in 78% yield. ¹HNMR, CDCl₃; 400 MHz): δ 3.79 (s, 3H), 4.55 (s, 2H), 6.76 (dd, J = 8.8, 1.2 Hz, 1H), 7.28 (m, 1H), 7.34-7.38 (M, 3H), 7.39 (m, 1H).

### (Int-21) Synthesis of Carbonic acid 3'-chloro-2-fluoro-6-methoxy-biphenyl-3-ylmethyl ester methyl ester.

A solution of (3'-Chloro-2-fluoro-6-methoxy-biphenyl-3-yl)-methanol (Int-19) (3.00g, 11.3 mmol) in pyridine (2.31 g, 29.3 mmol) and tetrahydrofuran (40 mL) was cooled to 0°C in an ice water bath. The reaction vessel was purged with nitrogen and methyl chloroformate (2.34 g, 24.8 mmol) was added. The ice bath was removed and the reaction was stirred at room temperature overnight. The white suspension was adjusted to pH 2 by addition of 1 N aqueous hydrochloric acid (∼25 mL) and the yellow biphasic solution was diluted with dichloromethane (200 mL) and water (150 mL). The layers were separated, and the aqueous layer was extracted with dichloromethane (2 x 100 mL). The dichloromethane extracts were combined, washed with water (2 x 200 mL) and brine (200 mL), dried over sodium sulfate, filtered, and the solvent removed under vacuum to give **Int-21** (3.84 g, quantitative yield). 1H NMR (400 MHz CDC13) d: 7.41-7.32 (m, 4H), 7.29-7.27 (m, 1H), 6.77 (dd, J = 8.4 Hz, 0.8 Hz, 1H), 5.20 (s, 2H), 3.80 (s, 3H), 3.80 (s, 3H). LCMS = 98.2% purity. MS(APCI+) = 249.0 (M-78), MS(APCI-) = 249.0 (M-78).

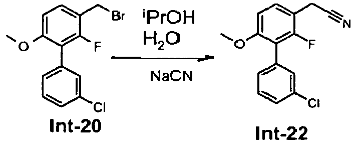

### (Int-22) [MCLS1426-077-1] Synthesis of (3'-Chloro-2-fluoro-6-methoxy-biphenyl-3-yl)-acetonitrile:

A suspension of **Int-20** (15.2 mmol; 1.0 eq.) and sodium cyanide (22.8 mmol; 1.5 eq.) in aqueous isopropyl alcohol was refluxed for 1 hour. The reaction was cooled to room temperature, and concentrated to remove the isopropyl alcohol. The resultant aqueous layer was extracted with 2 portions of ethyl acetate, and the organics washed successively with water and brine, and dried over magnesium sulfate. The residue was purified via flash chromatography on silica gel, using 10% acetone/hexanes as eluent to afford the title compound (**Int-22**) as a white solid in 57% yield. (¹HNMR, CDCl₃; 400 MHz): δ 3.80 (s, 3H), 3.93 (s, 2H), 6.80 (dd, J = 8.8, 1.6 Hz, 1 H), 7.26 (m, 1H), 7.35-7.41 (M, 4H).

### Synthesis of Int-23 :

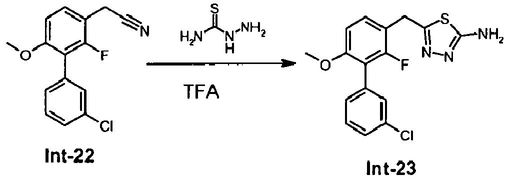

### Int-23 [MCLS1426-108-1]. Synthesis of 5-(3'-Chloro-2-fluoro-6-methoxy-biphenyl-3-ylmethyl)-[1,3,4]thiadiazol-2-ylamine:

A mixture **of Int-22** (0.91 mmol; 1.0 eq.) and thiosemicarbazide (1.03 mmol; 1.1 eq.) in trifluoroacetic acid was heated to 60°C for 6 hours, and allowed to cool to room temperature. The mixture was added to ice water, stirred for 1 hour, and filtered. The filter cake was washed with water, and the solid dried at 45-50°C under vacuum for 5 hours to afford the title compound in 99% yield.

### A-024 [MCLS1426-127-1]. Synthesis of {3-[5-(3'-Chloro-2-fluoro-6-methoxy-biphenyl-3-ylmethyl)-[1,3,4]thiadiazol-2-yl]-ureido}-acetic acid ethyl ester:

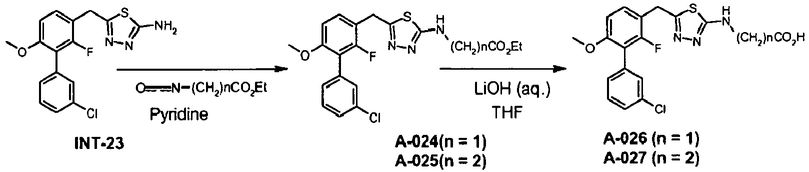

To a solution **of Int-23** in pyridine at room temperature was added the isocyanate. The resultant mixture was stirred at room temperature for 3 days, and added to water. The resultant suspension was stirred for 1 hour, filtered, and washed with water. The solid was dried *in vacuo* over ethyl acetate vapors to afford the desired product as a white solid in 67% yield.
(¹HNMR, DMSO-d₆; 400 MHz): δ 1.17 (t, J = 7.2 Hz, 3H), 3.34 (d, J = 6.4 Hz, 2H), 3,75 (s, 3H), 4.06 (q, J = 7.2 Hz, 2H), 4.27 (s, 2H), 6.69 (bs 1H), 6.99 (d, J = 8.8 Hz, 1H), 7.29 (d, J = 6.8 Hz, 1H), 7.3 7 (s, 1H), 7.40-7.48 (M, 4H), 10.79 (s, 1H). LC/MS(90.7%) APCI⁺ found: 479.0; calc'd: 478.9 m/z

**A-025** [MCLS1426-128-1]. S**ynthesis of 3-{3-[5-(3'-Chloro-2-fluoro-6-methoxy-biphenyl-3-ylmethyl)-[1,3,4]thiadiazol-2-yl]-ureido}-propionic acid ethyl ester:** This compopund was prepared analogous to A-024 in 51% yield.
(¹HNMR, DMSO-d₆; 400 MHz): 1.19 (t, J = 7.2 Hz, 3H), 3.75 (s, 3H), 3.90 (d, J = 6.0 Hz, 2H), 4.05-4.13 (M, 2H), 4.27 (s, 2H),6.51 (dd, J = 6.0 Hz, 1H), 6.99 (d, J = 8.4 Hz, 1H), 7.29 (d, J = 6.8 Hz, 1H), 7.37 (s, 1H), 7.40-7.48 (M, 4H), 11.11 (s, 1H).

### A-026 [MCLS1426-130-1]. Synthesis of {3-[5-(3'-Chloro-2-fluoro-6-methoxy-biphenyl-3-ylmethyl)-[1,3,4]thiadiazol-2-yl]-ureido}-acetic acid:

To a solution of starting ester in THF was added excess 1M aqueous lithium hydroxide. The biphasic mixture was stirred at room temperature until TLC indicated complete consumption of starting material. The THF was removed with a stream of nitrogen, and the resultant aq. solution was acidified with 3 N hydrochloric acid to pH 1-2. The resultant solids were filtered, washed with water, and dried *in vacuo* over ethyl acetate vapors to afford the desired product as a white solid in 70% yield.
(¹HNMR, DMSO-d₆; 400 MHz): δ 3.75 (s, 3H), 3.83 (d, J = 5.6 Hz, 2H), 4.27 (s, 2H), 6.83 (m, 1H), 6.99 (d, J = 8.4 Hz, 1H), 7.29 (d, J = 7.2 Hz, 1H), 7.38 (s, 1H), 7.40-7.48 (M, 4H), 11.06 s, 1H), 12.70 (s, 1H). LC/MS(91.7%) APCI⁻ found: 449.0 (M - 1); calc'd: 450.9 m/z.

**A-027** [MCLS1426-131-1]. **Synthesis of 3-{3-[5-(3'-Chloro-2-fluoro-6-methoxy-biphenyl-3-ylmethyl)-[1,3,4]thiadiazol-2-yl)-ureido}-propionic acid:** This compopund was prepared analogous to A-026 in 68% yield.
(¹HNMR, DMSO-d₆; 400 MHz): δ 2.42 (t, J = 6.4 Hz, 2H), 3.32 (t, J = 6.4 Hz, 2H), 3.75 (s, 3H), 4.26 (s, 2H), 6.71 (m, 1H), 6.99 (d, J = 8.4 Hz, 1H), 7.29 (d, J = 6.8 Hz, 1H), 7.38 (s, 1H), 7.40-7.46 (M, 4H), 10.80 (s, 1H), 12.32 (s, 1H).
LC/MS(91.7%) APCI⁻ found: 463.0 (M - 1); calc'd: 464.9 m/z

### Synthesis of A-033 and A-034

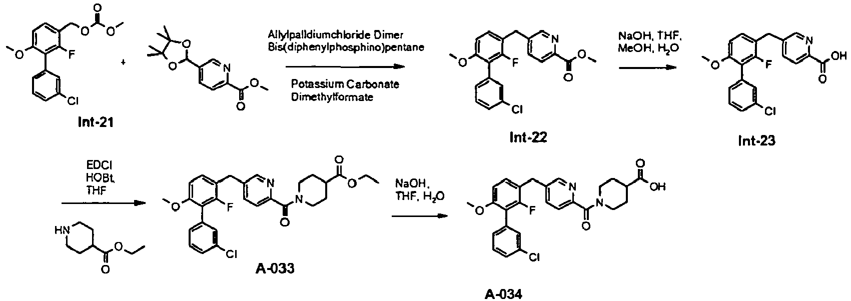

(**Int-22**) [MCLS1458-041-3] **Synthesis of 5-(3'-Chloro-2-fluoro-6-methoxy-biphenyl-3-ylmethyl)-pyridine-2-carboxylic acid methyl ester. Int-22 was synthesized from Int-21 (516 mg, 1.59 mmol) and** 2-methylcarboxypyridine-5-boronic acid pinocol ester (460 mg, 1.75 mmol) using the same procedure as **A-020.** The crude material was purified by flash silica gel column chromatography (0-5% acetone in dichloromethane) to give **Int-22** (381.4 mg, 62% yield) as an orange syrup.
**1H NMR (400 MHz CDCl3**) δ 8.65 (d, J = 2.4 Hz, I H), 8.06 (d, J = 8.0 Hz, 1H), 7.66 (dd, J = 8.0 Hz, 2.0 Hz, 1H), 7.3 7-7.39 (m, 3H), 7.26-7.24 (m, 1H), 7.11 (t, J = 8.6 Hz, 1H), 6.78 (dd, J = 8.6 Hz, 1.0 Hz, 1H), 4.04 (s, 2H), 4.00 (s, 3H), 3.77 (s, 3H). **LCMS** = **96.8% purity. MS(APCI+)= 386.0 (M+1).**

### (Int-23) [MCLS1458-043-2] Synthesis of 5-(3'-Chloro-2-fluoro-6-methoxy-biphenyl-3-ylmethyl)-pyridine-2-carboxylic acid.

To a solution of Int-22 (317 mg, 0.822 mmol) in tetrahydrofuran (2.2 mL), methanol (2.2 mL), and water (2.2 mL) was added 1 N aqueous sodium hydroxide (1.64 mL, 1.64 mmol). The resultant solution was stirred at room temperature for 23 h. The pH was adjusted with glacial acetic acid to pH 4. Approximately ¾ of the solvent was removed under vacuum and the remaining suspension was diluted with water (10 mL). The suspension was extracted with dichloromethane (3 x 10 mL), and the combined extracts were dried over magnesium sulfate, filtered, and the solvent removed under vacuum to give crude Int-23 (250.8 mg, 82% yield) as a beige solid.
**1H NMR (400 NMR CDCl3) δ 8.50 (m, 1H),** 8.13 (d, J = 7.6 Hz, 1H), 7.76-7.71 (m, 1H), 7.37-7.32 (m, 3H), 7.26-7.24 (m, 1H), 7.13 (t, J = 8.6 Hz, I H), 6.74 (d, J = 8.8 Hz, I H), 4.05 (s, 2H), 3.77 (s, 3H).

### A-033 [MCLS1458-057-2]. Synthesis of 1-[5-(3'-Chloro-2-fluoro-6-methoxy-biphenyl-3-ylmethyl)-pyridine-2-carbonyl]-piperidine-4-carboxylic acid ethyl ester.

A solution of **Int-23** (75.0 mg, 0.202 mmol), piperidine-4-carboxylic acid ethyl ester (34.9 mg, 0.222 mmol), EDCI (42.6 mg, 0.222 mol), and HOBt (30.0 mg, 0.222 mmol) in tetrahydrofuran (2 mL) was stirred at room temperature for 16 h. The solvent was removed under vacuum and the residue was suspended in ethyl acetate (20 mL). The organic suspension was washed with 1:1 saturated aqueous sodium bicarbonate and water (20 mL), and the wash was extracted with ethyl acetate (20 mL). The organic extracts were combined, washed with water (20 mL), brine (20 mL), dried over sodium sulfate, filtered and the solvent removed under vacuum to give crude product as a yellow grease. The product was purified by silica gel preparatory thin layer chromatography (10% mcthanol in dichloromethane) to give **A-033** (88.3 mg, 86% yield).
1H NMR (400 mHz CDCl3) δ: 8.47 (s, 1H), 7.63-7.54 (m, 3H), 7.39-7.28 (m, 3H), 7.12 (t, J = 8.6 Hz, 1H), 6.73 (d, J = 8.4 Hz, 1H), 4.55-4.52 (m, 1H), 4.15 (q, J = 14.0 Hz, 2H), 3.95 (m, 1H), 3.77 (s, 3H), 3.21-3.01 (m, 2H), 2.61-2.55 (m, 1H), 2.51-2.02 (m, 1H), 1.86-1.80 (m, 3H), 1.26 (t, J = 7.0 Hz, 3H). LCMS = 97.4%. MS(APCI+)= 511.0 (M+1).

### A-034 [MCLS1458-073-1]. Synthesis of 1-[5-(3'-Chloro-2-fluoro-6-methoxy-biphenyl-3-ylmethyl)-pyridine-2-carbonyl]-piperidine-4-carboxylic acid.

A solution of A-033 (60.0 mg, 0.117 mmol) in tetrahydrofuran (2 mL), water (2 mL), and 1 N aqueous sodium hydroxide (0.176 mL, 0.176 mmol) was stirred at room temperature for 3 h. The tetrahydrofuran was removed under vacuum, and glacial acetic acid was added (0.5 mL). The aqueous suspension was extracted with dichloromethane (5 mL), then diluted with water (10 mL), and extracted with dichloromethane (10 mL). The organic extracts were combined, washed with brine (15 mL), dried over magnesium sulfate, filtered, and the solvent removed under vacuum to give product as a gummy material. The gummy solid was dried under high vacuum at room temperature for 1 h, to give **A-034** (43.7 mg, 77% yield).
1H NMR (400 MHz CDCl3) δ: 8.55 (d, J = 2.0 Hz, I H), 7.65 (dd, J = 8.0 Hz, 2.0 Hz, 1H), 7.56-7.46 (m, 2H), 7.39-7.27 (m, 3H), 7.13 (t, J = 8.6 Hz, 1H), 6.73 (d, J = 7.6 Hz, 1H), 4.56-4.53 (m, 1H), 4.01 (s, 1H), 3.88-3.85 (m, 1H), 3.77 (s, 3H), 3.16-3.02 (m, 2H), 2.65-2.60 (m, 1H), 2.06 (brs 1H), 1.89 (brs, 3H). LCMS = 98.1% purity. MS(APCI-) = 481.1 (M-1).

### Synthesis of A-038

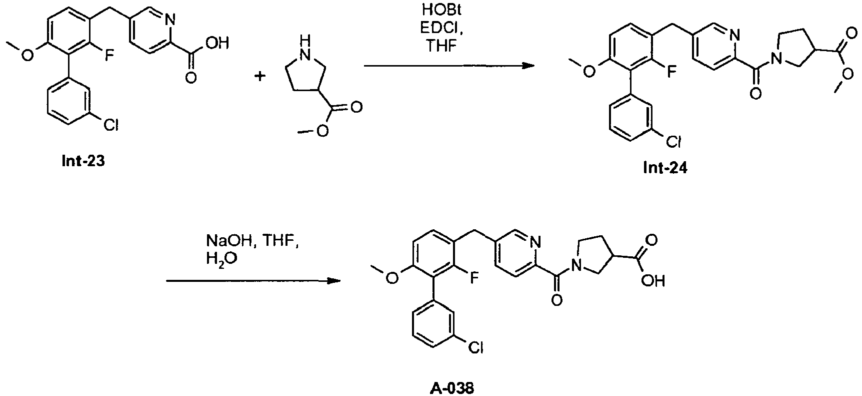

### (Int-24) [MCLS1458-081-2] Synthesis of 1-[5-(3'-Chloro-2-fluoro-6-methoxy-biphenyl-3-ylmethyl)-pyridine-2-carbonyl]-pyrrolidine-3-carboxylic acid methyl ester.

To a solution of **Int-23** (100 mg, 0.269 mmol) and pyrrolidine-3-carboxylic acid methyl ester (49.0 mg, 0.296 mmol) in tetrahydrofuran (3 mL), was added HOBt (40.0 mg, 0.296 mmol) and EDCI (56.7 mg, 0.296 mmol). Dimethylformamide (1 mL) was added and the reaction stirred at room temperature overnight. Additional pyrrolidine-3-carboxylic acid methyl ester (25.0 mg) was added, and the reaction stirred at room temperature overnight. The solvent was removed under vacuum and dichloromethane (10 mL) and water (10 mL) were added. The layers were separated, and the water extracted with dichloromethane (10 mL). The organic extracts were combined, washed with water (20 mL), saturated aqueous sodium bicarbonate (20 mL), and brine (20 mL), dried over magnesium sulfate, filtered, and the solvent removed under vacuum to give crude product. The crude material was purified by silica gel preparatory thin layer chromatography (eluting with 10% acetone in dichloromethane) to give Int-24 (28.4 mg, 22% yield) as a clear oil.

### A-038 [MCLS1458-091-1]. Synthesis of 1-[5-(3'-Chloro-2-fluoro-6-methoxy-biphenyl-3-ylmethyl)-pyridine-2-carbonyl]-pyrrolidine-3-carboxylic acid.

A solution of **Int-24** (25.0 mg, 0.0518 mmol) and 1 N aqueous sodium hydroxide (77.7 uL, 0.0777 mmol) in tetrahydrofuran (1 mL) and water (1 mL) was stirred at room temperature for 3 h. The solvent was removed under vacuum and the residue was neutralized with 10 drops of glacial acetic acid. The crude material was dissolved in water (5 mL) and dichloromethane (5 mL) and the two layers separated. The aqueous solution was extracted with dichloromethane (5 mL) and the combined dichloromethane layers were washed with water (5 mL) and brine (5 mL), dried over magnesium sulfate, filtered, and the solvent removed under a nitrogen stream to give crude product as a beige powder. The product was purified by silica gel preparatory thin layer chromatography (eluted with 25% acetone in dichloromethane with 3 developments) to give **A-038** (11.6 mg, 48% yield).
1H NMR (400 MHz d6-DMSO) δ: 8.51 (d, J = 7.6 Hz, 1H), 7.74-7.66 (m, 2H), 7.45-7.28 (m, 5H), 6.96 (d, J = 8.4 Hz, I H), 4.03 (s, 2H), 3.76-3.64 (m, 7H), 2.95 (s, 1H), 2.02 (m, 2H). LCMS = 98.1 % purity. MS(APCI-) = 467.1 (M-1).

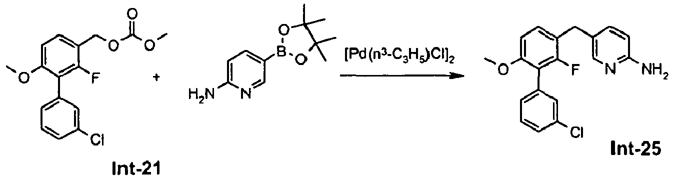

### Int-25 [MCLS1471-064-1] 5-(3'-Chloro-2-fluoro-6-methoxy-biphenyl-3-ylmethyl)-pyridin-2-ylamine

Into a 250 mL round bottom flask was added Int-21 (8.04 g, 24.76 mmol), 5-(4,4,5,5-Tetramethyl-[1,3,2]dioxaborolan-2-yl)-pyridin-2-ylamine (4.09 g, 18.57 mmol), K₂CO₃ (10.27 g, 74.27 mmol), and DMF (100 mL). After degassing for 20 minutes with N₂, Allylpalladium(II) chloride dimer (1.36 g, 3.71 mmol) and 1,5-bis(diphenylphosphano)pentane (3.27 g, 7.43 mmol) were added and the reaction stirred for 18 hours at 80 °C and room temperature for 2 days. Water was added and the product was extracted with EtOAc. The organics were concentrated and then the residue purified by flash column chromatography (0-5% MeOH/DCM) to provide **Int-25** (4.1 g, 48%) as a red oil. The compound was used as is in the next reaction.

### A-039 MCLS1471-072-2

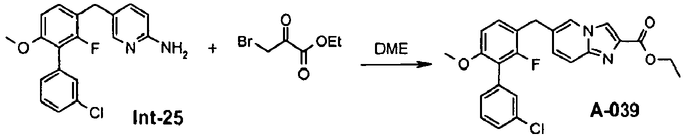

### 6-(3'-Chloro-2-fluoro-6-methoxy-biphenyl-3-ylmethyl)-imidazo[1,2-a]pyridine-2-carboxylic acid ethyl ester

Into an 18 mL vial were added **Int-25** (565 mg, 1.65 mmol), 3-Bromo-2-oxo-propionic acid ethyl ester (0.52 mL, 4.12 mmol), and dimethoxyethane (DME, 5 mL). The reaction was stirred at room temperature for 18 hours. Saturated NaHCO₃ (aq) was added and the product was extracted with EtOAc. The organics were concentrated and purified by flash column chromatography (5% Acetone/DCM) to give a tan solid. The solid was triturated with ether to obtain **A-039** (198 mg, 26%) as an off-white solid. ¹H-NMR (400 MHz, CDCl₃): 8.51 (s, 1 H), 8.39 (s, 1 H), 7.56 (d, *J*= 9.4 Hz, 1H), 7.49 - 7.35 (m, 4 H), 7.30 (d, *J*= 6.7 Hz, 1 H), 7.25 (dd, *J* = 1.3, 9.4 Hz, 1 H), 6.97 (d, *J*= 8.6 Hz, 1 H), 4.29 (q, *J* = 7.1 Hz, 2 H), 3.96 (s, 2 H), 3.74 (s, 3 H), 1.31 (t, J = 7.0 Hz, 3 H).

### A-043 MCLS1471-078-1

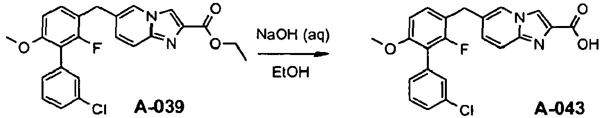

### 6-(3'-Chloro-2-fluoro-6-methoxy-biphenyl-3-ylmethyl)-imidazo[1,2-a]pyridine-2-carboxylic acid.

Into an 8 mL vial was added **A-039** (39 mg, 0.089 mmol), 1N NaOH (1 mL), and I mL EtOH. The reaction was stirred at room temperature for 18 hours and then 60 °C for 1 hour. The pH was adjusted to about 5 with 1N HCl and a light-colored precipitate formed. The solid was filtered, washed with water, and dried under high vacuum to obtain **A-043** (22.2 mg, 61%) as an off-white solid. ¹H-NMR (400 MHz, DMSO-d₆): 8.36 (s, 1H), 8.14 (s, 1H), 7.39 (m, 6H), 7.14 (dd, 1H, J = 9.2, 2.0 Hz), 6.96 (d, 1H, J = 8.4 Hz), 3.93 (s, 2H), 3.73 (s, 3H). LC/MS = 94.6%, 411.0 (APCI+).

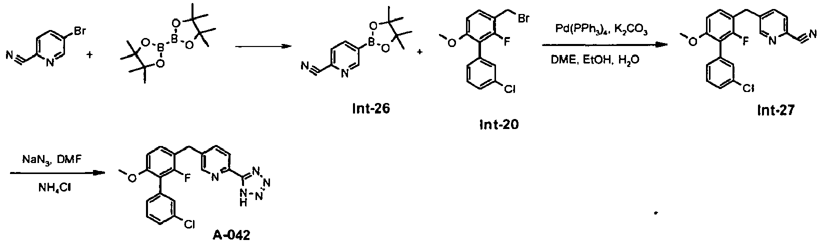

### Int-26 MCLS1430-173-1 5-(4,4,5,5-Tetramethyl-[1,3,2]dioxaborolan-2-yl)-pyridine-2-carbonitrile

Into a 250 mL RBF was added 3.0 g 5-Bromo-pyridine-2-carbonitrile (3.0 g, 16.39 mmol), Bis(pinacolato)diboron (4.58 g, 18.03 mmol), KOAc (5.47 g, 55.74 mmol), and DMSO (100 mL). After degassing for 20 minutes, PdCl₂dppf-CH₂Cl₂ (1.39 g, 1.64 mmol) was added and the solution was stirred for 24 hours at 80 °C, and then room temperature for 3 days. 50 mL water was added and the product was extracted with ethyl acetate. The combined organics were washed with brine, dried over Na₂SO₄ and concentrated. The dark-colored residue was purified by FCC eluting with 20% acetone/hexanes to give a red solid. The solid was triturated with hexane to give 1.72 g (46%) **Int-26** as a light-pink solid.

### Int-27 MCLS1430-126-1

### [5-(3'-Chloro-2-fluoro-6-methoxy-biphenyl-3-ylmethyl)-pyridin-2-ylmethyl]-carbamic acid tert-butyl ester

Into a 20 mL vial was added **Int-26** (401 mg, 1.22 mmol), **Int-20** (336 mg, 1.46 mmol), K₂CO₃ (504 mg, 3.65 mmol), DME (5 mL), water (0.5 mL), ethanol (0.5 mL), and the suspension was degasses for 15 minutes. Pd(PPh₃)₄ (141 mg, 0.12 mmol) was added and the reaction stirred at 80 °C for 16 hours. The reaction was diluted with water and extracted with ethyl acetate. The organics were concentrated and purified by FCC eluting with 15 - 20% ethyl acetate/hexanes to afford **Int-27** (64 mg, 15%) as a light-yellow oil. ¹H NMR (400MHz ,CHLOROFORM-d) δ = 8.62 (br. s., 1H), 7.68 - 7.56 (m, 2 H), 7.40 - 7.30 (m, 3 H), 7.26 - 7.21 (m, 1 H), 7.13 (t, *J*= 8.4 Hz, 1H), 6.75 (d, *J*= 8.3 Hz, I H), 4.03 (br. s., 2 H), 3.78 (s, 3 H). LC/MS = 98.5%, 353.0 (APCI+).

### A-042 MCLS1471-075-4 (Ex- 428)

### 5-(3'-Chloro-2-fluoro-6-methoxy-biphenyl-3-ylmethyl)-2-(1H-tetrazol-5-yl)-pyridine

Into a 20 mL vial was added **Int-27** (129 mg, 0.37 mmol), NH₄Cl (137 mg, 2.56 mmol), NaN₃ (119 mg, 1.83 mmol), and DMF (3 mL). The reaction was stirred for 18 hours at 80°C, then filtered and concentrated. The residue was purified by FCC eluting with 25 - 75% acetone/DCM, then 10% methanol/DCM to afford a colorless oil. The oil was treated with ether to afford **A-042** (13 mg, 9%) as a tan solid. ¹H NMR (400MHz ,DMSO-d₆) δ = 8.49 (br. s., 1 H), 7.95 (br. s., 1H), 7.66 (br. s., 1H), 7.49 - 7.33 (m, 4 H), 7.32 - 7.25 (m, 1H), 6.96 (d, *J*= 8.6 Hz, I H), 4.00 (br. s., 2 H), 3.73 (s, 3 H). LC/MS = 100.0%, 396.0 (APCI+).

### Synthesis of A-047

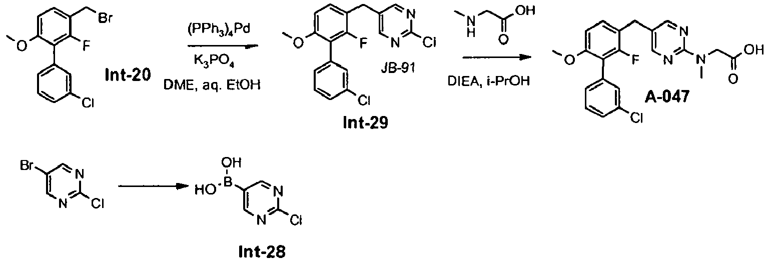

### (Int-28) [MCLS1448-146-1] Synthesis of 2-chloro-pyrimidine-5-boronic acid.

A 200mL round bottom was charged under nitrogen with 5-bromo-2-chloro pyrimidine (30mmol, 5.79g), toluene (48mL), THF (12mL) and triisopropyl borate (36mmol, 8.4mL). The mixture was stirred at -70°C and n-butyl lithium (2.5M in hexane, 36mmol, 14.4mL) was added slowly (1.5h period). After 2h of stirring at -70°C, the reaction was warmed to -20°C before 2N HCl (30mL) was added. Upon the HCl addition the reaction mixture turned from a pale homogeneous solution to a white bi-phasic solution. When the mixture reached rt, it was transferred to a reparatory funnel and the layers were separated. The aqueous layer was neutralized to PH=7 with 1N NaOH followed by extractions with THF (3 x 150mL). The combined organic layers were dried over NA2SO4 and concentrated. The crude was dissolved in THF (5mL) and ether (100mL) was added. A yellow solid precipitated out. The solid was collected and dried to give Int-28 (3.79g) as a pale yellow solid.

### (Int-29) [MCLS 1226-099-1] Synthesis of 3'-Chloro-2-fluoro-3-(4-iodo-benzyl)-6-methoxy-biphenyl:

Int-20 (2.5mmol, 824mg) and **Int-28** (2.5mmol, 400mg) were dissolved in toluene (10mL) and ethanol (2.5mL). Pd (PPh₃)₄ (144mg, 0.125mmol) was added followed by Na₂CO₃ in water (2M, 5mL, 2.5mmol). The reaction mixture was heated overnight at 85°C. The resultant mixture was diluted with EtOAc (25mL), washed with water and brine, and then dried over Na₂SO₄. The organic solution was filtered and concentrated under reduced pressure. The crude was purified by by silica gel (40g) column(1/2 inch diameter) chromatography, eluted with Hexane/EtOAc (9:1) gave the title compound (297.4mg) as a pale yellow solid.

### A-047 [MCLS 1457-144-1]. Synthesis of {[5-(3'-Chloro-2-fluoro-6-methoxy-biphenyl-3-ylmethyl)-pyrimidin-2-yl[-methyl-amino}-acetic acid:

In an 8 mL vial equipped with a stir bar was placed Int-29 (120 mg, 0.330 mmol), N-methylglycine (44.1 mg, 0.495 mmol), isopropanol (1.5 mL) and diisopropylethylamine (172 µL, 0.990 mmol). The reaction mixture was heated to 80 °C for 18 hours. This material was combined with the reaction mixture of an identical reaction run on a 20 mg scale. The mixture was treated with saturated sodium bicarbonate solution (60 mL), water (30 mL), (pH 8) and extracted with ethyl acetate (2 x 50 mL) to remove organic impurities. The organic portions were combined, washed with saturated sodium bicarbonate solution (75 mL) and all of the aqueous portions were then combined and acidified to pH 1 with 3M hydrochloric acid. The aqueous portion was extracted with ethyl acetate (3 x 75 mL) and the organic portions were combined, washed with brine (100 mL), dried (magnesium sulfate) and concentrated to produce 44 mg of A-047 as a white powder in 27% yield.
¹H NMR (400 MHz, DMSO-*d*₆) δ 3.1 (s, 3 H), 3.72 (s, 3 H), 3.77 (s, 2 H), 4.24 (s, 2 H), 6.93 (d, *J* = Hz, 1H), 7.284-7.328 (m, 2 H), 7.38 (s, 1 H), 7.43-7.46 (m, 2 H), 8.25 (bs, 2 H).

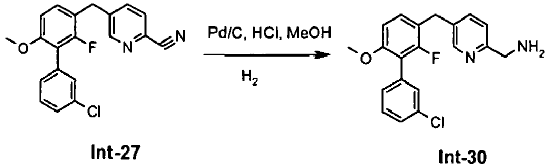

### Int-30 MCLS1430-114-3

### C-[5-(3'-Chloro-2-fluoro-6-methoxy-biphenyl-3-ylmethyl)-pyridin-2-yl]-methylamine

Into a 100 mL RBF was added **Int-27** (0.55 g, 1.56 mmol), methanol (20 mL), concentrated HCl (0.65 mL, 7.79 mmol), and 10% Pd/C (100 mg). The suspension was stirred under a H₂ balloon for 18 hours, and then filtered through Celite. The filtrate was concentrated. To the solid was added 1N NaOH and the product was extracted with DCM. The DCM was concentrated and purified by FCC eluting with 5-10% methanol/DCM to give **Int-30** (89 mg, 16%) as a colorless oil. ¹H NMR (400MHz ,DMSO-d₆) δ = 8.55 (d, *J* = 1.2 Hz, 1 H), 8.32 (br. s., 3 H), 7.71 (dd, *J* = 1.9, 7.9 Hz, 1 H), 7.50 - 7.37 (m, 3 H), 7.36 (d, *J* = 5.4 Hz, 2 H), 7.27 (d, *J* = 6.6 Hz, 1 H), 6.96 (d, *J* = 8.6 Hz, 1 H), 4.75 (br. s., 2 H), 4.14 (q, *J* = 5.8 Hz, 2 H), 4.01 (s, 2 H), 3.73 (s, 3 H). LC/MS = 95.1%, 357.1 (APCI+).

### A-048 MCLS1471-115-2

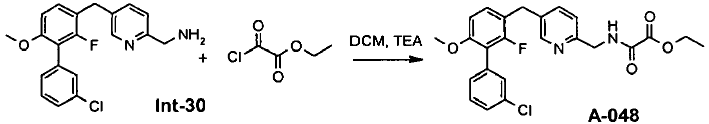

### N-[5-(3'-Chloro-2-fluoro-6-methoxy-biphenyl-3-ylmethyl)-pyridin-2-ylmethyl]-oxalamic acid ethyl ester

Into an 18 mL vial was added **Int-30** (152 mg, 0.43 mmol), TEA (0.11 mL, 0.85 mmol), and DCM (4 mL). The solution was cooled to 0 °C and Ethyl chlorooxoacetate (71 uL, 0.64 mmoL) was added. After 20 minutes at room temperature the reaction was washed with brine and the organics were concentrated. The semi-solid was triturated with 1:1 ether:EtOAc, filtered, and washed with EtOAc to provide the title compound (69 mg, 35%) as a gray-blue solid. ¹H NMR (400MHz ,DMSO-d*₆*) δ = 9.38 (t, *J*= 6.0 Hz, I H), 8.41 (s, 1H), 7.59 (dd, *J=* 4.0, 8.0 Hz, 2 H), 7.48 - 7.25 (m, 8 H), 7.22 (d, *J=* 8.1 Hz, 2 H), 6.94 (d, *J=* 8.6 Hz, 2 H), 4.39 (d, *J=* 6.0 Hz, 2 H), 4.32 - 4.18 (m, 2 H), 3.94 (s, 2 H), 3.72 (s, 3 H), 1.27 (t, *J* = 7.1 Hz, 3 H). LC/MS = 97.9%, 457.0 (APCI+).

### A-050 MCLS1471-143-2

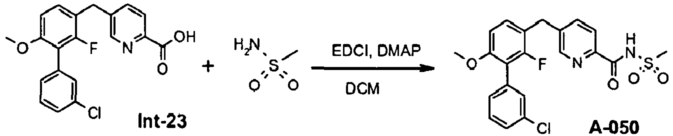

### N-[5-(3'-Chloro-2-fluoro-6-methoxy-biphenyl-3-ylmethyl)-pyridine-2-carbonyl]-methanesulfonamide

Into an 8 mL vial was added **Int-23** (85 mg, 0.23 mmol), Methanesulfonamide (26 mg, 0.27 mmoL), EDCI (88 mg, 0.46 mmol), DMAP (56 mg, 0.46 mmol), DCM (2 mL), and 0.5 mL of DMSO. After stirring for 3 days at room temperature 2 mL of water was added and the layers were separated. The organic layer was washed with 2 X 2 mL NH₄Cl (sat) and then concentrated. The residue was purified by flash column chromatography eluting with 50% Acetone/DCM. This material was triturated with ether to afford the title compound (27.2 mg, 26%) as a white solid. ¹H NMR (400MHz ,DMSO-d₆) δ = 8.66 (br. s., 1 H), 7.98 (d, *J*= 7.9 Hz, 1H), 7.72 (d, *J*= 7.5 Hz, 1 H), 7.50 - 7.32 (m, 4 H), 7.28 (d, *J*= 6.4 Hz, 1 H), 6.96 (d, *J*= 8.6 Hz, 1 H), 4.05 (s, 2 H), 3.73 (s, 3 H), 2.95 (s, 3 H). LC/MS = 100.0%, 449.0 (APCI+).

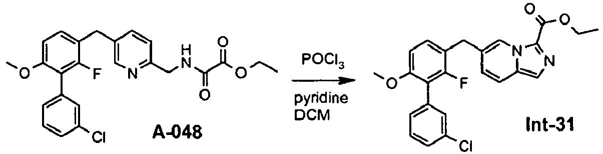

### Int-31 MCLS1471-127-1

### 6-(3'-Chloro-2-fluoro-6-methoxy-biphenyl-3-ylmethyl)-imidazo[1,5-a] pyridine-3-carboxylic acid ethyl ester

Into a 4 mL vial was added **A-048** (44mg, 0.096 mmol), DCM (1 mL), pyridine (31 uL, 0.48 mmol), and POCl₃ (13 uL, 0.14 mmol). The reaction was stirred at room temperature for 18 hours after which 2 mL of water was added. The layers were separated and the product was extracted with 2 mL of DCM. The organics were combined, concentrated, and purified by flash column chromatography eluting with 20% Acetone/Hexanes to afford a light-yellow semi-solid. Trituration of the residue with hexanes provided a solid which was filtered, washed with hexanes, and dried. The title compound was obtained as a tan solid (8.2 mg, 19%).

### A-051 MCLS1471-140-1

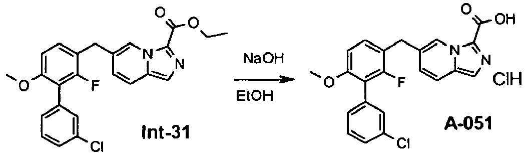

### 6-(3'-Chloro-2-fluoro-6-methoxy-biphenyl-3-ylmethyl)-imidazo[1,5-a] pyridine-3-carboxylic acid hydrochloride

Into an 8 mL vial was added **Int-31** (42 mg, 0.14 mmol), EtOH (1 mL), and 1N NaOH (1 mL). The reaction was stirred at 60 °C for 1 hour. After cooling to room temperature some insoluble matter was removed by filtration through a nylon filter. The EtOH was evaporated and the pH of the remaining aqueous phase was adjusted to pH = 1 with 1N HCl. The solids which formed were filtered, washed with water, and dried under vacuum to afford the title compound **(A-051)** (14.2 mg, 23%) as a yellow solid. ¹H NMR (400MHz, DMSO-d₆) δ = 9.15 (s, 1 H), 7.79 (d, *J*= 9.3 Hz, 1 H), 7.65 (s, 1H), 7.51 - 7.34 (m, 4 H), 7.33 - 7.25 (m, 1 H), 7.10 (s, 1 H), 6.97 (d, *J*= 8.6 Hz, 1 H), 4.02 (s, 2 H), 3.74 (s, 3 H).

### Synthesis of A-052

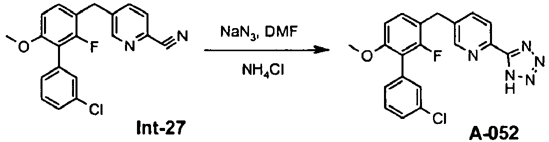

### A-052 MCLS1471-075-4

### 5-(3'-Chloro-2-fluoro-6-methoxy-biphenyl-3-ylmethyl)-2-(1H-tetrazol-5-yl)-pyridine

Into a 20 mL vial was added **Int-27** (129 mg, 0.37 mmol), NH₄Cl (137 mg, 2.56 mmol), NaN₃ (119 mg, 1.83 mmol), and DMF (3 mL). The reaction was stirred for 18 hours at 80 °C, then filtered and concentrated. The residue was purified by FCC eluting with 25 - 75% acetone/DCM, then 10% methanol/DCM to afford a colorless oil. The oil was treated with ether to afford **A-052** (13 mg, 9%) as a tan solid. ¹H NMR (400MHz ,DMSO-d₆) δ = 8.49 (br. s., 1 H), 7.95 (br. s., 1 H), 7.66 (br. s., 1 H), 7.49 - 7.33 (m, 4 H), 7.32 - 7.25 (m, 1 H), 6.96 (d, *J*= 8.6 Hz, 1 H), 4.00 (br. s., 2 H), 3.73 (s, 3 H). LC/MS = 100.0%, 396.0 (APCI+).

### Synthesis of A-055

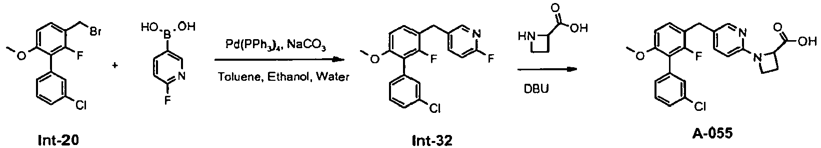

### Int-32 [MCLS1448-171-1]. Synthesis of 5-(3'-Chloro-2-fluoro-6-methoxy-biphenyl-3-ylmethyl)-2-fluoro-pyridine.

A suspension of **Int-20** (824 mg, 2.50 mmol), 2-fluoropyridin-5-boronic acid (352 mg, 2.50 mmol), 2 M aqueous sodium carbonate (2.5 mL, 5.00 mmol), and palladium(0)tetrakis(triphenylphosphine) (144 mg, 0.125 mmol) in toluene (10 mL) and ethanol (2.5 mL) was stirred at 80°C overnight under a high pressure nitrogen atmosphere. The reaction was cooled to room temperature, diluted with water (10 mL), and extracted with ethyl acetate (2 x 20 mL). The combined extracts were dried over sodium sulfate, filtered, and concentrated under vacuum. The residue was purified by silica gel column chromatography (4:1 hexanes/ethyl acetate) to give Int-32 (694.7 mg, 78% yield) as a white solid.

### A-055 [MCLS1486-102-1]. Synthesis of 1-[5-(3'-chloro-2-fluoro-6-methoxy-biphenyl-3-ylmethyl)-pyridin-2-yl]-azetidine-2-carboxylic acid:

To 5-(3'-chloro-2-fluoro-6-methoxy-biphenyl-3-ylmethyl)-2-fluoro-pyridine **(Int-32)** (0.2 g, 0.58 mmol) and azetidine-2-carboxylic acid (2) (0.18 g, 1.74 mmol) was added 1,8-diazabicyclo[5.4.0]undec-7-ene (DBU) (0.44 g, 2.89 mmol). The reaction mixture was stirred and heated at 160 °C for 20 min. Cooled to room temperature, diluted with dichloromethane (8 mL), washed with 0.5 N HCl (2 X 4 mL), dried with Na₂SO₄, filtered, and concentrated. The residue was purified by silica gel column chromatography using 3% methanol in dichloromethane to afford 0.19 g (77%) of **A-055** as off-white solid. 1H NMR (DMSO-d6, 400MHz): 7.87 (d, *J*= 2.0 Hz, 1 H), 7.4-7.47 (m, 2 H), 7.37 (s, 1 H), 7.2-7.3 (m, 3 H), 6.91 (d, *J* = 8.4 Hz, 1 H), 6.55 (d, *J* = 8.8 Hz, 1H), 4.08 (dd, J= 9.0, 6.8 Hz, 1H), 3.62-3.74 (m, 2 H), 3.74 (s, 2 H), 3.71 (s, 3 H), 2.18-2.3 (m, 2 H); MS(APCI+): 427.1 (M+1), LC-MS: 97.1%; HPLC 95.2%

### Synthesis of A-062

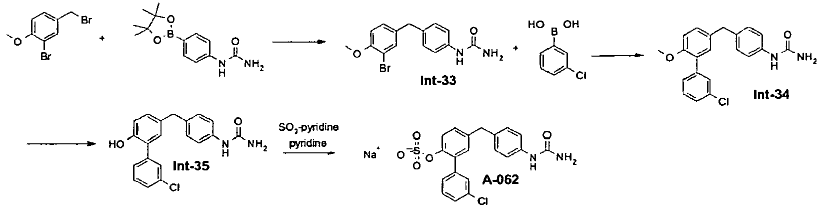

### Int-33 MCLS1435-080-1

### Synthesis of [4-(3-Bromo-4-methoxy-benzyl)-phenyl]-urea (Int-33)

To a 40 mL vial equipped with a teflon screw cap and a magnetic stir bar was added 2-bromo-4-bromomethyl-1-methoxybenzene (649 mg, 2.32 mmol), [4-(4,4,5,5-tetramethyl-[1,3,2]dioxaborolan-2-yl)-phenyl]-urea (577 mg, 2.20 mmol) and potassium phosphate 933 mg, 4.40 mmol). To the vial was then added dimethoxyethane (15 mL), ethanol (3.7 mL) and water (3.7 mL). To this stirring solution was added tetrakis(triphenylphosphine) palladium (127 mg, 0.11 mmol) and the solution was degassed by bubbling N2 gas through the solution for 20 min. The vial was capped and placed in an oil bath with stirring at 65 °C for 12.5 h. The cooled reaction mixture was concentrated under a stream of N2 gas to a total volume of ∼5 mL and then diluted with ethyl acetate (20 mL) and water (10 mL). Upon shaking a white solid precipitates. The solid is filtered and dried to afford 272 mg (34%) of compound **Int-33** as a white solid. 1H NMR (400 MHz, DMSO-d6) 3.78 (s, 2H), 3.80 (s, 3H), 5.77 (br s, 2H), 7.01 (d, J = 8.4 Hz, 1H), 7.05 (m, 2H), 7.17 (dd, J = 8.4, 2.0 Hz, 1H), 7.29 (m, 2H), 7.37 (d, J = 2.0 Hz, 1H), 8.43 (br s, 1H). MS(APCI+): 335.0 (M⁺); 337.0 (M⁺+2), LC-MS: 99.3%

### Int-34 MCLS1435-084-1

### Synthesis of [4-(3'-Chloro-6-methoxy-biphenyl-3-ylmethyl)-phenyl]-urea.

To a 20 mL vial equipped with a magnetic stir bar and a screw cap was added compound Int-33 (250 mg, 0.746 mmol), dimethoxyethane (5 mL), ethanol (1 mL) and water (1 mL). To this mixture was added 3-chlorophenylboronic acid (140 mg, 0.895 mmol), potassium phosphate (316 mg, 1.49 mmol) and tetrakis(triphenylphosphine) palladium (30 mg, 0.0254 mmol). The stirring reaction mixture was degassed by bubbling N₂ gas through the solution for 10 min. The vial was capped and placed in an oil bath with stirring at 80 °C for 16 h. The cooled reaction mixture was concentrated to dryness, then diluted with water (5 mL) and ethyl acetate (15 mL). The aqueous layer was extracted with ethyl acetate (2 x 5 mL) and the combined organic extracts were dried (Na₂SO₄), filtered and concentrated under a stream of N2 gas. The residue was purified by flash chromatography on silica gel (35 g) utilizing 9:1 dichloromethane/acetone as eluent. Fractions pure by TLC were combined and concentrated to give 139 mg (51%) of Int-34 as an off-white solid. 1H NMR (400 MHz, DMSO-d6) 3.74 (s, 3H), 3.83 (s, 2H), 5.75 (br s, 2H), 7.03 (d, J = 8.4 Hz, 1H), 7.08 (m, 2H), 7.15 (d, J = 2.4 Hz, 1H), 7.18 (dd, J = 8.4, 2.4 Hz, 1H), 7.28 (m, 2H), 7.36-7.41 (m, 3H), 7.48 (m, 1H), 8.40 (br s, 1H). MS(APCI+): 367.0 (M⁺+1) ; LC-MS: 95.9%.

### Int-35 MCLS1489-012-1

To a suspension of **Int-34** (2.2 mmol, 1.0 eq.) in dichloromethane at -70°C was added a 1.0 M solution of boron tribromide in dichloromethane (6.6 mmol, 3 cq) over 5 minutes. The resultant mixture was allowed to stir and warm to ambient temperature, and stirred for 20 minutes. The reaction was poured into ice water (100 mL), stirred for 30 minutes, filtered, and the cake washed with successive portions of water and hexanes. The solids were dried *in vacuo* over ethyl acetate vapors for 4 hours to afford the title compound, which was taken into further reactions as is. A portion of the title compound was further purified via chromatography on silica gel using acetone in dichloromethane as eluent, followed by trituration with dichloromethane to afford the title compound as a white solid. (¹NMR, DMSO-d₆; 400 MHz): 3.78 (s, 2H), 5.75 (s, 2H), 6.86 (d, J = 8.0 Hz, 1H), 7.00 (dd, J = 8.4, 2.4 Hz, 1H), 7.07 (d, J = 8.4 Hz, 2H), 7.12 (d, J = 2.4 Hz, 1H), 7.28 (d, J = 8.4 Hz, 2H), 7.4 (m, 1H), 7.41 (dd, J = 8.0, 7.6 Hz, 1H), 4.47 (m, 1H), 7.56 (m, 1H), 8.40 (s, 1H), 9.50 (s, 1H). LC/MS(100%) APCI⁺ found : 353.0, calc'd : 352.8 m/z

### A-062 [MCLS1457-189-4]. Synthesis of Sodium sulfate of [4-(3'-Chloro-6-hydroxy-biphenyl-3-ylmethyl)-phenyl]-urea:

In an 18 mL vial equipped with a stir bar and atmosphere of nitrogen was placed Int-35 (250 mg, 0.709 mmol), pyridine (2.5 mL) and sulfur trioxide-pyridine complex (226 mg, 1.42 mmol). The mixture was sirred at room temperature for 18 hours. Additional sulfur trioxide-pyridine complex (100 mg) was added and the reaction mixture was heated to 70 °C for 4 hours. The reaction mixture was concentrated with a stream of nitrogen, methanol (2.5 mL) was added and then 1M NaOH was added to attain pH 8. A white solid formed which was removed via suction filtration. The filtrate was concentrated and diethyl ether (3 mL) was added which resulted in a yellowish semi-solid. The solid was collected, and dried in a high vacuum oven at 35 °C for 5 hours resulting in an off-white powder. The material was purified by dissolving the crude material in water (4 mL, made basic by addition of a few drops of concentrated ammonium hydroxide) and washing with ethyl aceate (3 x 4 mL). The aqueous portion was lyophilized overnight to produce 56 mg of **A-062** as a fluffy white solid in 17% yield.
¹H NMR (400 MHz, DMSO-*d₆*) δ 3.83 (s, 2 H), 5.88 (s, 2 H), 6.95 (d, *J*= 6 Hz, 1 H), 7.08-7.13 (m, 3 H), 7.31 (d, *J*= 9 Hz, 2 H), 7.34-7.36 (m, 1 H), 7.40 (t, *J*= 8 Hz, 1 H), 7.48-7.50 (m, 1H), 7.56 (d, *J*= 8 Hz, 1H), 7.64 (t, *J*= 2 Hz, 1H), 8.74 (bs, 1 H). LC-MS: 98%

### Synthesis of A-064 and A-065

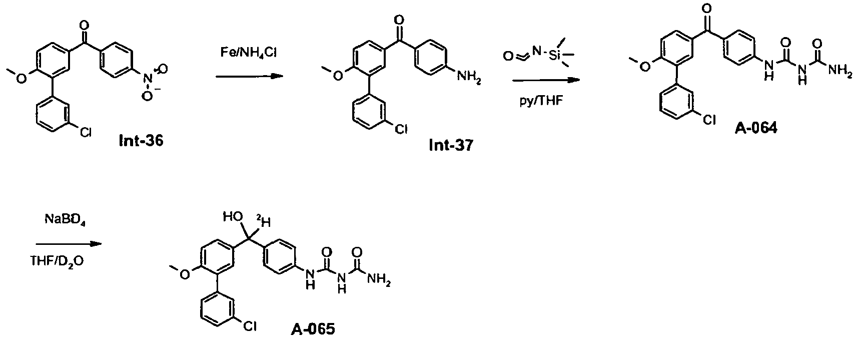

### Int-37 MCLS1475-078-2] Synthesis of (4-amino-phenyl)-(3'-chloro-6-methoxy-biphenyl-3-yl)-methanone.

To a 25 mL vial which contained (3'-chloro-6-methoxy-biphenyl-3-yl)-(4-nitro-phenyl)-methanone (**Int-36 (prepared in a similar manner as Int-12**), 500 mg, 1.3 mmole) in EtOH-H2O (1:1, 15 mL) was added NH4Cl (200 mg, 4 mmole) and then Fe (150 mg, 3 mmole) at rt. The reaction mixture was allowed to stir at rt for 72 h. The mixture was poured onto 50 mL water, extracted with ethyl acetate (3x30 mL), washed with water (20 mL), brine (30 mL) and dried over Na₂SO₄. The solvent was rermoved under vacuum to yield 420 mg (4-amino-phenyl)-(3'-chloro-6-methoxy-biphenyl-3-yl)-methanone (Int-37) in 85% yield 1H NMR (DMSO-d6 ,400MHz): δ = 7.70 (dd, J=8.4, 2.4 Hz, 1 H), 7.40-7.57 (m, 7 H), 7.25 (d, J=8.8 Hz, 1 H), 6.61 (d, J=8.8 Hz, 2 H), 6.09 (s, 2 H), 3.88 (s, 3 H)

### A-064 [MCLS1475-079-3] Synthesis of 1-acetic amido-3-[4-(3'-chloro-6-methoxy-biphenyl-3-carbonyl)-phenyl]-urea.

To a 25 mL vial which contained (4-amino-phenyl)-(3'-chloro-6-methoxy-biphenyl-3-yl)-methanone **(Int-37,** 420 mg, 1.3 mmol) in pyridine (2 mL) and THF (8 mL) was added TMS-isocyanate (1.7 mL, excess) at rt. The mixture was allowed to stir at rt for 100 h. The mixture was poured onto 25 mL ice-water solution, 5 mL of sat. NaHCO3 was added and the mixture which formed was allowed to stir at rt for 2 h. The mixture was extracted with ethyl acetate (3x30 mL), washed with water (20 mL), brine (30 mL) and dried over Na₂SO₄. The solvent was removed to provide the crude which was separated by silica gel column chromatography with ethyl acetate-hexane as eluent to yield 250 mg of 1-acetic amido-3-[4-(3'-chloro-6-methoxy-biphenyl-3-carbonyl)-phenyl]-urea **(A-064)** in 48.4 % yield. 1H NMR (DMSO-d6 ,400MHz): δ = 10.6 (br, s, 1 H), 9.37 (br. s., 1H), 7.40-7.81 (m., 7 H), 7.30 (d, J=8.4 Hz, 1 H), 6.84-7.10 (m, 2 H), 3.90 (s, 3 H). LC/MS Calc.423.86; APCI+(M+1): 424.1, 95%

### A-065 [MCLS1475-080-1]. Synthesis of 1-acetic amido-3-{4-[(3'-chloro-2-fluoro-6-methoxy-biphenyl-3-yl)-hydroxy-deutero-methyl]-phenyl}-urea.

To a 50 mL flask which contained 1-acetic amido-3-[4-(3'-chloro-6-methoxy-biphenyl-3-carbonyl)-phenyl]-urea (**A-064,** 100 mg, 0.3 mmole) in THF (7 mL) and D2O (3 mL) was added NaBD4 (130 mg, 0.7 mmole) at 0 °C. The reaction mixture was allowed to warm to rt and stir at rt for 24 h. The reaction mixture was poured onto 40 mL ice-water, neutralized with NH4Cl (sat. 4 mL), extracted with ethyl acetate (3x30 mL), washed with water (20 mL), brine (20 mL) and dried over Na₂SO₄. After removal of solvent, the residue was separated by silica gel column chromatography with ethyl acetate-hexane as eluent to give 70 mg of 1-acetic amido-3-{4-[(3'-chloro-2-fluoro-6-methoxy-biphenyl-3-yl)-hydroxy-deutero-methyl]-phenyl}-urea in 26% yield. 1H NMR (DMSO-d6 ,400MHz): δ = 9.89 (s, 1 H), 8.80 (s, 1H), 7.47 (br, s, 1H), 7.42 (d, J= 6.8 Hz, 1 H), 7.28 -7.39 (m, 7 H), 7.05 (d, J= 7.2 Hz, 1H), 5.75 (s, 1H), 3.74 (s, 3 H). LC/MS Calc.426.87; APCI⁺(M-OH): 309.1, 98%

### Synthesis of A-035, A-056, A-037, and A-057

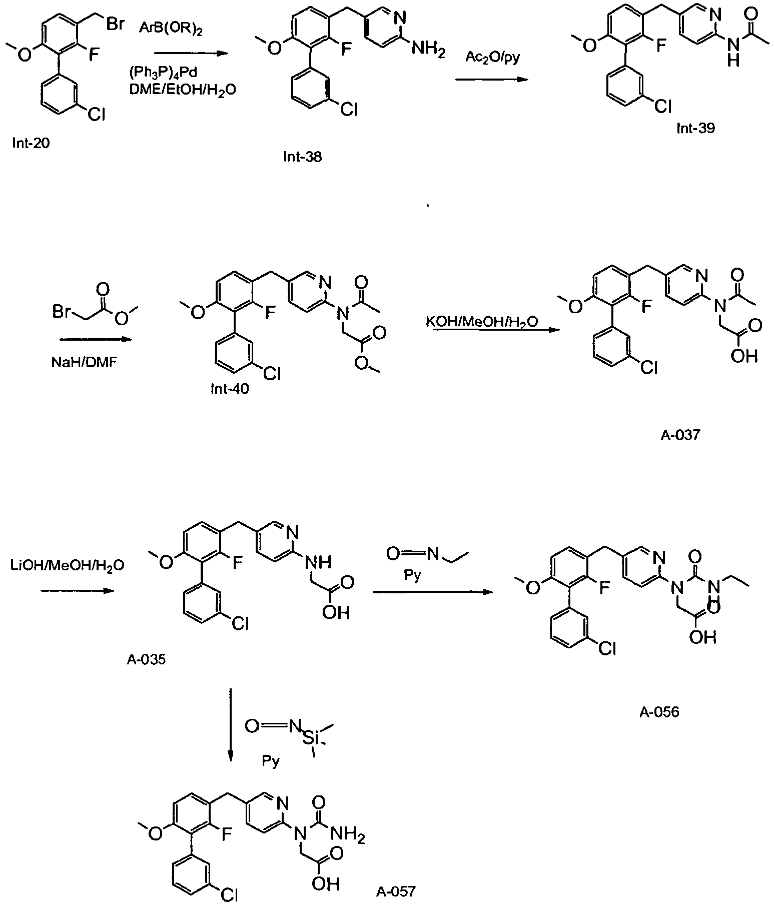

**(Int-38)** [MCLS 1316-161-4] **Synthesis of 5-(3'-Chloro-2-fluoro-6-methoxy-biphenyl-3-ylmethyl)-pyridin-2-ylamine** A mixture of **Int-20** (0.3 g, 0.91 mmol) and 2-aminopyridine-5-boronic acid pinacol (0.24 g, 1.1 mmol) in DME/EtOH/H2O (4/1/1, 12 ml) was added potassium phosphate (0.39 g, 1.82 mmol) and tetrakis(triphenylphosphine)palladium (0) (53 mg, 0.046 mmol) under nitrogen. The reaction mixturc was hcatcd to 80 C for 2 h. The reaction was dilutcd with watcr, extracted with ethyl acetate, washed with water and brine, and dried over Na₂SO₄. After it was concentrated in vacuo, the residue was purified by a chromatography on silica gel to yield the desire product, which was converted to its HCl salt **Int-38** (0.165 g, 50%).

### (Int-39) [MCLS1325-188-1 Synthesis of N-[5-(3'-chloro-2-fluoro-6-methoxy-biphenyl-3-ylmethyl)-pyridin-2-yl]-acetamide

To a 20 mL vial which contained the mixture of 5-(3'-Chloro-2-fluoro-6-methoxy-biphenyl-3-ylmethyl)-pyridin-2-ylamine (**Int-38**, 420 mg, 1.2 mmol) in pyridine (2 mL) was added Ac₂O (2 mL, excess) at 0 C. The mixture was allowed to warm to rt and stir at rt for 16 h and then was poured onto 50 mL ice-water solution. The solid which formed was filtered out, washed with water (3x15 mL), dried over air to yield 360 mg (85% yield) of the title product (**Int-39**).

### (Int-40) [MCLS1 325-201-2] Synthesis of {acetyl-[5-(3'-chloro-2-fluoro-6-methoxy-biphenyl-3-ylmethyl)-pyridin-2-yl]-amino}-acetic acid methyl ester

To a 25 mL vial which contained NaH (40% in mineral oil, 40 mg, 1 mmol) in DMF (2 mL) was added N-[5-(3'-chloro-2-fluoro-6-methoxy-biphenyl-3-ylmethyl)-pyridin-2-yl]-acetamide (**Int-39**, 77 mg, 0.2 mmol) at 0 °C. The reaction mixture was allowed to warm to rt and stir at rt for 1h, cooled to 0 °C, bromo-acetic acid methyl ester (153 mg , 1.0 mmol) was added. The mixture was allowed to warm to rt and stir at rt for 16 h and then was poured onto 25 mL ice-water solution which was extracted with ethyl acetate (3x 20 mL), washed with water (3x20 mL), brine (20 mL) and dried over Na2SO4. After removal of solvent, the residue was purified by silica gel column chromatography with ethyl acctate-hexane as clucnt to give 68 mg of the title compound in 74 % yield.

### A-035, [MCLS1325-207-1] Synthesis of [5-(3'-chloro-2-fluoro-6-methoxy-biphenyl-3-ylmethyl)-pyridin-2-ylamino]-acetic acid

To a 20 mL vial which contained **Int-40** (37 mg, 0.08 mmol) in MeOH (2 mL) was added LiOH (1 N in water, 2 mL, 2 mmol) at rt. The reaction mixture was allowed to stir at rt for 48 h. The mixture was poured onto 25 mL ice-water solution, acidified with the addition of 2N HCl to pH=1-2 and then was extracted with ethyl acetate (3x 20 mL), washed with water (3x20 mL), brine (20 mL) and dried over Na2SO4. After removal of solvent, the crude was purified by dissolving in acetone, adding 30 mL 1:9 ethyl acetate-hexane and then removal the acetone slowly to yield the semi-solid which was separated from the aq. layer and dried under vacuum to give 20 mg of **A-035** in 60 % yield.
1H NMR (DMSO-d6 ,400MHz): δ = 7.85 (br, s, 1 H), 7.68-7.80 (m, 2 H), 7.28-7.46 (m, 5 H), 7.07-7.10 (m, 1H), 6.96 (d, J=8.8 Hz, 1 H), 4.18 (s, 2 H), 3.88 (s, 2 H), 3.73 (s, 3 H). Calc.400.8; APCI⁺(M+1): 401.0

### A-056 [MCLS1475-056-3] Synthesis of {1-[5-(3'-chloro-2-fluoro-6-methoxy-biphenyl-3-ylmethyl)-pyridin-2-yl]-3-ethyl-ureido}-acetic acid

To a 20 mL vial which contained **A-035** (18 mg, 0.05 mmol) in pyridine (2 mL) was added ethylisocyanate (0.2 mL, excess) at rt. The mixture was allowed to stir at rt for 48 h. The mixture was poured onto 20 mL ice-water solution and the solid which formed was filtered out, washed with water (20 mL) and dissolved in dioxane-H2O (1:1, 10 mL). 250 mg KOH was added to the solution at rt. The mixture was allowed to stir at rt for 16 h and then the solution was adjusted to pH= 1 with the addition of 2 N HCl, extracted with ethyl acetate (3x 20 mL), washed with water (20 mL), brine (20 mL) and dried over Na2SO4. After removal of solvent, crude was separated by a chromatography on silica gel with ethyl acetate-hexane as eluent to give 17 mg of **A-056** in 50 % yield.
1H NMR (CHLOROFORM-d ,400MHz): δ = 8.20 (br, s, 1 H), 8.15 (d, J=8.8 Hz, 1 H), 7.57 (dd, J=8.8, 2.4 Hz, I H), 7.25 - 7.39 (m, 4 H), 7.09 (t, J=8.4 Hz, 1 H), 6.71 (d, J=8.4 Hz, 1H), 4.50 (s, 2 H), 3.93 (s, 2 H), 3.74 (s, 3 H), 3.66 (q, J=7.6 Hz, 2 H), 1.27 (t, J=7.6 Hz, 3 H),). LC/MS Calc.471.9; APCT⁺(M-OH): 454.1, 100%

### A-057 [MCLS 1475-62-2] Synthesis of {1-[5-(3'-chloro-2-fluoro-6-methoxy-biphenyl-3-ylmethyl)-pyridin-2-yl]-ureido}-acetic acid

To a 20 mL vial which **A-035** (80 mg, 0.2 mmol) in pyridine (5 mL) was added TMS-isocyanate (0.5 mL, excess) at rt. The mixture was allowed to stir at rt for 48 h. The mixture was poured onto 20 mL ice-water solution and the solid which formed was filtered out, washed with water (20 mL) and dissolved in dioxane-5%NaHCO3 (1:1, 10 mL). The mixture was allowed to stir at rt for 24 h. After the solution was adjusted to pH= 1 with the addition of 2H HCl, the solid which formed was filtered out, washed with water (20 mL), dried through air to yield 20 mg of desired **A-057** in 22 % yield.
1H NMR (DMSO-d6 ,400MHz): δ = 11.26 (s, 1 H), 8.24 (d, J=2.0 Hz, 1 H), 8.05 (d, J=8.4 Hz, 1 H), 7.67 (dd, J=8.8, 2.4 Hz, 1H), 7.25 - 7.49 (m, 5 H), 6.93 (d, J=8.8 Hz, 1 H), 4.43 (s, 2 H), 3.92 (s, 2 H), 3.72 (s, 3 H), 3.57 (s, 3 H). LC/MS Calc.443.9; APCI⁺(M-OH):426.1, 100%

### A-037 [MCLS1475-013-1] Synthesis of (acetyl-[5-(3'-chloro-2-fluoro-6-methoxy-biphenyl-3-ylmethyl)-pyridin-2-yl]-amino) -acetic acid.

To a 20 mL vial which contained **Int-40** (150mg, 0.34 mmol) in MeOH (2 mL) was added KOH (1 N in water, 2 mL, 2 mmol) at 0 °C. The reaction mixture was allowed to warm to rt and stir at rt for 24 h. The mixture was cooled to 0 °C, acidified with the addition of 2N HCl to pH=1-2 and then 20 mL water was added. The mixture was extracted with CH2C12-MeOH (20:1, 5 x 20 mL). The combined organic layers washed with brine (20 mL) and dried over Na2SO4. Removal of solvent under vacuum to afford 120 mg of the title compound (**A-037**) in 77 % yield. 1 H NMR (DMSO-d6 ,400MHz): δ = 8.33 (s, 1H), 7.85 (br, s, 1 H), 7.78-7.80 (m, 2 H), 7.28-7.46 (m, 4 H), 6.95 (d, J=8.0 Hz, I H), 4.44 (s, 2 H), 3.97 (s, 2 H), 3.73 (s, 3 H), 2.07 (s, 3H).

### Synthesis of A-061

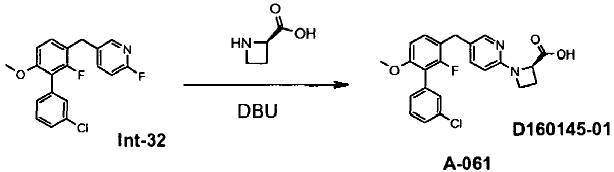

### A-061 [MCLS1486-111-1]. Synthesis of (R)-1-[5-(3'-chloro-2-fluoro-6-methoxy-biphenyl-3-ylmethyl)-pyridin-2-yl]-azetidine-2-carboxylic acid:

To 5-(3'-chloro-2-fluoro-6-methoxy-biphenyl-3-ylmethyl)-2-fluoro-pyridine (**Int-32**) (0.2 g, 0.58 mmol) and D-azetidine-2-carboxylic acid (2) (0.18 g, 1.74 mmol) was added 1,8-diazabicyclo[5.4.0]undec-7-ene (DBU) (0.44 g, 2.89 mmol). The reaction mixture was stirred and heated at 160°C for 20 min. Cooled to room temperature, diluted with dichloromethane (8 mL), washed with 0.5 N HCl (2X4 mL), dried with Na₂SO₄, filtered, and concentrated. The residue was purified by silica gel column chromatography using 3% methanol in dichloromethane to afford 0.1 g (40%) of **A-061** as an off-white solid. 1H NMR (DMSO-d6, 400MHz): 7.98 (d, *J*= 2.0 Hz, 1 H), 7.4-7.47 (m, 3 H), 7.37 (s, 1H), 7.24-7.3 (m, 2 H), 6.92 (d, *J* = 8.4 Hz, 1 H), 6.41 (d, *J* = 8.8 Hz, 1 H), 4.08 (dd, *J* = 8.8, 7.2 Hz, 1 H), 3.7-3.792 (m, 2 H), 3.8 (s, 2 H), 3.72 (s, 3 H), 2.3-2.56 (m, 2 H); MS(APCI+): 427.1 (M+1), LC-MS: 90.1%.

### Synthesis of A-063

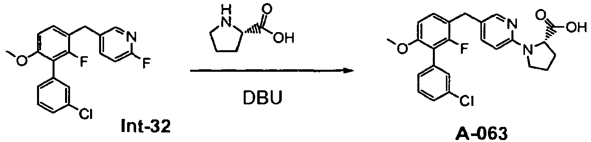

### A-063 [MCLS1486-137-1]. Synthesis of (S)-1-[5-(3'-chloro-2-fluoro-6-methoxy-biphenyl-3-ylmethyl)-pyridin-2-yl]-pyrrolidine-2-carboxylic acid:

To 5-(3'-chloro-2-fluoro-6-methoxy-biphenyl-3-ylmethyl)-2-fluoro-pyridine (**Int-32**) (0.15 g, 0.43 mmol) and (S)-pyrrolidine-2-carboxylic acid (2) (0.15 g, 1.3 mmol) was added 1,8-diazabicyclo[5.4.0]undec-7-cnc (DBU) (0.33 g, 2.17 mmol). The reaction mixture was stirred and heated at 160 °C for 20 min. Cooled to room temperature, diluted with dichloromethane (8 mL), washed with 0.5 N HCl (2X4 mL), dried with Na₂SO₄, filtered, and concentrated. The residue was purified by silica gel column chromatography using 5% methanol in dichloromethane to afford 0.04 g (21%) of **A-063** as an off-white solid. 1H NMR (DMSO-d6, 400MHz): 7.93 (d, *J*= 2.4 Hz, I H), 7.4-7.65 (m, 2 H), 7.34-7.38 (m, 2 H), 7.23-7.3 (m, 2 H), 6.92 (d, *J*= 8.4 Hz, 1 H), 6.4 (d, *J*= 8.4 Hz, 1 H), 4.6-4.68 (m, 1 H), 3.77 (s, 2 H), 3.71 (s, 3 H), 3.26-3.48 (m, 2 H), 2.16-2.25 (m, 1 H), 1.9-2.03 (m, 3 H); MS(APC1+): 441.1 (M+1), LC-MS: 97.8%.

### Synthesis of A-066

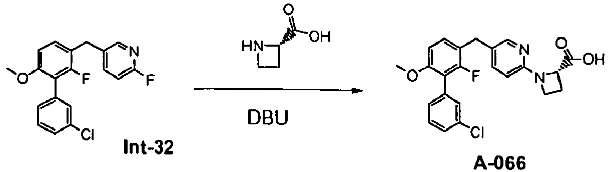

### A-066 [MCLS1486-130-2]. Synthesis of (S)-1-[5-(3'-chloro-2-fluoro-6-methoxy-biphenyl-3-ylmethyl)-pyridin-2-yl]-azetidine-2-carboxylic acid:

To 5-(3'-chloro-2-fluoro-6-methoxy-biphenyl-3-ylmethyl)-2-fluoro-pyridine (**Int-32**) (0.2 g, 0.58 mmol) and L-azetidine-2-carboxylic acid (2) (0.18 g, 1.74 mmol) was added 1,8-diazabicyclo[5.4.0]undec-7-ene (DBU) (0.44 g, 2.89 mmol). The reaction mixture was stirred and heated at 160 °C for 20 min. Cooled to room temperature, diluted with dichloromethane (8 mL), washed with 0.5 N HCl (2X4 mL), dried with Na₂SO₄ filtered, and concentrated. The residue was purified by silica gel column chromatography using 5% methanol in dichloromethane to afford 0.042 g (17%) of **A-066** as an off-white solid.. 1H NMR (DMSO-d6, 400MHz): 7.98 (d, *J*= 2.0 Hz, 1 H), 7.39-7.48 (m, 3 H), 7.37 (s, 1 H), 7.24-7.3 (m, 2 H), 6.92 (d, *J*= 8.4 Hz, 1 H), 6.4 (d, *J*= 8.4 Hz, 1 H), 4.58 (dd, *J*= 9.2, 6.4 Hz, I H), 3.7-3.9 (m, 2 H), 3.8 (s, 2 H), 3.72 (s, 3 H), 2.3-2.55 (m, 2 H); MS(APCI+): 427.1 (M+1), LC-MS: 100%.

### Synthesis of A-067

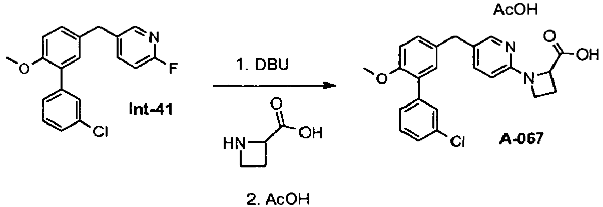

### A-067 [MCLS1486-143-1]. Synthesis of 1-[5-(3'-chloro-6-methoxy-biphenyl-3-ylmethyl)-pyridin-2-yl]-azetidine-2-carboxylic acid acetate salt:

To 5-(3'-chloro-6-methoxy-biphenyl-3-ylmethyl)-2-fluoro-pyridine(**Int-41, prepared in a similar manner as Int-32**) (0.27 g, 0.82 mmol) and azetidine-2-carboxylic acid (0.12 g, 1.24 mmol) was added 1,8-diazabicyclo[5.4.0]undec-7-ene (DBU) (0.63 g, 4.12 mmol). The reaction mixture was stirred and heated at 160 °C for 30 min. Cooled to room temperature, diluted with dichloromethane (8 mL), washed with 0.5 N HCl (2X4 mL), dried with Na₂SO₄ filtered, and concentrated. The residue was purified by silica gel column chromatography using 5% methanol in dichloromethane to afford 0.1 g off-white solid. The solid was dissolved in acetic acid (5 mL), lyophilized to afford 0.102 g (30%) of **A-067** as an off-white solid. 1H NMR (DMSO-d6, 400MHz): 8.02 (d, *J*= 2.0 Hz, 1 H), 7.36-7.5 (m, 5 H), 7.14-7.22 (m, 2 H), 7.04 (d, *J* = 8.4 Hz, 1 H), 6.38 (d, *J* = 8.4 Hz, 1 H), 4.58 (dd, *J*= 8.8, 6.8 Hz, I H), 3.7-3.9 (m, 2 H), 3.79 (s, 2 H), 3.73 (s, 3 H), 2.3-2.55 (m, 2 H), 1.91 (s, 3 H); MS(APCI+): 409.1 (M+1), LC-MS: 100%.

### Synthesis of A-068

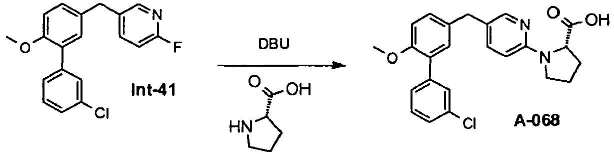

### A-068 [MCLS1486-147-2]. Synthesis of (S)-1-[5-(3'-chloro-6-methoxy-biphenyl-3-ylmethyl)-pyridin-2-yl]-pyrrolidine-2-carboxylic acid:

To 5-(3'-chloro-6-methoxy-biphenyl-3-ylmethyl)-2-fluoro-pyridine (**Int-41**) (0.15 g, 0.46 mmol) and (S)-pyrrolidine-2-carboxylic acid (0.11 g, 0.92 mmol) was added 1,8-diazabicyclo[5.4.0]undec-7-ene (DBU) (0.35 g, 2.29 mmol). The reaction mixture was stirred and heated at 160°C for 20 min. Cooled to room temperature, diluted with dichloromethane (8 mL), washed with 0.5 N HCl (2X4 mL), dried with Na₂SO₄, filtered, and concentrated to afford 0.19 g (98%) of **A-068** as light yellow solid. 1 H NMR (DMSO-d6, 400MHz): 7.96 (d, *J* = 1.2 Hz, 1 H), 7.78 (br s, 1 H), 7.36-7.54 (m, 4 H), 7.24-7.27 (m, 2 H), 7.06 (d, *J* = 8.8 Hz, 1 H), 6.84 (br s, 1 H), 4.65-4.7 (m, 1 H), 3.86 (s, 2 H), 3.76 (s, 3 H), 3.45-3.65 (m, 2 H), 1.85-2.3 (m, 4 H); MS(APCI+): 423.2 (M+1), LC-MS: 95.8%.

### Synthesis of A-069

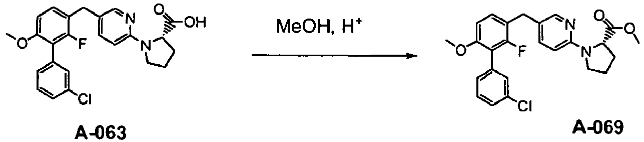

### A-069 [MCLS1486-144-1]. Synthesis of (S)-1-[5-(3'-chloro-2-fluoro-6-methoxy-biphenyl-3-ylmethyl)-pyridin-2-yl]-pyrrolidine-2-carboxylic acid methyl ester:

To **A-063** (0.1 g, 0.22 mmol) in methanol (2.5 mL) was added con. sulfuric acid (0.1 mL). The reaction mixture was stirred and heated at 60 °C for 2 h. Cooled to room temperature, concentrated. Water (5 mL) was added and the product was extracted with dichloromethane (2 x 6 mL), washed with brine (4 mL), dried with Na₂SO₄, filtered, and concentrated to afford 0.088 g (90%) of **A-069** as white solid. 1H NMR (DMSO-d6, 400MHz): 7.91 (d, *J* = 2.0 Hz, 1 H), 7.75 (br s, 1 H), 7.41-7.48 (m, 2 H), 7.26-7.38 (m, 3 H), 6.95 (d, *J*= 8.8 Hz, 1 H), 6.9 (br s, I H), 4.7-4.8 (m, 1 H), 3.89 (s, 2 H), 3.73 (s, 3 H), 3.66 (s, 3 H), 3.45-3.65 (m, 2 H), 1.85-2.35 (m, 4 H); MS(APCI+): 455.1 (M+1), LC-MS: 94.1%.

### Synthesis of A-072

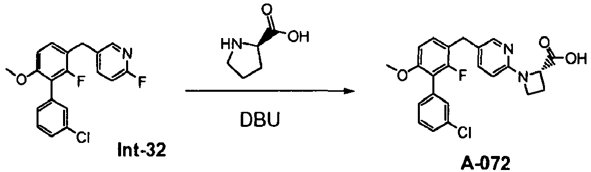

### A-072 [MCLS1486-184-1]. Synthesis of (R)-1-[5-(3'-chloro-2-fluoro-6-methoxy-biphenyl-3-ylmethyl)-pyridin-2-yl]-pyrrolidine-2-carboxylic acid:

To 5-(3'-chloro-2-fluoro-6-methoxy-biphenyl-3-ylmethyl)-2-fluoro-pyridine (**Int-32**) (0.15 g, 0.43 mmol) and (R)-pyrrolidine-2-carboxylic acid (2) (0.1 g, 0.87 mmol) was added 1,8-diazabicyclo[5.4.0]undec-7-ene (DBU) (0.33 g, 2.17 mmol). The reaction mixture was stirred and heated at 160 °C for 20 min. Cooled to room temperature, diluted with dichloromethane (8 mL), washed with 0.5 N HCl (2X4 mL), dried with Na₂SO₄, filtered, and concentrated. The residue was purified by silica gel column chromatography using 5% methanol in dichloromethane to afford 0.14 g (73%) of **A-072** as white solid.
1H NMR (DMSO-d6, 400MHz): 7.93 (d, *J* = 2.4 Hz, 1 H), 7.34-7.46 (m, 4 H), 7.26-7.3 (m, 2 H), 6.91 (d, *J* = 8.4 Hz, 1 H), 6.4 (d, *J* = 8.8 Hz, I H), 4.3 5 (dd, *J* = 9.2, 2.8 Hz, 1 H), 3.77 (s, 2 H), 3.72 (s, 3 H), 3.3-3.48 (m, 2 H), 2.15-2.25 (m, 1 H), 1.9-2.06 (m, 3 H); MS(APCI+): 441.1 (M+1), LC-MS: 98.0%.

### Synthesis of A-074

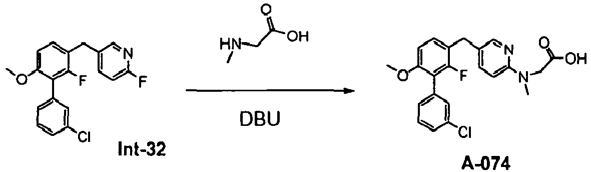

### A-074 [MCLS1621-033-2]. Synthesis of {[5-(3'-chloro-2-fluoro-6-methoxy-biphenyl-3-ylmethyl)-pyridin-2-yl]-methyl-amino}-acetic acid:

To 5-(3'-chloro-2-fluoro-6-methoxy-biphenyl-3-ylmethyl)-2-fluoro-pyridine (**Int-32**) (0.15 g, 0.43 mmol) and sarcosine (2) (0.08 g, 0.87 mmol) was added 1,8-diazabicyclo[5.4.0]undec-7-ene (DBU) (0.33 g, 2.17 mmol). The reaction mixture was stirred and heated at 160°C for 35 min. Cooled to room temperature, diluted with dichloromethane (8 mL), washed with 0.5 N HCl (2X4 mL), dried with Na₂SO₄, filtered, and concentrated. The residue was purified by silica gel column chromatography using 5-10% methanol in dichloromethane to afford 0.09 g (50%) of **A-074** as an off-white solid. 1H NMR (DMSO-d6, 400MHz): 7.94 (d, J= 2.4 Hz, I H), 7.24-7.48 (m, 6 H), 6.91 (d, *J* = 8.8 Hz, 1 H), 6.56 (d, *J* = 8.4 Hz, 1 H), 4.16 (s, 2 H), 3.77 (s, 2 H), 3.71 (s, 3 H), 2.99 (s, 3 H); MS(APCI+): 416.6 (M+1), LC-MS: 100%.

### Synthesis of A-077

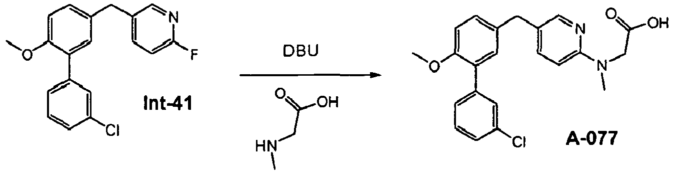

### A-077 [MCLS1621-059-1]. Synthesis of {[5-(3'-chloro-6-methoxy-biphenyl-3-ylmethyl)-pyridin-2-yl]-methyl-amino}-acetic acid:

To 5-(3'-chloro-6-methoxy-biphenyl-3-ylmethyl)-2-fluoro-pyridine (**Int-41**) (0.12 g, 0.37 mmol) and sarcosine (2) (0.07 g, 0.73 mmol) was added 1,8-diazabicyclo[5.4.0]undec-7-ene (DBU) (0.283 g, 1.8 mmol). The reaction mixture was stirred and heated at 150 °C for 40 min. Cooled to room temperature, diluted with dichloromethane (8 mL), washed with 0.5 N HCl (2X4 mL), dried with Na₂SO₄, filtered, and concentrated. The residue was purified by silica gel column chromatography using 5-10% methanol in dichloromethane to afford 0.11 g (74%) of **A-077** as light green solid. I H NMR (DMSO-d6, 400MHz): 7.9 (d, *J*= 2.0 Hz, 1 H), 7.36-7.5 (m, 5 H), 7.18-7.22 (m 2 H), 7.03 (d, *J* = 8.4 Hz, 1 H), 6.56 (d, *J* = 8.8 Hz, I H), 4.2 (s, 2 H), 3.77 (s, 2 H), 3.73 (s, 3 H), 2.99 (s, 3 H); MS(APCI+): 397.1 (M+1), LC-MS: 100%.

### Synthesis of A-070

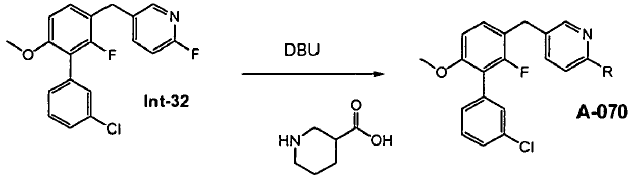

### A-070 [MCLS 1546-013-2]. Synthesis of 5'-(3'-Chloro-2-fluoro-6-methoxy-biphenyl-3-ylmethyl)-3,4,5,6-tetrahydro-2H-[1,2']bipyridinyl-3-carboxylic acid:

In an 8 mL vial equipped with a stir bar was placed **Int-32** (200 mg, 0.578 mmol), piperidine-3-carboxylic acid (224 mg, 1.73 mmol) and 1,8-diazabicyclo[5.4.0]undec-7-ene (407 uL, 2.89 mmol). The mixture was heated to 160 °C for I hour and then cooled to room temperature. The reaction mixture was treated with water (4 mL) and 1M HCl (6 mL). The aqueous portion was extracted with dichloromethane (2 x 30 mL), the organic portions were combined, washed with brine (15 mL), dried (MgSO₄) and concentrated. The crude material was purified by silica gel column chromatography utilizing 10% isopropanol/DCM as the eluent to provide 100 mg of **A-070** as a pale yellow viscous oil in 38% yield. ¹H NMR (400 MHz, DMSO-d₆) δ 1.04 (d, *J* = 6 Hz, 2 H), 1.58-1.67 (m, 1 H), 1.91-1.95 (m, 1 H), 2.35-2.42 (m, 1 H), 2.87-2.98 (m, 2 H), 3.72 (s, 3 H), 3.78 (s, 2 H), 3.95 (bd, *J=* 12 Hz, 1 H), 4.26 (dd, *J=* 13, 4 Hz, 1 H), 6.78 (d, *J*= 9 Hz, 1 H), 6.92 (d, *J*= 8 Hz, 1 H), 7.26-7.30 (m, 2 H), 7.35 (d, *J*= 3 Hz, I H), 7.37 (s, 1 H), 7.41-7.47 (m, 2 H), 8.00 (d, *J*= 2 Hz, 1 H), 12.27 (bs, 1 H). MS(APCI+): 455.2 (M+1), LC-MS: 97%

### Synthesis of A-075

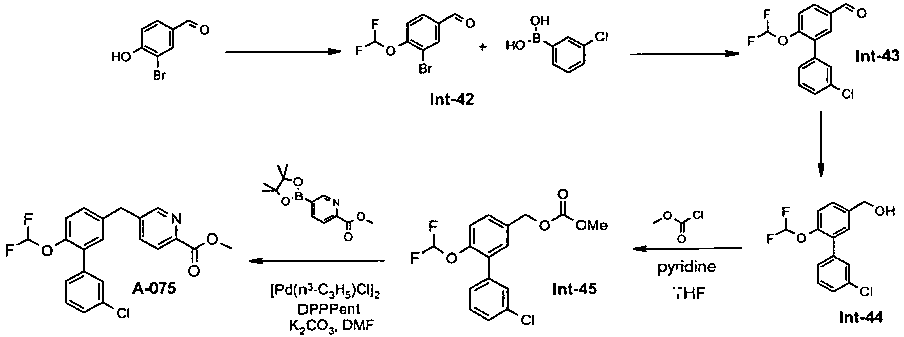

### (Int-42) [MCLS1544-032-4]

### 3-Bromo-4-difluoromethoxy-benzaldehyde

Into a 5L round bottom flask equipped with a mechanical stirrer and a heating mantle was added 3-bromo-4-hydroxy benzaldehyde (100.5 g, 0.50 mol), Cs₂CO₃ (244.4 g, 0.75 mol), Sodium chlorodifluoroacetate (190.6 g, 1.25 mol), and DMF (1L). The reaction was stirred at 65 °C for 1 hour and then cooled to ∼ 35 °C. The reaction mixture was added by a separatory funnel over 10 minutes to 12L of ice water. The mixture was stirred for I hour and then the solids were filtered through a glass frit. The filter cake was washed with 2 X 500 mL water and then dried in a 40 °C vacuum for 18 hours followed by a vacuum dessicator for 5 days. The title product (65.7 g, 52%) was obtained as a tan solid.
¹H NMR (400MHz ,DMSO-d₆) δ = 9.96 (s, 1 H), 8.26 (d, *J*= 1.7 Hz, 1 H), 8.00 (dd, *J*= 1.9, 8.5 Hz, 1 H), 7.54 (d, *J*= 8.5 Hz, 1 H), 7.49 (t, *J*= 72.6 Hz, 1 H)

### (Int-43) [MCLS1544-058-1]

### 3'-Chloro-6-difluoromethoxy-biphenyl-3-carbaldehyde

Into a 5L 3-neck round bottom flask was added **Int-42** (58.4 g, 232.6 mmol), 3-chlorophenylboronic acid (40.0 g, 255.9 mmol), DMF (1.17 L), and 2M aqueous Cs₂CO₃ (233 mL, 464.2 mmol). The suspension was degassed with bubbling N₂ for 30 minutes and then Pd(OAc)₂ (5.3 g, 23.3 mmol) was added. The mixture was slowly warmed to 50 °C over 1 hour, cooled to room temperature, and then filtered through Celite, washing with DMF. 2L of water was added to the filtrate and the product was extracted with 4 X 750 mL EtOAc. The combined organics were washed with 1 L brine, dried over Na₂SO₄, and then concentrated. The residue was passed through a 200g SiO₂ plug eluting with 15% Acetone/Hexane and then concentrated to afford the title compound as a red oil which was used as is.

### (Int-44) [MCLS1544-063-1 and MCLS1544-063-2]

### (3'-Chloro-6-difluoromethoxy-biphenyl-3-yl)-methanol

Into a 3L round bottom flask equipped with magnetic stirring was added **Int-43** (78.1 g, 276.5 mmol), THF (500 mL) and water (500 mL). The cloudy solution was cooled to 0 °C and then NaBH₄ (15.7 g, 414.8 mmol) was added in 4 portions over 15 minutes. After stirring for an additional 15 minutes, the layers were separated. The aqueous layer was extracted with 2 X 500 mL EtOAc. The organics were combined, washed with brine, and concentrated. The residue was purified by flash column chromatography eluting with 5-20% Acetone/Hexane to provide the title compound (43.3 g, 65% 2 steps) as a near colorless oil ¹H NMR (400MHz ,DMSO-d₆) δ = 7.56 - 7.38 (m, 6 H), 7.27 (d, *J* = 9.0 Hz, I H), 7.14 (t, *J =* 73.9 Hz, I H), 5.28 (t, *J =* 5.8 Hz, 1 H), 4.54 (d, *J*= 5.6 Hz, 2 H)

### (Int-45) [MCLS1546-097-1] Synthesis of carbonic acid 3'-chloro-6-difluoromethoxy-biphenyl-3-ylmethyl ester methyl ester-

The same procedure as described for **Int-21** was followed. Isolated 1.55 g of **Int-45** as a white solid in 75% yield.

### A-075 [MCLS1546-083-1]. Synthesis of 5-(3'-Chloro-6-difluoromethoxy-biphenyl-3-ylmethyl)-pyridine-2-carboxylic acid methyl ester:

In a 40 mL vial equipped with a stir bar was placed **Int-45** (700 mg, 2.04 mmol), 5-(4,4,5,5-tetramethyl-[1,3,2]dioxaborolan-2-yl)-pyridine-2-carboxylicacid methyl ester (590 mg, 2.24 mmol), potassium carbonate (846 mg, 6.12 mmol), 1,5-bis(diphenylphosphino)pentane (270 mg, 0.612 mmol) and DMF (10 mL). The reaction mixture was degassed for 15 minutes by bubbling nitrogen and then allylpalladium(II) chloride dimer (112 mg, 0.306 mmol) was added. The reaction mixture was heated to 85 °C for 4 hours. To the reaction mixture was added water (40 mL) and ethyl acetate (40 mL) and the mixture was filtered through Celite. The layers of the filtrate were separated and the aqueous portion was extracted with ethyl acetate (40 mL). The organic portions were combined, washed with brine (40 mL), dried (MgSO₄) and concentrated. The crude material was purified by a 50 gram silica gel SNAP cartridge (Biotage SP4 Flash Chromatography instrument) utilizing gradient elution of 12-100% ethyl acetate/hexanes to produce 458 mg **A-075** as a viscous yellow solid in 56% yield.
¹H NMR (400 MHz, DMSO-d₆) δ 3.86 (s, 3 H), 4.12 (s, 2 H), 7.13 (t, *J =* 74 Hz, 1 H), 7.25 (d, *J =* 8 Hz, 1 H), 7.37 (dd, *J =* 8, 2 Hz, 1 H), 7.57 (dt, *J* = 7, 2 Hz, 1 H), 7.44-7.52 (m, 4 H), 7.88 (dd, *J* = 8, 2 Hz, 1 H), 7.99 (d, *J* = 8 Hz, 1 H), 8.71 (s, 1 H). MS(APCI+): 404.5 (M+1) LC/MS: 98%

### Synthesis of A-076

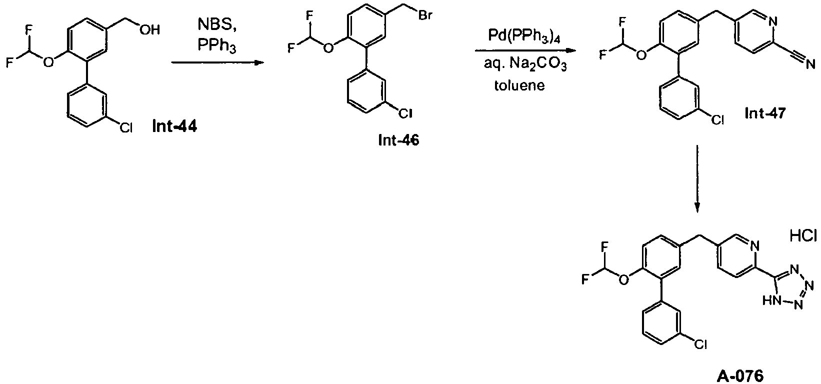

### (Int-46) [MCLS2544-064-1]

### 5-Bromomethyl-3'-chloro-2-difluoromethoxy-biphenyl

Into a 2L round bottom flask equipped with magnetic stirring were added **Int-44** (39.6 g, 139.1 mmol), DCM (500 mL), and the solution was cooled to 0 °C. PPh₃ (73.0 g, 278.2 mmol) and NBS (49.5 g, 278.2 mmol) were added and the solution was stirred for 30 minutes. The solution was washed with 2 X 500 mL water, 500 mL brine, dried over Na₂SO₄, and concentrated. Purification by flash column chromatography (15% Acetone/Hexane) provided the title compound (43.5 g, 90%) as colorless oil. ¹H NMR (400MHz ,DMSO-d₆) δ = 7.62 - 7.38 (m, 5 H), 7.37 (s, 1 H), 7.31 (d, *J*= 8.3 Hz, 1 H), 7.21 (t, *J*= 73.7 Hz, 1 H), 4.77 (s, 2 H)

### (Int-47) [MCLS 1546-052-2] Synthesis of 5-(3'-Chloro-6-difluoromethoxy-biphenyl-3-ylmethyl)-pyridine-2-carbonitrile:

In a 40 mL vial equipped with a stir bar was placed **Int-46** (1.0 g, 2.88 mmol), 5-(4,4,5,5-tetramethyl-[1,3,2]dioxaborolan-2-yl)-pyridine-2-carbonitrile (729 mg, 3.17 mmol), tetrakis(triphenylphosphine)palladium (166 mg, 0.144 mmol), ethanol (4.4 mL) and toluene (17.6 mL). After stirring for 5 minutes, a 2M aqueous solution of sodium carbonate (2.88 mL) was added and the reaction mixture was degassed by bubbling nitrogen through the mixture. After heating at 80 °C for 2.5 hours, the reaction was filtered through Celite and the filtrate was diluted with water (40 mL) and extracted with ethyl acetate (2 x 60 mL). The organic portions were combined, dried (MgSO₄), concentrated and purified by silica gel column chromatography utilizing 40% ethyl acetate/hexanes as the eluent to produce 859 mg of **Int-47** as a yellow viscous oil in 80% yield.

### A-076 [MCLS1546-084-2]. Synthesis of 5-(3'-Chloro-6-difluoromethoxy-biphenyl-3-ylmethyl)-2-(1 H-tetrazol-5-yl)-pyridine hydrochloride salt:

In an 8 mL vial equipped with a stir bar was placed **Int-47** (150 mg, 0405 mmol), sodium azide (29.0 mg, 0.466 mmol), ammonium chloride (23.9 mg, 0.466 mmol) and dimethylformamide (700 uL). The solution was heated to 100°C for 5 hours and then the inorganic salts were filtered off. After concentrating the reaction mixture, water (3 mL) and 1M HCl (3 mL) were added. The semi-solid was collected and dried to produce 167 mg of **A-076 (free base)** as an off-white solid in quantitative yield. To **A-076 (free base)** (140 mg, 0.341 mmol) was added diethyl ether (3 mL) and 2M HCl in diethyl ether (800 uL). The mixture was allowed to stir at room temperature for 10 minutes, concentrated and dried to produce 131 mg of A-076 as a white solid in 86% yield. ¹H NMR (400 MHz, DMSO-d₆) δ 4.15 (s, 2 H), 7.13 (t, *J =* 74 Hz, 1 H), 7.27 (d, *J* = 8 Hz, 1 H), 7.40-7.53 (m, 6 H), 7.98 (dd, *J* = 8, 2 Hz, 1 H), 8.16 (d, *J* = 8 Hz, 1 H), 8.81 (D, *J* = 2 Hz, 1 H).

### Synthesis of A-078 and A-079

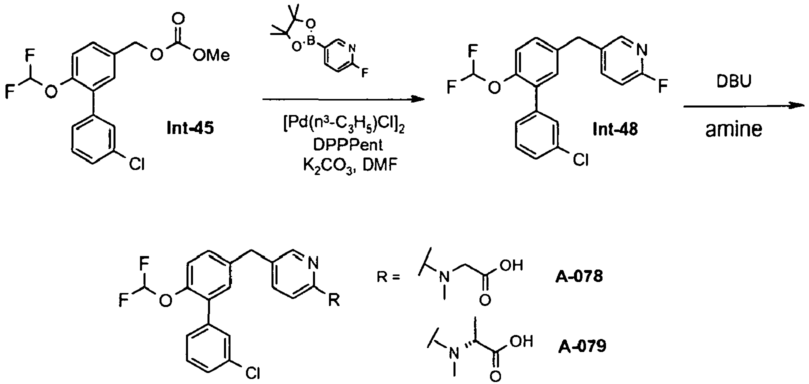

### (Int-48) [MCLS 1546-091-1] Synthesis of 5-(3 '-Chloro-6-difluoromethoxy-biphenyl-3-ylmethyl)-2-fluoro-pyridine:

Prepared in a similar manner as **A-075**. Isolated 372 mg of **Int-48** as a viscous oil with slight dark yellow tinge in 70% yield.

### A-078 [MCLS 1546-096-1]. Synthesis of {[5-(3'-Chloro-6-difluoromethoxy-biphenyl-3-ylmethyl)-pyridin-2-yl]-methyl-amino}-acetic acid:

In an 8 mL vial equipped with a stir bar was placed **Int-48** (190 mg, 0.522 mmol), N-methylglycine (140 mg, 1.57 mmol) and 1,8-diazabicyclo[5.4.0]undec-7-ene (368 uL, 2.61 mmol). The mixture was heated to 160°C for 35 minutes and then diluted with DCM (15 mL). The organic portion was washed with 0.5M HCl (3 x 10 mL) and the aqueous washes were combined and extracted with DCM (15 mL). The organic portions were combined, dried (MgSO₄ and concentrated. The crude material was purified by silica gel column chromatography utilizing 10% isopropanol/DCM (with 1% AcOH) as the eluent to produce 17 mg of **A-078** as a light green solid in 8% yield. ¹H NMR (400 MHz, DMSO-*d*₆) δ 2.99 (s, 3 H), 3.83 (s, 2 H), 4.20 (s, 2 H), 6.58 (d, *J* = 8 Hz, 1 H), 7.10 (t, *J* = 74 Hz, 1 H), 7.21 (d, *J* = 8 Hz, 1 H), 7.30 (dd, *J* = 9, 2 Hz, 1 H), 7.37 (d, *J =* 2 Hz, 1 H), 7.40-7.51 (m, 5 H), 8.01 (d, *J* = 2 Hz, 1 H), 12.3 7 (s, 1 H). MS(ESI+): 435.0 (M+2), LC/MS: 97%

### A-079 [MCLS1546-101-1]. Synthesis of (R)-2-{[5-(3'-Chloro-6-difluoromethoxy-biphenyl-3-ylmethyl)-pyridin-2-yl]-methyl-amino}-propionic acid:

In an 8 mL vial equipped with a stir bar was placed **Int-48** (173 mg, 0.476 mmol), N-α-methyl-D-alanine HCl (199 mg, 1.43 mmol) and 1,8-diazabicyclo[5.4.0]undec-7-ene (403 uL, 2.86 mmol). The mixture was heated to 160 °C for 35 minutes and then diluted with DCM (15 mL). The organic portion was washed with 0.5M HCl (3x10 mL) and the aqueous washes were combined and extracted with DCM (15 mL). The organic portions were combined, dried (MgSO₄) and concentrated. The crude material was purified by silica gel column chromatography utilizing 10% isopropanol/DCM (with 1% AcOH) as the eluent to produce 56 mg of a yellow solid which still contained impurities. The impure material was purified by preparative TLC (20x20 cm, 1500 microns) using 10% isopropanol/DCM as the eluent to produce 18 mg of A-079 as a yellow solid in 4% yield.
¹H NMR (400 MHz, DMSO-d₆) δ 1.35 (t, *J* = 8 Hz, 3 H), 2.84 (s, 3 H), 3.84 (s, 2 H), 3.94 (q, *J* = 7 Hz, 1 H), 6.59 (d, *J* = 9 Hz, 1 H), 7.10 (t, *J* = 74 Hz, 1 H), 7.21 (d*, J* = 8 Hz, 1 H), 7.29-7.32 (m, 2 H), 7.37 (d, *J* = 2 Hz, 1 H), 7.40-7.51 (m, 4 H), 8.01 (d, *J* = 2 Hz, 1 H), 12.37 (bs, 1 H). MS(APCI+): 447.1 (M+1)

### Synthesis of A-073

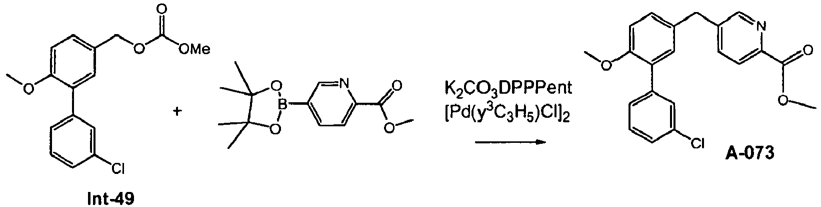

### A-073 MCLS1475-133-2] Synthesis of 5-(3'-chloro-6-methoxy-biphenyl-3-ylmethyl)-pyridine-2-carboxylic methyl ester.

To a 250 mL flask which contained the mixture of carbonic acid 3'-chloro-6-methoxy-biphenyl-3-ylmethyl ester methyl ester (**Int-49, Prepared in a similar manner as Int-021**, 2.0 g, 6.6 mmol) and 5-(4,4,5,5-tetramethyl-[1,3,2]dioxaborolan-2-yl)-pyridine-2-carboxylic acid methyl ester (1.6 g, 6 mmol) in DME (30 mL) was added K2CO3 (2.5 g, 18 mmol), [Pd(y³C₃H₅Cl)]₂ (300 mg, 0.8 mmol) and DPPPent (800 mg, 1.9 mmol) at rt under nitrogen. The reaction mixture was heated to 85 °C and stirred at 85 °C for 16 h. The reaction mixture was cooled to rt, poured onto ice-water (200 mL), The semi-solid which formed was separated from the aq. layer to provide the crude which was purified by a chromatography on silica gel with dichloromethane-acetone as eluent to yield the title compound (1200 mg, 50%) 1H NMR (CHLOROFORM-d ,400MHz): δ = 8.64 (s, 1 H), 8.05 (d, J=8.0 Hz, I H), 7.62 (d, J=8.0 Hz, 1 H), 7.47 (s, 1 H), 7.29 - 7.36 (m, 2 H), 7.11 (d, J=8.0 Hz, 1 H), 7.09 (d, J=2.0 Hz, 1 H), 6.93 (d, J=8.4 Hz, 1 H), 4.04 (s, 2 H), 3.99 (s, 3 H), 3.80 (s, 3 H). LC/MS: Calc.367.84; APCI+(M+1): 368.1, 99%

### Synthesis of A-058

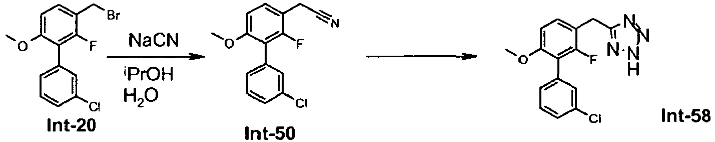

### Int-50 [MCLS1426-077-1] Synthesis of (3'-Chloro-2-fluoro-6-methoxy-biphenyl-3-yl)-acetonitrile:

A suspension of **Int-20** (15.2 mmol; 1.0 eq.) and sodium cyanide (22.8 mmol; 1.5 eq.) in aqueous isopropyl alcohol was refluxed for 1 hour. The reaction was cooled to room temperature, and concentrated to remove the isopropyl alcohol. The resultant aqueous layer was extracted with 2 portions of ethyl acetate, and the organics washed successively with water and brine, and dried over magnesium sulfate. The residue was purified via flash chromatography on silica gel, using 10% acetone/hexanes as eluent to afford the title compound as a white solid in 57% yield.
(¹HNMR, CDCl₃; 400 MHz): 3.80 (s, 3H), 3.93 (s, 2H), 6.80 (dd, J = 8.8, 1.6 Hz, 1H), 7.26 (m, 1H), 7.35-7.41 (M, 4H)

### A-058 MCLS1471-166-1

### 5-(3'-Chloro-2-fluoro-6-methoxy-biphenyl-3-ylmethyl)-1 H-tetrazole

Into an 18 mL vial was added **Int-50** (138 mg, 0.50 mmol), NH₄Cl (187 mg, 3.50 mmol), NaN₃ (65 mg, 1.00 mmol), and DMF (4 mL). The reaction was stirred at 80 °C for 4 hours and then 100 °C for 18 hours. The suspension was filtered through a glass frit and a SiO₂ plug and then concentrated. The residue was purified by flash column chromatography eluting with 25% - 50% Acetone/Hexanes. The solid was dried on a 50 °C vacuum oven for 8 hours, the room temperature for 18 hours. The title compound (25 mg, 16%) was obtained as a tan solid. ¹H NMR (400MHz ,DMSO-d₆) δ = 16.19 (br. s., 1 H), 7.50 - 7.40 (m, 2 H), 7.40 - 7.33 (m, 2 H), 7.29 (d, *J* = 6.6 Hz, 1 H), 6.99 (d, *J* = 8.6 Hz, 1 H), 4.27 (s, 2 H), 3.75 (s, 3 H). LC/MS = 98.7%, 317.0 (APCl+).

### Synthesis of A-071

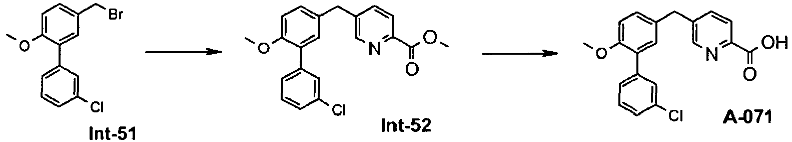

### Int-52 MCLS1544-062-1

### 5-(3'-Chloro-6-methoxy-biphenyl-3-ylmethyl)-pyridine-2-carboxylic acid methyl ester

Into a 250 mL round bottom flask was added 5-Bromomethyl-3'-chloro-2-methoxy-biphenyl (**Int-51, prepared in a similar manner as Int-20**, 4.89 g, 15.69 mmol), 5-(1,5-Dimethyl-2,4-dioxa-3-bora-bicyclo[3.1.0]hex-3-yl)-pyridine-2-carboxylic acid methyl ester (4.52 g, 17.26 mmol), toluene (120 mL), EtOH (20 mL), water (20 mL), and K₃PO₄ (6.66 g, 31.38 mmol). The suspension was degassed with N₂ for 15 minutes and then Pd(PPh₃)₄ (1.81 g, 1.57 mmol) was added and the reaction was stirred at 80°C for I hour. The layers were separated and the aqueous was extracted with 50 mL EtOAc. The organics were combined and washed with 50 mL brine, dried over Na₂SO₄, and concentrated. The residue was purified by flash column chromatography eluting with 10% Acetone/Hexanes to afford the title compound (0.89 g, 15%) as a light-yellow oil.

### A-071 MCLS 1544-068-1

### 5-(3'-Chloro-6-methoxy-biphenyl-3-ylmethyl)-pyridine-2-carboxylic acid

Into an 18 mL vial was added Int-52 (185 mg, 0.50 mmol), EtOH (4 mL), and 1N NaOH (4 mL). After stirring at room temperature for 1 hour the EtOH was evaporated. The pH was adjusted to about 4 with conc. HCl and the resulting solids were filtered and dried in a 50°C vacuum oven for 4 hours. The title compound (99 mg, 56%) as a white solid. ¹H NMR (400MHz ,DMSO-d₆) δ = 8.66 (d, *J* = 1.6 Hz, 1 H), 7.96 (d, *J* = 7.9 Hz, 1 H), 7.82 (dd, *J =* 1.9, 7.9 Hz, 1 H), 7.50 (s, 1 H), 7.46 - 7.34 (m, 3 H), 7.31 - 7.22 (m, 2 H), 7.12 - 7.03 (m, 1 H), 4.05 (s, 2 H), 3.75 (s, 3 H)

### Synthesis of A-059 and A-060

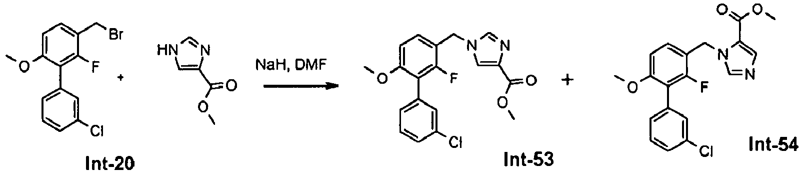

### Int-53 and Int-54

### (Int-53) 1-(3'-Chloro-2-fluoro-6-methoxy-biphenyl-3-ylmethyl)-1 H-imidazole-4-carboxylic acid methyl ester

### (Int-54) 1-(3'-Chloro-2-fluoro-6-methoxy-biphenyl-3-ylmethyl)-1 H-imidazole-2-carboxylic acid methyl ester

Into an 18 mL vial was added Methyl 4-imidazolecarboxylate (113 mg, 0.90 mmol), DMF (3 mL), and NaH (43 mg, 1.08 mmol). After 20 minutes at room temperature **Int-20** (295 mg, 0.90 mmol) was added. The reaction was stirred for 2 hours at room temperature and then water was added. The product was extracted with EtOAc and the organics were concentrated. The residue was purified by flash column chromatography eluting with 6% - 10% Acetone/DCM to separate the regioisomers. The 4-substituted ester (**Int-53**, 67 mg, 20%) and the 2-substituted ester (**Int-54**, 79 mg, 23%) were obtained as colorless oils.

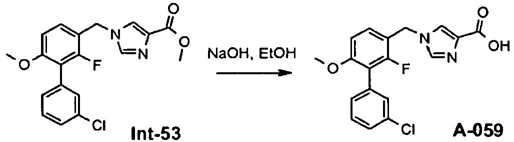

### A-059

### 1-(3'-Chloro-2-fluoro-6-methoxy-biphenyl-3-ylmethyl)-1 H-imidazole-4-carboxylic acid

Into an 8 mL vial was added **Int-53** (31 mg, 0.08 mmol), EtOH (1 mL), and 2 N NaOH (1 mL). After stirring for 2 hours at room temperature the EtOH was evaporated. The pH was adjusted to about 5 with concentrated HCl which produced w white precipitate. The solid was filtered and dried in a 50°C vacuum oven for 2 hours to provide the title compound (6 mg, 20%) as a white solid. ¹H NMR (400MHz ,DMSO-d₆) δ = 7.80 (s, 2 H), 7.50 - 7.36 (m, 4 H), 7.29 (d, *J* = 6.2 Hz, I H), 7.02 (d, *J* = 8.7 Hz, 1 H), 5.26 (s, 2 H), 3.75 (s, 3 H). MS = 361.1 (APCI+).

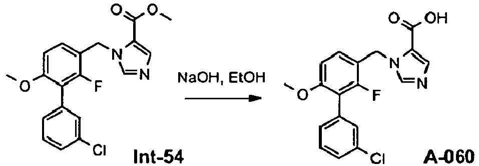

### A-060 MCLS1471-180-1

### 3-(3'-Chloro-2-fluoro-6-methoxy-biphenyl-3-ylmethyl)-3H-imidazole-4-carboxylic acid

Into an 8 mL vial was added **Int-54** (38 mg, 0.10 mmol), EtOH (1 mL), and 2 N NaOH (1 mL). After stirring for 2 hours at room temperature the EtOH was evaporated. The pH was adjusted to about 5 with concentrated HCl which produced w white precipitate. The solid was filtered and dried in a 50°C vacuum oven for 2 hours to provide the title compound (17 mg, 47%) as a white solid. ¹H NMR (400MHz ,DMSO-d₆) δ = 12.87 (br. s., 1 H), 7.97 (s, 1 H), 7.61 (s, 1 H), 7.50 - 7.42 (m, 2 H), 7.39 (s, 1 H), 7.30 (d, *J* = 6.4 Hz, 1 H), 7.08 - 6.99 (m, 1 H), 6.98 - 6.91 (m, 1 H), 5.55 (s, 2 H), 3.73 (s, 3 H). LC/MS = 361.1 (APCI+).

### Synthesis of A-030 and A-031

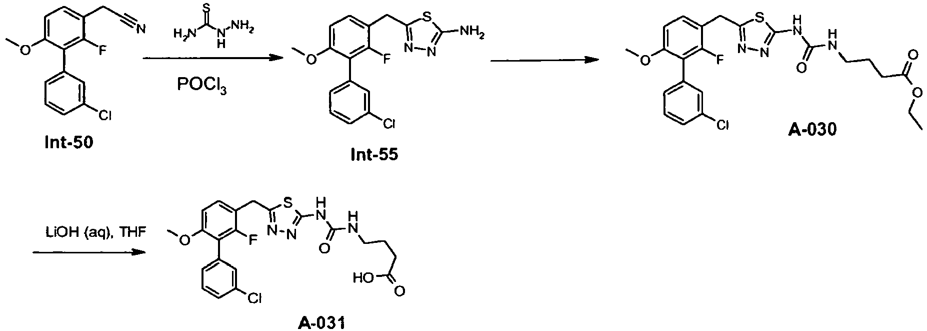

### Int-55 [MCLS1426-108-1] Synthesis of 5-(3'-Chloro-2-fluoro-6-methoxy-biphenyl-3-ylmethyl)-[1,3,4]thiadiazol-2-ylamine:

A mixture of **Int-50** (0.998 mmol, 1.0 eq.) and thiosemicarbazide (2.99 mmol; 3 eq.) in excess phosphorous oxychloride was heated to 120°C for 45 minutes, and allowed to cool to room temperature. The resultant mixture was added to water, and extracted with 2 portions of ethyl acetate. The organics were washed with brine, and dried over magnesium sulfate. The residue was purified via flash chromatography on silica gel using 5% (IN NH₃ in MeOH) in dichloromethane as cluent to afford the title compound in 39% yield.
(¹HNMR, DMSO-d₆; 400 MHz): 3.75 (s, 3H), 4.17 (s, 2H), 6.98 (d, J = 8.0 Hz, 1H), 7.29 (d, J = 6.4 Hz, 1H), 7.37-7.41 (M, 2H), 7.43-7.49 (M, 2H), 7.58-7.76 (bs, 2H)

### A-030 MCLS1430-143-1

### 4-{3-[5-(3'-Chloro-2-fluoro-6-methoxy-biphenyl-3-ylmethyl)- [1,3,4]thiadiazol-2-yl]-ureido}-butyric acid ethyl ester

To a solution of **Int-55** (125 mg, 0.36 mmol) in pyridine (0.5 mL) at room temperature was added 4-Isocyanato-butyric acid ethyl ester (136 uL, 0.89 mmol). The resultant solution was stirred at room temperature for 18 hours and then at 50 °C for 24hours. To the solution was added 10 mL of water and the product was extracted with 3X15 mL EtOAc and the combined organics were dried over Na₂SO₄, and concentrated. 5 mL of ether was added to the yellow oil and the solution was allowed to stand at room temperature for 18 hours. The resulting solid was filtered, washed with ether, and dried to afford the title compound (66 mg, 36%) as a white solid. ¹H NMR (400MHz ,CHLOROFORM-d) δ 10.72 (s, 1H), 7.48 - 7.37 (m, 4H), 7.30 - 7.28 (m, 1H), 6.99 (d, *J* = 8.4 Hz, 1H), 6.91 (bs, 1H), 4.26 (s, 2H), 4.03 (q, *J* = 7.1 Hz, 2H), 3.75 (s, 3H), 3.12 (q, *J* = 6.0 Hz, 2H), 2.28 (t, *J*= 7.6 Hz, 2H), 1.66 (quin, *J* = 7.6 Hz, 2H), 1.15 (t, *J*= 7.0 Hz, 3H). LC/MS = 91.1 %, 507.0 (APCI+).

### A-031 MCLS1430-150-1

### 4-{3-[5-(3'-Chloro-2-fluoro-6-methoxy-biphenyl-3-ylmethyl)-[1,3,4]thiadiazol-2-yl]-ureido}-butyric acid

To a solution of **A-030** (35 mg, 0.069 mmol) in THF (0.5 mL) was added 1M aqueous lithium hydroxide (345 uL, 0.35 mmol). The reaction was stirred at room temperature for 1 hour. The THF was removed with a stream of nitrogen, and the resultant aq. solution was acidified with 6N HCl to pH **1**-2. After stirring for 30 minutes at room temperature, the resultant solids were filtered, washed with water, and dried in a 50°C vacuum oven for 4 hours. The title compound (19.8 mg, 60%) was obtained as a tan solid. ¹H NMR (400MHz ,CHLOROFORM-d) δ 10.78 (bs, 1H), 7.48 - 7.38 (m, 3H), 7.30 - 7.28 (m, 1H), 6.99 (d, *J*= 8.0 Hz, 1H), 6.94 (bs, 1H), 4.26 (s, 2H), 3.75 (s, 3H), 3.12 (q, *J*= 6.7 Hz, 2H), 2.21 (t, *J=* 7.4 Hz, 2H), 1.65 (t, quin = 7.0 Hz, 2H).

### Synthesis of A-002

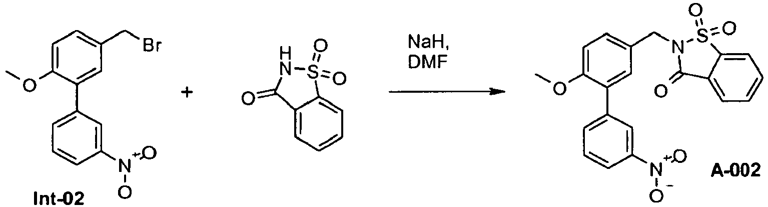

### A-002 [MCLS1226-178-1]. Synthesis of 2-(6-Methoxy-3'-nitro-biphenyl-3-ylmethyl)-1,1-dioxo-1,2-dihydro-1lambda*6*-benzo [d] isothiazol-3-one.

To a solution of **Int-02** (161 mg, 0.500 mmol) and saccharin (183 mg, 1.00 mmol) in dimethyl formamide (1.5 mL) was added sodium hydride (60% weight dispersion, 40 mg, 1.00 mmol) at -78°C. After hydrogen gas evolution ceased the reaction was stirred at 120°C overnight. The reaction mixture was filtered, and the filtrate concentrated under reduced pressure. The residue was diluted with ethyl acetate (15 mL), washed with water, brine, dried over sodium sulfate, filtered, and the solvent removed under vacuum. The product was purified by trituration with hexanes (50 mL) and ethyl acetate (5 mL) to give **A-002** (190 mg, 90% yield). I H NMR (400 MHz, CHLOROFORM-d) δ 3.83 (s, 3 H) 4.92 (s, 2 H) 6.99 (d, J=8.45 Hz, 1 H) 7.46 - 7.61 (m, 3 H) 7.78 - 7.98 (m, 4 H) 8.06 (d, J=7.11 Hz, 1 H) 8.17 (dd, J=8.25, 1.41 Hz, 1 H) 8.42 (t, J=1.74 Hz, 1 H) LCMS = 94% purity.

### Synthesis of A-040

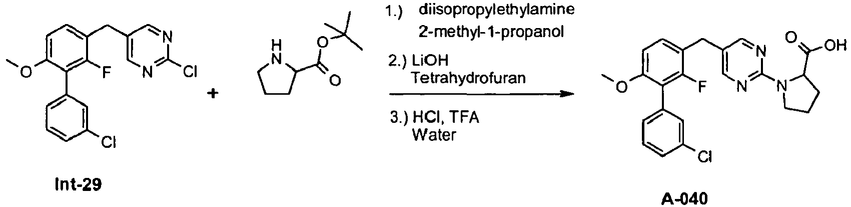

### A-040 [MCLS1448-152-1]. Synthesis of 1-[5-(3'-Chloro-2-fluoro-6-methoxy-biphenyl-3-ylmethyl)-pyrimidin-2-yl]-pyrrolidine-2-carboxylic acid.

A solution of **Int-29** (100 mg, 0.28 mmol), pyrrolidine-2-carboxylic acid tert-butyl ester (72 mg, 0.42 mmol), and diisopropylethylamine (0.15 mL, 0.84 mmol) in 2-methyl-1-propanol (1 mL) was stirred at 80°C for 4 days. The reaction was cooled to room temperature and concentrated under vacuum. To the crude material was added tetrahydrofuran (5 mL) and 1 M aqueous lithium hydroxide (0.56 mL, 0.56 mmol) and the solution heated to 80°C overnight. The reaction was cooled to room temperature, and acidified to pH 3 with 2 N aqueous hydrochloric acid. To the reaction was added trifluoroacetic acid (0.84 mmol) and the reaction heated to 80°C overnight. The reaction was cooled to room temperature and concentrated en vacuo, and the residue was purified by silica gel column chromatography (2:1 hexanes/ethyl acetate) to give **A-040** (49.1 mg, 35% yield) as a white solid. ¹H NMR (400MHz ,CHLOROFORM-d) δ = 8.32 (br. s., 2 H), 7.40 - 7.30 (m, 3 H), 7.14 (t, *J =* 8.6 Hz, 1 H), 6.74 (d, *J =* 8.5 Hz, 1 H),4.59 -4.51 (m, 1 H), 3.83 (s, 2 H), 3.77 (s, 3 H), 3.75 - 3.66 (m, 1 H), 3.65 - 3.55 (m, 1 H), 2.13-2.02 (m, 4 H). LC/MS = 95%, 442.0 (APCI+).

### Synthesis of A-044

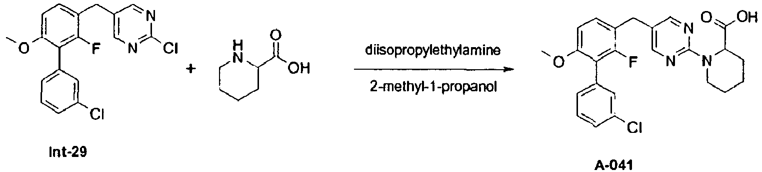

### A-041 [MCLS1448.155-3]. Synthesis of 1-[5-(3'-Chloro-2-fluoro-6-methoxy-bipenyl-3-ylmethyl)-pyrimidin-2-yl]-piperidine-2-carboxylic acid.

A solution of **Int-29** (100 mg, 0.28 mmol), nipecotic acid (54 mg, 0.42 mmol), and diisopropylethylamine (0.15 mL, 0.84 mmol) in 2-methyl-1-propanol (1 mL) was stirred at 80°C overnight. The reaction was cooled to room temperature and concentrated en vacuo. The crude was purified by silica gel column chromatography (20:1 dichloromethane/methanol), triturated in dichloromethane/hexanes to give **A-041** (36.5 mg, 29% yield) as a cream colored solid.
1 H NMR (400 MHz, CHLOROFORM-d) δ ppm 1.56 (br. s., 1 H) 1.71 - 1.87 (m, 2 H) 2.17 (s, 2 H) 2.50 - 2.68 (m, 1 H) 3.05 - 3.15 (m, I H) 3.26 (dd, J=13.28, 10.06 Hz, 1 H) 3.71 - 3.80 (m, 5H) 4.45 (d, J=13.15 Hz, 1 H) 4.69 (dd, J=13.35, 3.56 Hz, 1 H) 6.70 (d, J=8.45 Hz, 1 H) 7.10 (t, J=8.52 Hz, 1 H) 7.29 - 7.41 (m, 3 H) 8.22 (s, 2 H). LCMS = 97%.

### Synthesis of A-044

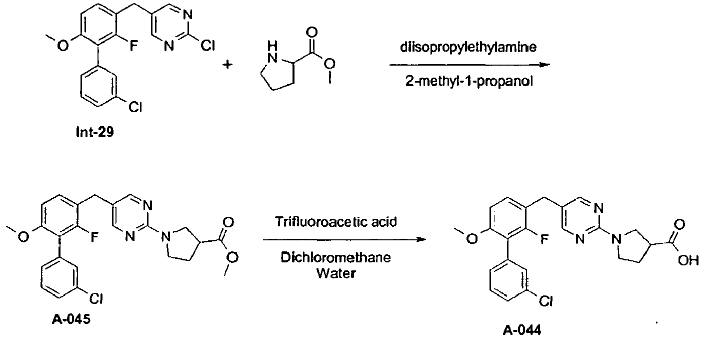

### A-045 [MCLS1448-154-4]. Synthesis of 1-[5-(3'-Chloro-2-fluoro-6-methoxy-biphenyl-3-ylmethyl)-pyrimidin-2-yl]-pyrrolidine-3-carboxylic acid methyl ester.

A mixture of **Int-29** (100 mg, 0.28 mmol), pyrrolidine-2-carboxylic acid methyl ester (70 mg, 0.42 mmol), and diisopropylethylamine (0.15 mL, 0.84 mmol) in 2-methyl-1-propanol (1 mL) was stirred at 80°C overnight. The reaction was cooled, and the solvent removed under vacuum. The crude was purified twice by silica gel column chromatography (4:1 hexanes/ethyl acetate, 2:1 hexanes/ ethyl acetate) to give **A-045** (44.7 mg, 35% yield ¹H NMR (400MHz, CHLOROFORM-d) δ = 8.21 (s, 2 H), 7.40-7.29 (m, 3 H), 7.08 (t, *J* = 8.6 Hz, 1 H), 6.69 (d, *J* = 8.5 Hz, 1 H), 3.88 - 3.74 (m, 3H), 3.76 (s, 2 H), 3.75 (s, 3 H), 3.72 (s, 3 H), 3.63 - 3.53 (m, 1 H), 3.19 (quin, *J* = 7.4 Hz, 1 H), 2.33 - 2.22 (m, 2 H). LC/MS = 94.7%, 456.1 (APCI+).

### A-044 [MCLS1448-162-1]. Synthesis of 1-[5-(3'-Chloro-2-fluoro-6-methoxy-biphenyl-3-ylmethyl)-pyrimidin-2-yl]-pyrrolidine-3-carboxylic acid.

A mixture of **Int-29** (35 mg, 0.075 mmol) and trifluoroacetic acid (85 mg, 0.75 mmol) in water (0.5 mL) and dichloromethane (2 mL) was stirred at room temperature overnight. The reaction was heated to 80°C for 3 days. The reaction was diluted with dichloromethane (2 mL) and water (1 mL), separated, and the aqueous portion extracted with dichloromethane (2 x 2 mL). The combined organic extracts were dried over sodium sulfate, filtered, and concentrated. The residue was purified by silica gel column chromatography (40:1 dichloromethane/methanol) to give **A-044** (30.7 mg, 93% yield). ¹H NMR (400MHz ,DMSO-d₆) δ = 8.26 (s, 2 H), 7.48 - 7.40 (m, 2 H), 7.37 (s, 1 H), 7.33 - 7.25 (m, 2 H), 6.93 (d, *J* = 8.6 Hz, 1 H), 3.77 (s, 2 H), 3.72 (s, 3 H), 3.67 - 3.60 (m, 2 H), 3.55 - 3.43 (m, 2 H), 3.15 (quin, *J* = 7.0 Hz, 1 H), 2.23 - 2.04 (m, 2 H). LC/MS = 93.6%, 442.0 (APCI+).

### Synthesis of A-053

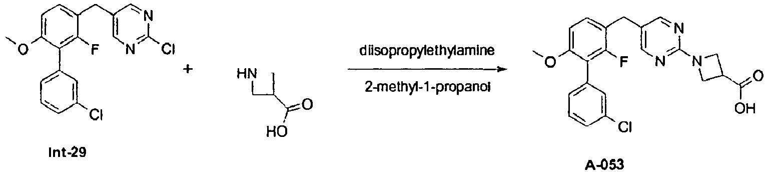

### A-053 [MCLS1448-159-4]. Synthesis of 1-15-(3'-Chloro-2-fluoro-6-methoxy-biphenyl-3-ylmethyl)-pyrimidin-2-yl]-azetidine-3-carboxylic acid.

A mixture of **Int-29** (100 mg, 0.28 mmol), azetidine-3-carboxylic acid (42 mg, 0.42 mmol), and diisopropylethylamine (0.15 mL, 0.84 mmol) in 2-methyl-1-propanol (1mL) was stirred at 80°C overnight. The reaction was cooled, and the solvent removed under vacuum. The crude was purified twice by silica gel column chromatography (18: hexanes/ethyl acetate), triturated with diethyl ether/dichloromethane, and purified by silica gel preparatory thin layer chromatography (9:1 dichloromethane/ methanol) to give **A-053** (11.5 mg, 9.6% yield).
1H NMR (400 MHz, DMSO-d6) δ 2.03 - 2.24 (m, 2 H) 3.15 (t, J=6.98 Hz, 1H) 3.25 (s, 1 H) 3.44 - 3.55 (m, 1 H) 3.64 (t, J=6.91 Hz, 1 H) 3.72 (s, 2 H) 3.77 (s, 2 H) 6.93 (d, J=8.59 Hz, 1 H) 7.25 - 7.34 (m, 2 H) 7.37 (s, 1 H) 7.40 - 7.47 (m, 2 H) 8.19 - 8.28 (m, 2 H)

### Synthesis of A-049

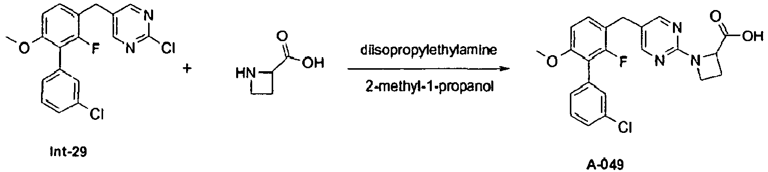

### A-049 [MCLS1448-180-1]. Synthesis of 1-[5-(3'-Chloro-2-fluoro-6-methoxy-biphenyl-3-ylmethyl)-pyrimidin-2-yl]-azetidine-2-carboxylic acid.

A mixture of azetidine-2-carboxylic acid (56 mg, 0.56 mmol) and sodium hydride (60% weight dispersion, 33 mg, 0.84 mmol) in dimethylformamide (1 mL) was stirred under gas evolution ceased. After 2 min of stirring **Int-29** (102 mg, 0.28 mmol) was added, and the reaction heated at 120 °C overnight. The reaction was cooled to room temperature, diluted with water (15 mL), and extracted with ethyl acetate (3 x 5 mL). The combined extracts were dried over sodium sulfate, filtered, and the solvent removed under vacuum. The residue was purified by silica gel column chromatography (9:1 dichloromethane/methanol) to give **A-049** (65.3 mg, 58% yield) as a cream solid.
1H NMR (400 MHz, DMSO-d6) δ 2.17 - 2.28 (m, 1 H) 2.55 - 2.67 (m, 1 H) 3.72 (s, 3 H) 3.78 (s, 2 H) 3.87- 4.05 (m, 2 H) 4.63 (dd, J=9.12, 5.77 Hz, 1 H) 6.93 (d, J=8.59 Hz, I H) 7.25 - 7.36 (m, 2 H) 7.38 (s, 1 H) 7.41-7.47 (m, 2 H) 8.25 (s, 2 H) 12.64 (br. s., 1 H). LCMS = 95.0%

### Preparation of B-080

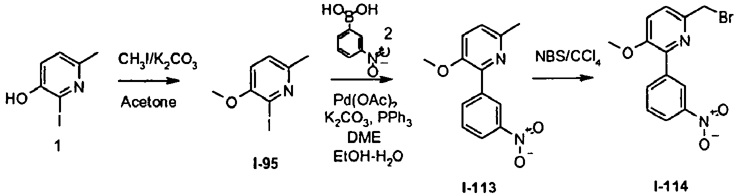

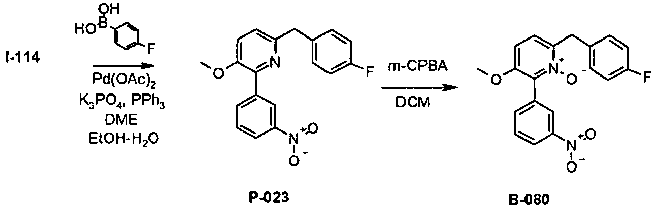

**Synthesis of 2-iodo-3-methoxy-6-methyl-pyridine (I-95).** To 2-iodo-6-methyl-pyridin-3-ol (1.0 g, 4.25 mmol) and K₂CO₃ (1.18 g, 8.51 mmol) in acetone (20 mL) was added Mel (0.91 g, 6.38 mmol). The reaction was stirred at 45 °C under N₂ for 20 h. The reaction was cooled to room temperature and concentrated. The residue was purified by silica gel column chromatography using dichloromethane to afford 1.04 g (98%) of 1-95 as light yellow solid.

**Synthesis of 3-methoxy-6-methyl-2-(3-nitro-phenyl)-pyridine (I-113).** To 1-95 (0.5 g, 2.0 mmol), 3-nitrophcnylboronic acid (0.5 g, 3.06 mmol), triphcnylphosphinc (0.11 g, 0.4 mmol), K₂CO₃ (0.83 g, 6.0 mmol) and palladium(II) acetate (0.045 g, 0.2 mmol) was added DME (16 mL), and EtOH-H₂O (1:1, 4 mL). Argon gas was bubbled through the stirred reaction for 5 min. The reaction was stirred at 60 °C under argon for 18 h. The reaction was cooled to room temperature, concentrated, and H₂O and dichloromethane (40 mL each) were added. The organic layer was separated and the aqueous layer was extracted with dichloromethane (2 x 25 mL). The combined organic extracts were dried with Na₂SO₄, filtered, and concentrated. The residue was purified by silica gel column chromatography using 1:1 dichloromethane-hexanes then dichloromethane to afford 0.22 g (44%) of 1-113 as a light yellow solid.

**Synthesis of 6-bromomethyl-3-methoxy-2-(3-nitro-phenyl)-pyridine (I-114).** To 1-113 (0.21 g, 0.86 mmol) and NBS (0.17 g, 0.95 mmol) in CCl₄ (10mL) was added benzoylperoxide (0.02 g, 0.08 mmol). The reaction was stirred at 60 °C under N₂ for 18 h. The reaction was cooled to room temperature and concentrated. The residue was dissolved in a mixture of dichloromethane and hexanes (1:1, 8 mL) and purified by silica gel column chromatography using 1:1 dichloromethane:hexanes then dichloromethane to afford 0.15 g (55%) of I-114 as a light brown solid.

**Synthesis of 6-(4-fluoro-benzyl)-3-methoxy-2-(3-nitro-phenyl)-pyridine** (P-023). To I-114 (0.05 g, 0.15 mmol), 4-fluorophenylboronic acid (1) (0.032 g, 0.23 mmol), triphenylphosphine (0.004 g, 0.015 mmol), K₃PO₄ (0.066 g, 0.31 mmol), and palladium(II)acetate (0.002 g, 0.008 mmol) was added DME (1.8 mL), and EtOH-H₂O (1:1, 0.6 mL). The reaction was stirred at 160 °C for 5 min using Biotage-60 Microwave Synthsizer. The reaction was cooled to room temperature and concentrated. The residue was purified by silica gel column chromatography using 1:1 dichloromethane-hexanes then dichloromethane to afford 0.024 g (46%) of P-023 as a light brown viscous liquid. 1H NMR (CDCl₃, 400 MHz): 8.89 (dd, *J* = 2.0, 1.6 Hz, 1 H), 8.32-8.36 (m, I H), 8.2-8.24 (m, 1 H), 7.59 (t, *J* = 8.4 Hz, 1 H), 7.22-7.3 (m, 3 H), 7.07 (d, *J* = 8.4 Hz, 1 H), 6.96-7.04 (m, 2 H), 4.14 (s, 2 H), 3.88 (s, 3 H); MS(APCI+): 339.1 (M+1), LC-MS: 100%.

**6-(4-Fluoro-benzyl)-3-methoxy-2-(3-nitro-phenyl)-pyridine 1-oxide (B-080).** A reaction mixture of compound P-023 (100 mg, 0.3 mmole) and 3-chloroperoxybenzoic acid (101 mg, 0.45 mmole) in DCM (10 ml) was stiired at r.t. over night. The reaction mixture washed with saturated NaHCO3 (3x 6 ml), water (2 x 8 ml), dried over Na2SO4. After removal of solvent, the residue was washed with ether (5 ml)/Hexane (5 ml) to give 66 mg of product B-080. Yield: 62 %; ¹H-NMR (400 MHz, DMSO-d₆); MS(APCI+): 355.1 (M+1); LC-MS: 97 %. 1H NMR (CDCl3, 400MHz): d = 3.79 (3H, s), 4.19 (2H, s), 6.90 (1H, d, J = 9.2 Hz), 6.98 (1H, d, J = 8.8 Hz), 7.04 (2H, m), 7.27 (2H, m), 7.64 (1H, dd, J = 8 and 8 Hz), 7.89 (1H, m), 8.28 (1H, m), 8.39 (1H, m).

### Synthesis of B-081

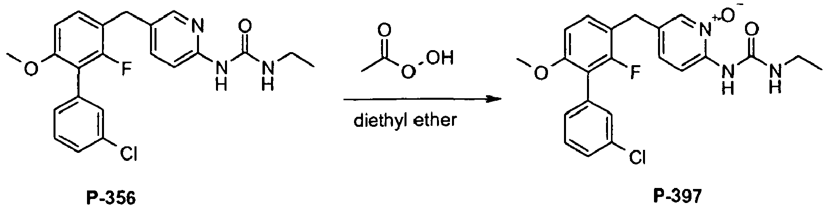

Synthesis of 1-[5-(3'-Chloro-2-fluoro-6-methoxy-biphenyl-3-ylmethyl)-1-oxy-pyridin-2-yl]-3-ethyl-urea (P-397). A solution of P-356 (82.7 mg, 0.200 mmol) in diethyl ether (5 mL) was stirred at room temperature with peracetic acid (32% wt., 0.06 mL, 0.300 mmol) for 3 h. The mixture was concentrated and the residue purified by silica gel column chromatography (19:1 dichloromethane/methanol) to give P-397 (27.6 mg, 32% yield). ¹H NMR (400 MHz, CDCl₃): 1.12 (t, 3 H) 3.17 - 3.35 (m, 2 H), 3.72 - 3.80 (m, 3 H), 3.87 (s, 2 H), 6.64 - 6.78 (m, 1 H), 6.86 (br s, 1 H), 7.02 - 7.17 (m, 1 H), 7.22 (s, I H), 7.29 - 7.43 (m, 3 H), 7.93 (s, 1 H), 8.36 (d, *J* = 8.9 Hz, 1 H), 9.73 (s, 1 H) ppm. LCMS = 100% purity. APCI (-) = 428.1 (M-2).

### Synthesis of B-082

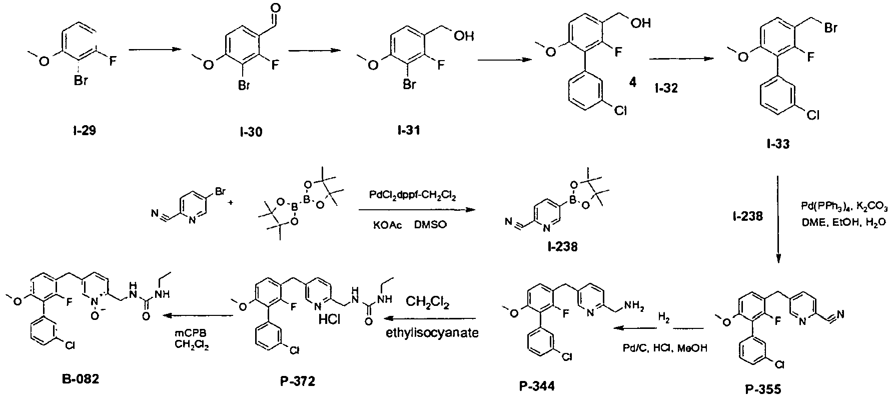

Synthesis of 3-bromo-2-fluoro-4-methoxy-benzaldehyde (1-30). In a 3-necked 250 mL round-bottomed flask equipped with nitrogen lines and a stir bar was placed 2-bromo-1-fluoro-3-methoxy-benzene (I-29, 2.0 g, 9.75 mmol) and dichloromethane (48 mL). The solution was cooled in an ice water bath for 15 minutes and then titanium tetrachloride (5.02 mL, 45.8 mmol) and dichloromethyl methyl ether (1.32 mL, 14.6 mmol) were added and the reaction mixture was allowed to warm to room temperature and react for 2 hours. The reaction mixture was slowly added to ice water (250 mL) and extracted with dichloromethane (2 x 100 mL). The organic portions were combined, washed with a saturated sodium bicarbonate solution (75 mL), water (75 mL) and brine (75 mL), dried (MgSO₄) and concentrated. The crude material was triturated with hexanes (15 mL) to produce 1.67 g of3-bromo-2-fluoro-4-methoxybenzaldehyde (1-30) as an off-white solid in 74% yield. MS(ESI+): 233.2 (M+).

Synthesis of (3-bromo-2-fluoro-4-methoxy-phenyl)-methanol (I-31). In a 100 mL round bottomed flask equipped with a stir bar was placed 3-bromo-2-fluoro-4-methoxy-benzaldehyde (I-30, 1.67 g, 7.17 mmol), methanol (12 mL), dichloromethane (12 mL) and sodium borohydride. The reaction mixture was allowed to stir at room temperature for 17 hours, quenched with water (10 mL) and 1M HCl (5 mL) and extracted with dichloromethane (2 x 30 mL). The organic portions were combined, washed with brine (30 mL), dried (MgSO₄) and concentrated. The crude material was triturated with hexanes (15 mL) to produce 955 mg (57%) of (3-bromo-2-fluoro-4-methoxy-phenyl)-methanol (I-31) as a white solid.

Synthesis of (3'-chloro-2-fluoro-6-methoxy-biphenyl-3-yl)-methanol (I-32). Into a 100 mL round bottom flask was added (3-bromo-2-fluoro-4-methoxy-phenyl)-methanol (I-31, 1.04 g, 4.0 mmol), 3-chlorophenylboronic acid (0.76 g, 4.8 mmol), Pd(PPh₃)₄ (0.45 g, 0.41 mmol), Na₂CO₃ (6 mL, 2M aq), toluene, (32 mL), and EtOH (11 mL). The reaction was degassed with N₂, then stirred at 80 °C for 24 hours. Water was added and the product was extracted with ethyl acetate. The combined organics were concentrated and filtered through a SiO₂ plug eluting with 50% ethyl acetate/hexanes. The solid was triturated with ether and filtered. The filtrate was concentrated and triturated with ether and filtered. The filter cakes were combined and purified by flash column chromatography eluting with 20% acetone/hexanes to give (3'-chloro-2-fluoro-6-methoxy-biphenyl-3-yl)-methanol (I-32, 0.79 g, 67%) as a white solid.

Synthesis of 3-bromomethyl-3'-chloro-2-fluoro-6-methoxy-biphenyl (I-33). Into a 250 mL round bottom flask was added (3'-Chloro-2-fluoro-6-methoxy-biphenyl-3-yl)-methanol synthesized above (1.21 g, 4.54 mmol), dichloromethane (20 mL), PPh₃ (1.19 g, 4.54 mmol), and the solution was cooled to 0 °C. NBS (0.81 g, 4.54 mmol) was added and the reaction stirred for 2 hours at 0 °C. The organics were washed with H₂O and concentrated. The residue was purified by flash column chromatography eluting with 8% ethyl acetate/hexanes to give 3-bromomethyl-3'-chloro-2-fluoro-6-methoxy-biphenyl (I-33, 956 mg, 64%) as an off-white solid.

Synthesis of 5-(4,4,5,5-Tetramethyl-[1,3,2]dioxaborolan-2-yl)-pyridine-2-carbonitrile (I-238). Into a 250 mL round-bottomed flask was added 3.0 g of 5-bromopyridine-2-carbonitrile (3.0 g, 16.39 mmol), bis(piniacolato)diboron (4.58 g, 18.03 mmol), KOAc (5.47 g, 55.74 mmol), and DMSO (100 mL). After degassing for 20 minutes, PdCl₂dppf-CH₂Cl₂ (1.39 g, 1.64 mmol) was added and the solution was stirred for 24 hours at 80 °C, and then at room temperature for 3 days. 50 mL water was added and the product was extracted with ethyl acetate. The combined organics were washed with brine, dried over Na₂SO₄ and concentrated. The dark-colored residue was purified by flash column chromatography eluting with 20% acetone/hexanes to give a red solid. The solid was triturated with hexane to give 1.72 g (46%) of 1-238 as a light-pink solid.

**Synthesis of [5-(3'-Chloro-2-fluoro-6-methoxy-biphenyl-3-ylmethyl)-pyridin-2-ylmethyl]-carbamic acid tert-butyl ester (P-355).** Into a 20 mL vial was added I-33 (401 mg, 1.22 mmol), 1-238 (336 mg, 1.46 mmol), K₂CO₃ (504 mg, 3.65 mmol), DME (5 mL), water (0.5 mL), ethanol (0.5 mL), and the suspension was degasses for 15 minutes. Tetrakis(triphenylphosphine) palladium(0) (141 mg, 0.12 mmol) was added and the reaction stirred at 80 °C for 16 hours. The reaction was diluted with water and extracted with ethyl acetate. The organics were concentrated and purified by flash column chromatography eluting with 15 - 20% ethyl acetate/hexanes to afford P-355 (64 mg, 15%) as a light-yellow oil. ¹H NMR (400 MHz,CDCl₃) δ 8.62 (br s, 1 H), 7.68 - 7.56 (m, 2 H), 7.40 - 7.30 (m, 3 H), 7.26 - 7.21 (m, 1 H), 7.13 (t, *J* = 8.4 Hz, 1 H), 6.75 (d, *J* = 8.3 Hz, 1 H), 4.03 (br s, 2 H), 3.78 (s, 3 H) ppm. LC/MS = 98.5%, 353.0 (APCI+).

**Synthesis of C-[5-(3'-Chloro-2-fluoro-6-methoxy-biphenyl-3-ylmethyl)-pyridin-2-yl]-methylamine (P-344).** Into a 100 mL round bottom flask was added P-355 (0.55 g, 1.56 mmol), methanol (20 mL), concentrated HCl (0.65 mL, 7.79 mmol), and 10% Pd/C (100 mg). The suspension was stirred under a H₂ balloon for 18 hours, then filtered through Celite. The filtrate was concentrated. To the solid was added 1N aqueous NaOH and the product was extracted with dichloromethane. The dichloromethane was concentrated and purified by flash column chromatography eluting with 5-10% methanol/dichloromethane to give P-344 (89 mg, 16%) as a colorless oil. ¹H NMR (400MHz, DMSO-*d₆*) 8.55 (d, *J* = 1.2 Hz, 1 H), 8.32 (br s, 3 H), 7.71 (dd, *J* = 1.9, 7.9 Hz, 1 H), 7.50 - 7.37 (m, 3 H), 7.36 (d, *J* = 5.4 Hz, 2 H), 7.27 (d, *J* = 6.6 Hz, 1 H), 6.96 (d, *J* = 8.6 Hz, 1 H), 4.75 (br s, 2 H), 4.14 (q, *J* = 5.8 Hz, 2 H), 4.01 (s, 2 H), 3.73 (s, 3 H) ppm. LC/MS = 95.1%, 357.1 (APCI+).

**Synthesis of C-[5-(3'-Chloro-2-fluoro-6-methoxy-biphenyl-3-ylmethyl)-pyridin-2-yl]-methylamine (P-344).** Into a 100 mL round bottom flask was added P-355 (0.55 g, 1.56 mmol), methanol (20 mL), concentrated HCl (0.65 mL, 7.79 mmol), and 10% Pd/C (100 mg). The suspension was stirred under a H₂ balloon for 18 hours, then filtered through Celite. The filtrate was concentrated. To the solid was added 1N aqueous NaOH and the product was extracted with dichloromethane. The dichloromethane was concentrated and purified by flash column chromatography eluting with 5-10% methanol/dichloromethane to give P-344 (89 mg, 16%) as a colorless oil. ¹H NMR (400MHz, DMSO-*d₆*) 8.55 (d, *J* = 1.2 Hz, 1 H), 8.32 (br s, 3 H), 7.71 (dd, *J* = 1.9, 7.9 Hz, 1 H), 7.50 - 7.37 (m, 3 H), 7.36 (d, *J* = 5.4 Hz, 2 H), 7.27 (d, *J* = 6.6 Hz, 1 H), 6.96 (d, *J* = 8.6 Hz, 1 H), 4.75 (br s, 2 H), 4.14 (q, *J* = 5.8 Hz, 2 H), 4.01 (s, 2 H), 3.73 (s, 3 H) ppm. LC/MS = 95.1 %, 357.1 (APCI+).

**Synthesis of 1-[5-(3'-Chloro-2-fluoro-6-methoxy-biphenyl-3-ylmethyl)-pyridin-2-ylmethyl]-3-ethyl-urea (P-372).** Into an 8 mL vial was added P-344 (23 mg, 0.064 mmol), dichloromethane (1.5 mL), and the solution was cooled to room temperature. Ethyl isocyanate (8 uL, 0.097 mmol) was added and the reaction was stirred for 18 hours at room temperature and then concentrated. To the resulting solid was added 4.0 M HCl/dioxane and the solution was stirred for 18 hours at room temperature and then concentrated. The oil which was obtained was treated with ether to form a solid, which was filtered to afford P-372 (15.1 mg, 50%) as a white solid. ¹H NMR (400 MHz, DMSO-*d₆*) 8.63 (s, 1 H), 8.14 (d, *J* = 8.3 Hz, 1 H), 7.63 (d, *J* = 8.2 Hz, 1 H), 7.51 - 7.33 (m, 4 H), 7.28 (d, *J* = 6.3 Hz, 1 H), 6.98 (d, *J* = 8.6 Hz, 1 H), 6.62 (br s, 1 H), 6.31 (br s, 1 H), 4.42 (s, 2 H), 4.10 (s, 2 H), 3.74 (s, 3 H), 3.01 (q, *J* = 7.1 Hz, 2 H), 0.99 (t, *J* = 7.1 Hz, 3 H) ppm. MS: 428.1 (APCI+).

**1-[5-(3'-Chloro-2-fluoro-6-methoxy-biphenyl-3-ylmethyl)-1-oxy-pyridin-2-ylmethyl]-3-ethyl-urea (B-082).** Into an 18 mL vial was added P-372 (66 mg, 0.15 mmol), dichloromethane (4 mL), and the solution was cooled to 0 °C. mCPBA (69 mg, 0.31 mmol) was added and the reaction was stirred at room temperature for 1 hour after which 5 mL of saturated aqueous NaHCO₃ was added. The layers were separated and the organic layer was washed sequentially with 5 mL each of saturated aqueous NaHCO₃, H₂O, and brine. The residue was then washed with 1N NaOH (2 x 5 mL), water (5 mL), and brine (5 mL). The product was dried over Na₂SO₄, filtered and concentrated to obtain B-082 (14.8 mg, 22%) as a white solid. ¹H NMR (400 MHz, DMSO-*d₆*) 8.19 (s, 1 H), 7.53 - 7.34 (m, 4 H), 7.29 (d, *J* = 6.6 Hz, 1 H), 7.25 - 7.16 (m, 2 H), 6.96 (d, *J* = 8.6 Hz, 1 H), 6.38 (t, *J* = 6.0 Hz, 1 H), 6.17 (t, *J* = 5.4 Hz, 1 H), 4.22 (d, *J* = 6.2 Hz, 2 H), 3.91 (s, 2 H), 3.73 (s, 3 H), 3.09 - 2.91 (m, 2 H), 0.97 (t, *J* = 7.2 Hz, 3 H) ppm. LC/MS = 100.0%, 444.1 (APCI+).

### Synthesis of B-083

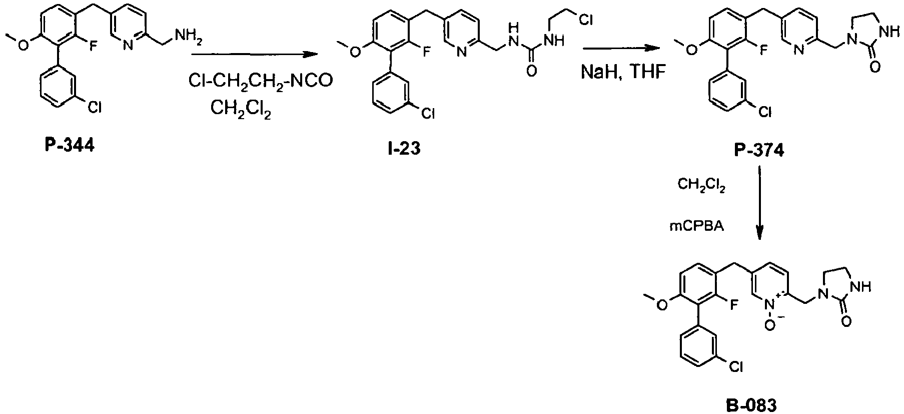

**Synthesis of 1-(2-Chloro-ethyl)-3-[5-(3'-chloro-2-fluoro-6-methoxy-biphenyl-3-ylmethyl)-pyridin-2-ylmethyl]-urea (I-239).** Into an 8 mL vial was added P-344 (63 mg, 0.177 mmol), dichloromethane (2 mL), and the reaction was cooled to 0 °C. Chloroethyl isocyanate (19 mg, 0.177 mmol) was added and the reaction was stirred at room temperature for 1 hour and then concentrated to yield I-239 which was used as is.

**Synthesis of 1-[5-(3'-Chloro-2-fluoro-6-methoxy-biphenyl-3-ylmethyl)-pyridin-2-ylmethyl]-imidazolidin-2-one (P-374).** Into an 8 mL vial was added I-239 (82 mg, 0.177 mmol), THF (2 mL), and the suspension was cooled to 0 °C. Sodium hydride (8 mg, 0.212 mmol) was added and the reaction was stirred at room temperature for 3 days. An additional 4 mg of sodium hydride was added and the reaction was stirred at 50 °C for 1 hour. Water was added and the product was extracted with ethyl acetate. The organics were concentrated and purified by flash column chromatography eluting with 25 - 50% acetonc/dichloromethane to give P-374 (35 mg, 47%, 2 steps) as a white solid. ¹H NMR (400 MHz, DMSO-d₆) 8.42 (d, *J* = 1.5 Hz, 1 H), 7.60 (dd, *J* = 1.9, 7.9 Hz, 1 H), 7.49 - 7.39 (m, 2 H), 7.37 (s, 1 H), 7.34 (t, *J* = 8.7 Hz, 1 H), 7.28 (d, *J* = 6.7 Hz, 1 H), 7.19 (d, *J* = 7.9 Hz, 1 H), 6.94 (d, *J* = 8.6 Hz, 1 H), 6.41 (s, 1 H), 4.28 (s, 2 H), 3.95 (s, 2 H), 3.72 (s, 3 H), 3.31 - 3.19 (m, 4 H) ppm. LC/MS = 100.0%, 426.1 (APCI+).

**1-[5-(3'-Chloro-2-fluoro-6-methoxy-biphenyl-3-ylmethyl)-1-oxy-pyridin-2-ylmethyl]-imidazolidin-2-one (B-083).** Into an 18 mL vial was added P-374 (62 mg, 0.15 mmol), dichloromethane (4 mL), and the solution was cooled to 0 °C. mCPBA (82 mg, 0.36 mmol) was added and the reaction was stirred at room temperature for 1 hour after which 5 mL of aqueous 1N NaOH was added. The layers were separated and the aqueous layer was extracted with dichloromethane (2 x 3 mL). The organics were combined and washed with water (5 mL) and brine (5 mL) and then concentrated. The residue was taken up in 5 mL of EtOAc and it was washed with 5 mL of brine, dried over Na₂SO₄, and concentrated to a solid. The residue was triturated with ether to afford B-083 (23.5 mg, 35%) as a white solid. ¹H NMR (400 MHz, DMSO-*d₆*) 8.23 (s, I H), 7.49 - 7.34 (m, 4 H), 7.32 - 7.16 (m, 3 H), 6.96 (d, *J* = 8.6 Hz, 1 H), 6.56 (s, 1 H), 4.31 (s, 2 H), 3.93 (s, 2 H), 3.73 (s, 3 H), 3.45 - 3.36 (m, 2 H), 3.32 - 3.25 (m, 2 H) ppm. LC/MS = 93.4%, 442.0 (APCI+).

### Synthesis of B-084

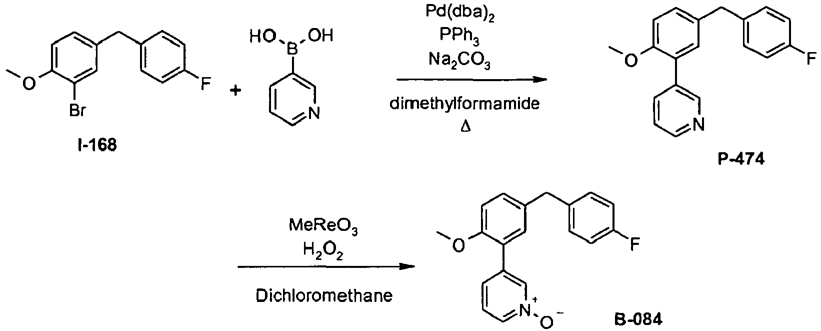

**Synthesis of 3-[5-(4-Fluoro-benzyl)-2-methoxy-phenyl]-pyridine (P-474).** A suspension of I-168 (158 mg, 0.53 mmol), 3-pyridineboronic acid (61.5 mg, 0.50 mmol), palladium(0)bis(dibenzylideneacetone) (14.4 mg, 0.025 mmol), and triphenylphosphine (13.1 mg, 0.050 mmol) in dimethylformamide (5 mL) and I M aqueous sodium carbonate (1.5 mL, 1.5 mmol) was heated to 85 °C with stirring overnight. The solvent was removed under vacuum and the residue suspended in ethyl acetate (15 mL). The organic suspension was washed with water (3 x 15 mL) and brine, dried over sodium sulfate and the solvent removed under vacuum to give crude material. The residue was purified by silica gel preparatory thin layer chromatography to afford 69.1 mg (47%) of P-474

**Synthesis of 3-[5-(4-Fluoro-benzyl)-2-methoxy-phenyl]-pyridine 1-oxide (B-084).** A vial was charged with P-474 (60 mg, 0.20 mmol), methyl ruthenium oxide (2.5 mg, 0.010 mmol), 30% aqueous hydrogen peroxide (0.5 mL), and dichloromethane (1.0 mL). The reaction was allowed to stir at room temperature for 3 days. The biphasic mixture was treated with catalytic amount of manganese dioxide (1.7 mg, 0.02 mmol) and carefully stirred until the oxygen evolution ceased (1 h). The phases were separated, the aqueous layer extracted into dichloromethane (2 x 1 mL), the organic layers combined, dried over sodium sulfate, and the solvent removed under vacuum to afford 20.9 mg of B-084 in 34% yield. ¹H NMR (400 MHz, CDCl₃) 3.82 (s, 3 H) 3.94 (s, 2 H) 6.89 - 7.24 (m, 8 H) 7.41 (d, J = 8.1 Hz, 1 H) 8.16 (d, J = 6.3 Hz, I H) 8.44 (s, 1 H) ppm. LCMS = 92.0 % purity.

The following compounds were prepared by methods analogous to those described above

**Table of 1HNMR data:**

| PCTUS ExNo | NB_No | 1H NMR data | LCMS |
|---|---|---|---|
| A-001 | MCLS1226-162-5 | 1H NMR (400 MHz, CHLOROFORM-d) δ ppm 3.85 (s, 3 H) 4.55 (s, 2 H) 6.98 (d, J=8.45 Hz, 1H) 7.31 (d, J=7.78 Hz, 1H) 7.38 (d, J=2.28 Hz, 1 H) 7.43 (dd, J=8.45, 2.28 Hz, 1 H) 7.48 - 7.68 (m, 3 H) 7.77 (s, 1 H) 7.85 (d, J=7.65 Hz, 1 H) 8.19 (dd, J=8.18, 1.34 Hz, 1 H) 8.40 (t, J=1.81 Hz, 1 H) | |
| A-008 | MCLS1407-012-1 | 1H NMR (CHLOROFORM-d, 400MHz): δ = 7.29 - 7.41 (m, 7 H), 7.19 - 7.29 (m, 12 H), 7.07 - 7.18 (m, 7 H), 6.71 (s, 2 H), 6.38 (br. s., 2 H), 4.64 (br. s., 4 H), 3.94 (s, 5 H), 3.75 (s, 7 H), 2.14 (s, 1 H), 1.26 (s, 3 H), 0.89 ppm (s, 1 H) | |
| A-010 | MCLS1316-168-6 | 1H NMR (400 MHz, CHLOROFORM-d) δ ppm 7.94 (br. s., 1 H) 7.65 (dd, J=9.1, 1.9 Hz, 1 H) 7.30 - 7.40 (m, 3 H) 7.23 - 7.29 (m, 2 H) 7.12 (t, J=8.5 Hz, 1 H) 6.73 (d, J=8.6 Hz, 1 H) 3.87 (s, 2 H) 3.77 (s, 3 H) | |
| A-011 | MCLS1417-034-2 | 1H NMR (400 MHz, DMSO, d-6)3.72 (3H, s), 3.82 (2H, s), 6.64 - 6.68 (2H, m) 6.91 - 6.94 (3H, m), 7.20 (1 H, d, J = 8 Hz), 7.26 -7.31 (2H, m), 7.37 (1H, br), 7.41 - 7.48 (2H, m), 7.70 (1H, s). | |
| A-012 | MCLS1316-191-5 | 1H NMR (400 MHz, DMSO-d6) δ ppm 10.95 (s, 1 H) 8.45 (s, 2 H) 7.19 - 7.54 (m, 5 H) 7.07 (s, 2 H) 6.96 (d, J=8.6 Hz, 1 H) 3.89 (s, 2 H) 3.74 (s, 3 H) | |
| A-013 | MCLS1316-196-5 | 1H NMR (400 MHz, CHLOROFORM-d) δ ppm 7.89 - 8.04 (m, 2 H) 7.54 - 7.63 (m, 1 H) 7.53 (d, J=7.6 Hz, 1 H) 7.25 - 7.34 (m, 7 H) 7.09 (t, J=8.6 Hz, 1 H) 6.71 (d, J=8.5 Hz, 1 H) 6.46 (d, J=8.5 Hz, 1 H) 3.83 (s, 3 H) 3.75 (s, 3 H) 2.62 (s, 3 H) | |
| A-014 | MCLS1316-198-1 | 1H NMR (400 MHz, CHLOROFORM-d) δ ppm 7.98 (s, 1 H) 7.26 - 7.46 (m, 5 H) 7.05 - 7.17 (m, 3 H) 7.00 (s, 1 H) 6.70 (s, 1 H) 5.47 (br. s., 1 H) 3.81 (br. s., 2 H) 3.76 (s, 3 H) | |
| A-015 | MCLS1316-199-1 | 1 H NMR (400 MHz, CHLOROFORM-d) δ ppm 7.98 (s, 1 H) 7.29 - 7.44 (m, 3 H) 7.06 - 7.15 (m, 2 H) 7.00 (s, 1 H) 6.70 (s, 1 H) 5.47 (br. s., I H) 3.81 (br. s., 2 H) 3.76 (s, 3 H) | |
| A-028 | MCLS1448-049-4 | ¹H NMR (400MHz, DMSO-d₆) δ = 8.09 (s, 1 H), 7.63 (br. s., 1 H), 7.48 - 7.40 (m, 3 H), 7.37 (s, 1 H), 7.32 - 7.25 (m, 4 H), 6.94 (d, *J* = 8.6 Hz, 1 H), 3.89 (s, 2 H), 3.87 (s, 2 H), 3.73 (s, 3 H) | |
| A-029 | MCLS1448-052-2 | 1H NMR (400 MHz, CHLOROFORM-d) δ ppm 1.24 (t, 3 H) 2.63 (t, J=6.37 Hz, 2 H) 3.64 (q, J=6.31 Hz, 2 H) 3.76 (s, 3 H) 3.87 (s, 2 H) 4.15 (q, J=7.20 Hz, 2 H) 6.57 (d, J=8.45 Hz, 1 H) 6.71 (d, J=8.45 Hz, 1 H) 6.92 (s, 1H) 7.09 (t, J=8.52 Hz, 1 H) 7.29 - 7.40 (m, 3 H) 7.44 (dd, J=8.32, 2.15 Hz, 1 H) 8.07 (d, J=1.74 Hz, 1 H) 9.52 (br. s., 1 H) | |
| A-032 | MCLS1448-052-4 | ¹H NMR (400MHz, DMSO-d₆) δ = 9.37 (br. s., 1 H), 8.15 (br. s., 1 H), 8.04 (s, 1 H), 7.59 (d, *J* = 7.6 Hz, 1 H), 7.49 - 7.23 (m, 6 H), 6.94 (d, *J* = 8.6 Hz, 1 H), 3.87 (s, 2 H), 3.73 (s, 3 H), 2.57 - 2.53 (m, 2H), 2.47 - 2.41 (m, 2 H) | |
| A-036 | MCLS1475-009-2 | 1 H NMR (CHLOROFORM-d, 400MHz): δ = 8.06 (s, 1 H), 7.25 - 7.39 (m, 5 H), 7.07 (t, J=8.6 Hz, 1 H), 6.67 (d, J=8.5 Hz, 1 H), 6.47 (d, J=8.7 Hz, 1 H), 3.80 (s, 2 H), 3.74 (s, 3 H). | Calc.343.8; APCI⁺(M+CH O): 371.1, 97.6% |
| A-054 | MCLS1448-179-1 | 1H NMR (400 MHz, DMSO-d6) δ ppm 3.72 (s, 3 H) 3.75 - 3.94 (m, 3 H) 4.56 (br. s., 1 H) 6.40 (d, J=8.45 Hz, 1 H) 6.92 (d, J=8.59 Hz, 1 H 7.21 - 7.47 (m, 5 H) 7.97 (s, 1 H) | |

### Items

1. A compound of formula Ia, Ib, Ic or Id: 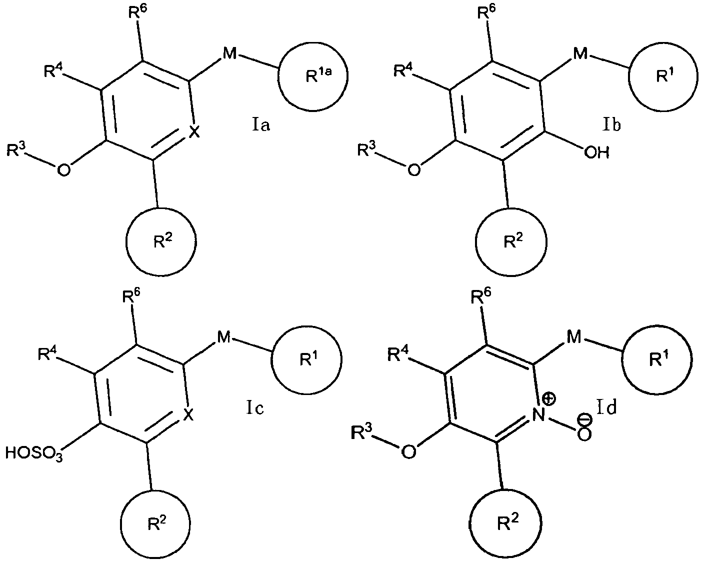 or salt thereof wherein
   R¹ is an optionally substituted carbocycle or optionally substituted heterocycle of three or fewer rings;
   R^{1a} is chosen from
      (a) a residue chosen from 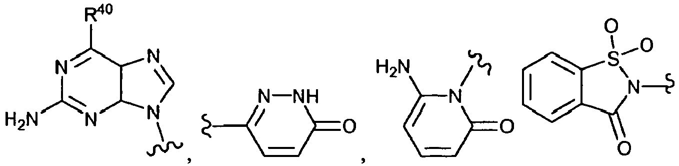 and 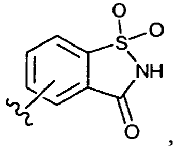 wherein R⁴⁰ is chosen from H, halogen, OH, NH₂ and CH₃;
      (b) a substituted heterocycle of three or fewer rings or substituted carbocycle of three or fewer rings; and
      (c) a heterocycle that is itself substituted with a heterocycle carrying a further substituent;
   wherein substituents on the heterocycle or carbocycle are chosen from hydroxy, carboxy, alkoxycarbonyl, carboxyalkylcarbonylamino, carboxyalkyl, carboxyalkoxy, carboxyalkylthio, carboxyalkylaminocarbonylamino, guanidino, the residue of an amino acid and the residue of an N-methylated amino acid;
   R² is an optionally substituted carbocycle or optionally substituted heterocycle of two or fewer rings;
   R³ is chosen from H, -C(=O)NH₂, -(C₁-C₆)alkyl, halo(C₁-C₆)alkyl, -(C₁-C₆)alkyl-R³⁰, -(C₂-C₆)alkyl-R³¹, and saturated 4- or 5-membered heterocycle optionally substituted with methyl;
   R³⁰ is chosen from -C(=O)NH₂ and 4- or 5-membered heterocycle optionally substituted with methyl;
   R³¹ is chosen from (C₁-C₄)alkoxy, amino, hydroxy, (C₁-C₆)alkylamino and di(C₁-C₆)alkylamino;
   R⁴ is chosen from H and F;
   R⁶ is chosen from H, (C₁-C₆)alkyl and halogen;
   X is N, N→O, or C-R⁵;
   R⁵ is chosen from H, halogen, OH, (C₁-C₆)alkyl, (C₁-C₆)alkoxy, CF₃, CN, NH₂, CH₂OH, CH₂NH₂ and C≡CH; and
   M is chosen from direct bond, -C(R²⁰)(R²¹)-, -O-, -NR²²-, -S(O)ₙ-, -C(=O)-, -C(R²⁰)(R²¹)C(R²⁰)(R²¹)-, -C(R²⁰)=C(R²¹)-, -C(R²⁰)(R²¹)-O-, -C(R²⁰)(R²¹)-NR²²-, - C(R²⁰)(R²¹)-S(O)ₙ-, -C(R²⁰)(R²¹)-C(=O)-, -O-C(R²⁰)(R²¹)-, -NR²²-C(R²⁰)(R²¹)-, -S(O)ₙ-C(R²⁰)(R²¹)-, -C(=O)-C(R²⁰)(R²¹)- and 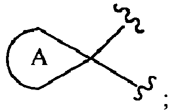 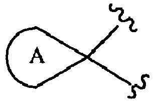 is a five or six-membered ring optionally substituted with methyl; n is zero, one or two; and
   R²⁰, R²¹ and R²² are selected independently in each occurrence from H and (C₁-C₄)alkyl.
2. A compound or salt of formula Ia or Ic according to item 1 wherein X is N or N→O.
3. A compound or salt of formula Ia or Ic according to item 1 wherein X is CR⁵.
4. A compound or salt according to any of items 1, 2 or 3 wherein M is chosen from direct bond, -CH₂-, -CH(OH)-, -C[(CH₃)(OH)]-, -C[(CH₃)(NH₂)]-, -C(=O)-, -O-, - NH-, -N(CH₃)-, -S(O)ₙ-, -CH₂NH-, -CH₂CH₂-, -CH=CH-, -CH₂S(O)ₙ-, -CH₂O- and 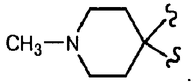
5. A compound or salt according to any of items 1, 2 or 3 wherein R¹ or R^{1a} is a substituted phenyl.
6. A compound or salt according to any of items 1, 2 or 3 wherein R¹ or R^{1a} is an optionally substituted heterocycle chosen from pyrazole, pyrrole, indole, quinoline, isoquinoline, tetrahydroisoquinoline, benzofuran, benzodioxan, benzodioxole, morpholine, thiazole, pyridine, pyridine N-oxide, pyrimidine, thiene, furan, oxazole, oxazoline, oxazolidine, isoxazolidine, isoxazole, dioxane, azetidine, piperazine, piperidine, pyrrolidine, pyridazine, azepine, pyrazolidine, imidazole, imidazoline, imidazolidine, purine, imidazolopyridine, pyrazine, thiazolidine, isothiazole, 1,2-thiazine-1,1-dioxide, 2,6,7-trioxabicyclo[2.2.2]octane, quinuclidine, isothiazolidine, benzimidazole, thiadiazole, benzopyran, benzothiazole, benzotriazole, benzoxazole, benzoxadiazole, tetrahydrofuran, tetrahydropyran, benzothiene, thiamorpholine, thiamorpholine sulfoxide, thiamorpholine sulfone, oxadiazole, triazole, tetrazole, isoindole, pyrrolopyridine, triazolopyridine and the dihydro and tetrahydro congeners thereof.
7. A compound or salt according to item 6 wherein R¹ or R^{1a} is an optionally substituted heterocycle chosen from pyrazole, benzodioxole, morpholine, thiazole, pyridine, pyridine N-oxide, pyrimidine, thiene, oxazolidine, isoxazole, azetidine, piperazine, pyrrolidine, imidazole, imidazolidine, imidazolopyridine, pyrazine, 1,2-thiazine-1, 1-dioxide, benzimidazole, thiadiazole, benzotriazole, benzoxazole, oxadiazole, triazole, tetrazole, isoindole, pyrrolopyridine, triazolopyridine and the dihydro and tetrahydro congeners thereof.
8. A compound or salt according to item 5 wherein R¹ is phenyl substituted with a substituent chosen from halogen, haloalkyl, alkyl, acyl, alkoxyalkyl, hydroxyalkyl, carbonyl, phenyl, heteroaryl, benzenesulfonyl, hydroxy, alkoxy, haloalkoxy, oxaalkyl, carboxy, alkoxycarbonyl, alkoxycarbonylalkyl, alkoxycarbonylamino, carboxyalkyl, carboxyalkoxy, carboxyalkylthio, alkoxycarbonylaminoalkyl, carboxyalkylcarbonylamino, carboxamido, aminocarbonyloxy, alkylaminocarbonyl, dialkylaminocarbonyl, aminocarbonylalkyl, cyano, acetoxy, nitro, amino, alkylamino, dialkylamino, aminoalkyl, (alkyl)(aryl)aminoalkyl, alkylaminoalkyl, dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, benzyloxy, the residues of amino acids, amino acid amides, protected residues of aminoacids, protected residues of amino acid amides, N-methylated amino acids and N-methylated amino acid amides.
9. A compound or salt according to item 5 wherein R¹ is phenyl substituted with a substituent chosen from -CH₃, -CH₂CF₃, -CF₃, -CHO, -COOH, -CN, halogen, -OH, , -OEt, -C(=O)NH₂, -C(=O)NHEt, -C(=O)NMe₂ -COOCH₃, -COOEt, -CH₂NHC(=O)NH₂, -CH(CH₃)NHC(=O)NH₂, -CH₂NHC(=O)H, -CH₂NHC(=O)CH₃, -CH₂C(=O)NH₂, -CH₂COOH, -CH₂COOEt, -CH₂NHC(=O)OEt, -CH₂NHC(=O)O-C₆H₅, -CH₂NHC(=O)C(=O)NH₂, -CH₂NHC(=O)NHEt, -C(CH₃)₂OH, -CH₂NHC(=O)N(CH₃)₂, -CH₂NHC(=O)NHCH₃, -CH₂NH₂, -CH(CH₃)NH₂, -C(CH₃)₂NH₂, -CH₂OH, -CH₂CH₂OH, -CH₂NHSO₂CH₃, -CH₂OC(=O)NHEt, -OCH₃, -OC(=O)NH₂, -OCH₂CH₂N(CH₃)₂, -OCH₂CH₂OCH₃, -OCH(CH₃)COOH, -SCH₂COOH, -NHC(=O)NH₂, -NHC(=O)NHEt, -NHCH₃, -NHEt, -NH(tBoc), -NHCH₂COOH, -N(CH₃)CH₂COOH, -NHC(=O)NHCH₂CH₂Cl, -NHSO₂NH₂, -NHEt, -N(CH₃)₂, -NH₂, , -NH(CH₃)C(=O)NH₂, -NHSO₂CH₃, -N(SO₂CH₃)₂, -NHC(=O)OCH₃, -NHC(=O)OtBu, -NHC(=O)CH₃, -SO₂NH₂, -NHC(=O)CH₂CH₂COOH, -NHC(=O)NHCH₂COOH, -CH₂NHCHO, -NHC(=O)NHCH₂COOEt, -NHC(=O)NH(CH₂)₃COOEt, -NHC(=O)NH(CH₂)₂COOEt, -N(CH₃)CH₂CH₂OH, -NHC(=O)OEt, -N(Et)C(=O)OEt, -NHC(=O)NH(CH₂)₂COOH, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc), 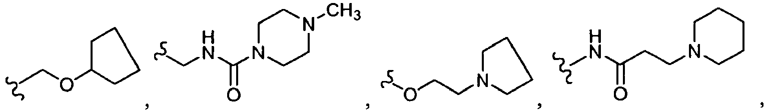 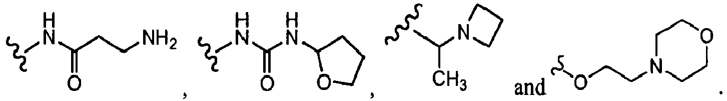
10. A compound or salt according to item 6 wherein R¹ is a heterocycle substituted with a substituent chosen from halogen, haloalkyl, alkyl, acyl, alkoxyalkyl, hydroxyalkyl, carbonyl, phenyl, heteroaryl, benzenesulfonyl, hydroxy, alkoxy, haloalkoxy, oxaalkyl, carboxy, alkoxycarbonyl, alkoxycarbonylamino, alkoxycarbonylaminoalkyl, carboxyalkyl, carboxyalkoxy, carboxyalkylthio, carboxyalkylcarbonylamino, carboxamido, aminocarbonyloxy, alkylaminocarbonyl, dialkylaminocarbonyl, aminocarbonylalkyl, cyano, acetoxy, nitro, amino, alkylamino, dialkylamino, aminoalkyl, (alkyl)(aryl)aminoalkyl, alkylaminoalkyl, dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, benzyloxy, the residues of amino acids, amino acid amides, protected residues of aminoacids, protected residues of amino acid amides, N-methylated amino acids and N-methylated amino acid amides.
11. A compound or salt according to item 6 wherein R¹ is a heterocycle substituted with a substituent chosen from -CH₃, -CH₂CF₃, -CF₃, -CHO, -COOH, -CN, halogen, -OH, , -OEt, -C(=O)NH₂, -C(=O)NHEt, -C(=O)NMe₂ -COOCH₃, -COOEt, -CH₂NHC(=O)NH₂, -CH(CH₃)NHC(=O)NH₂, -CH₂NHC(=O)H, -CH₂NHC(=O)CH₃, -CH₂C(=O)NH₂, -CH₂COOH, -CH₂COOEt, -CH₂NHC(=O)OEt, -CH₂NHC(=O)O-C₆H₅, -CH₂NHC(=O)C(=O)NH₂, -CH₂NHC(=O)NHEt, -C(CH₃)₂OH, -CH₂NHC(=O)N(CH₃)₂, -CH₂NHC(=O)NHCH₃, -CH₂NH₂, -CH(CH₃)NH₂, -C(CH₃)₂NH₂, -CH₂OH, -CH₂CH₂OH, -CH₂NHSO₂CH₃, -CH₂OC(=O)NHEt, -OCH₃, -OC(=O)NH₂, -OCH₂CH₂N(CH₃)₂, -OCH₂CH₂OCH₃, -OCH(CH₃)COOH, -SCH₂COOH, -NHC(=O)NH₂, -NHC(=O)NHEt, -NHCH₃, -NHEt, -NH(tBoc), -NHCH₂COOH, -N(CH₃)CH₂COOH, -NHC(=O)NHCH₂CH₂Cl, -NHSO₂NH₂, -NHEt, ₋N(CH₃)₂, -NH₂,, -NH(CH₃)C(=O)NH₂, -NHSO₂CH₃, -N(SO₂CH₃)₂, -NHC(=O)OCH₃, -NHC(=O)OtBu, -NHC(=O)CH₃, -SO₂NH₂, -NHC(=O)CH₂CH₂COOH, -NHC(=O)NHCH₂COOH, -CH₂NHCHO, -NHC(=O)NHCH₂COOEt, -NHC(=O)NH(CH₂)₃COOEt, -NHC(=O)NH(CH₂)₂COOEt, -N(CH₃)CH₂CH₂OH, -NHC(=O)OEt, -N(Et)C(=O)OEt, -NHC(=O)NH(CH₂)₂COOH, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc), 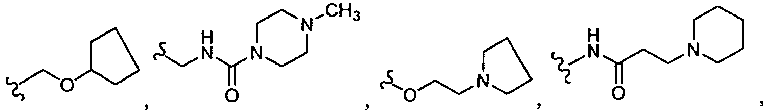 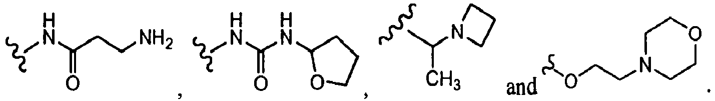
12. A compound or salt of formula Ia according to item 1 wherein
   R^{1a} is chosen from
   a substituted heterocycle of three or fewer rings;
   a monocyclic heterocycle attached to a substituted monocyclic heterocycle; and
   a substituted carbocycle of three or fewer rings
   wherein substituents on the heterocycle or carbocycle are chosen from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂, -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH and 5-tetrazolyl;.
13. A compound or salt according to any of item 1, 2 or 3 wherein R² is chosen from optionally substituted phenyl, optionally substituted monocyclic unsaturated heterocycle, unsubstituted bicyclic unsaturated heterocycle and fluoro-substituted bicyclic unsaturated heterocycle.
14. A compound or salt according to item 13 wherein R² is chosen from optionally substituted phenyl, indole, benzodioxole, benzoxadiazole, benzodioxan, benzimidazole, oxadiazole, pyrazole, pyridine and pyridine N-oxide.
15. A compound or salt according to item 14 wherein R² is chosen from meta-substituted phenyl, indole, benzodioxole, 2,2-difluorobenzodioxole, benzooxadiazole, benzimidazole, 5-(pyridin-4-yl)[1,2,4]oxadiazole, 5-(pyridin-4-yl)[1,3,4]oxadiazole, benzodioxan, 4-chloropyrazole, 4-(pyridin-4-yl)pyrazole, 6-chloropyridine, 3-(trifluoromethyl)pyrazole, and pyridine N-oxide.
16. A compound or salt according to item 14 wherein R² is substituted phenyl: 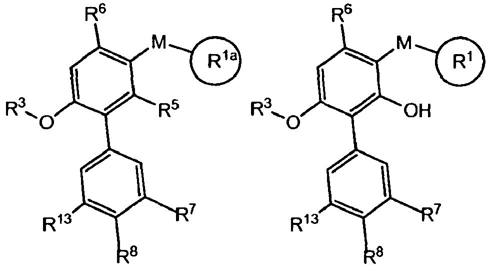 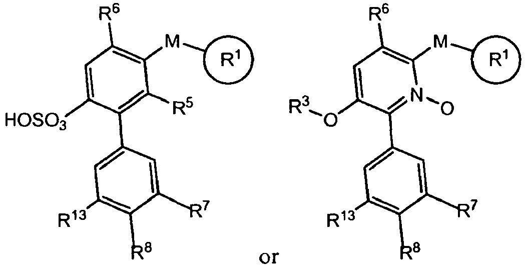
   wherein R⁷ is chosen from hydrogen, halogen, nitro, cyano, halo(C₁-C₆)alkyl, hydroxy, (C₁-C₆)alkoxy, (C₁-C₆)oxaalkyl, carboxy, (C₁-C₆)alkoxycarbonyl, aminocarbonyl (-CONH₂), (C₁-C₆)alkylaminocarbonyl, acyl, hydroxy(C₁-C₆)alkyl, halo(C₁-C₆)alkoxy, amino(C₁-C₆)alkyl, ,amino, (C₁-C₆)alkylamino, di[(C₁-C₆)alkyl]amino, mercapto, (C₁-C₆)alkylthio, (C₁-C₆)alkylsulfinyl, (C₁-C₆)allcylsulfonyl, (C₁-C₆)alkylsulfonamido, acylamino, amidino, phenyl, benzyl, hetercyclyl, phenoxy, benzyloxy, and heteroaryloxy; and
   R⁸ and R¹³ are chosen independently from H and F.
17. A compound or salt according to item 16, wherein R⁸ and R¹³ are H and R⁷ is chosen from hydrogen, fluoro, chloro, bromo, nitro, cyano, acetyl, trifluoromethyl, methoxy, trifluoromethoxy, oxadiazolyl, tetrazolyl, methylthio, methanesulfinyl, methanesulfonyl, methansulfonamido, amino, methoxymethyl, hydroxyethyl, and morpholinyl.
18. A compound or salt of formula Ib, Ic or Id according to item 1, wherein R¹ is chosen from optionally substituted phenyls; optionally substituted five membered heteroaryls selected from thiazoles, thiadiazoles, pyrazoles, oxadiazole, isoxazoles, triazoles, imidazoles, thiophenes, tetrazoles and oxazoles; optionally substituted six membered hereroaryls selected from pyridines, pyrimidines, pyridazinones, pyrimidinone, pyridinone, pyrazines and diazines; optionally substituted 5-and 6-membered non-aryl heterocyclics selected from tetrahydrothiophenes, piperazine, oxazolidinones, imidazolidinones, morpholines, piperidines, pyrrolidinones, pyrrolidinediones, pyrrolidines, piperidinones, piperidinediones and trioxa-bicyclo[2.2.2]octanes; and optionally substituted fused bicycles selected from benzoxazolones, indoles, isoindolinediones, 2H-pyrrolopyridinediones, purines, indolinediones, triazolopyridinones, benzimidazoles, benzoxadiazoles, quinolines and quinolones; wherein the substituents are chosen independently from hydrogen, halogen, halo(C₁-C₆)alkyl, hydroxyl, (C₁-C₆)alkoxy, carboxy, (C₁-C₆)alkoxycarbonyl, aminocarbonyl (-CONH₂), (C₁-C₆)allcylaminocarbonyl, cyano, carbonyl (oxo), acyl, hydroxy(C₁-C₆)alkyl, halo(C₁-C₆)alkoxy, amino(C₁-C₆)alkyl, (C₁-C₆)alkoxy(C₁-C₆)alkyl, nitro, amino, (C₁-C₆)alkylamino, di[(C₁-C₆)alkyl]amino, mercapto, (C₁-C₆)alkylthio, sulfoxide, sulfone, sulfonate, sulfonimide, acylamino, amidino, phenyl, benzyl, heteroaryl, phenoxy, benzyloxy, heteroaryloxy, aminocarbonyl(C₁-C₆)alkyl, (C₁-C₆)alkoxycarbonyl(C₁-C₆)alkyl, carboxy(C₁-C₆)alkyl, formylamino(C₁-C₆)alkyl, carboxy(C₁-C₆)alkylamino, -(CH₂)ₚ-NR¹²CO-(CH₂)_{q}-NR⁹R¹⁰, -NHSO₂R¹¹, - OCH₂CH₂NR⁹R¹⁰ -NHSO₂NR⁹R¹⁰, -SO₂NR⁹R¹⁰, -(CH₂)ₚ-NHCOR⁹, OCONR⁹R¹⁰ and NR¹²COOR¹¹;
   R³ is chosen from -CH₃, -CH₂CH₃, -CF₃, -CHF₂ and -CH₂F;
   R⁵ is chosen from H, -F, -OH, -CH₃, -OCH₃, -CF₃, -CN, -NH₂ and -C≡CH;
   R² is
      (a) phenyl and R⁷ is chosen from H, halogen, nitro, acetyl, hydroxyethyl, -NH₂, - SCH₃, methoxycarbonyl, -SOCH₃, -SO₂CH₃, -OCH₃, -OCF₃, -CN, -CF₃, -CH₂OCH₃ ; or
      (b) benzoxadiazole, benzodioxole, 2,2-difluorobenzodioxole, benzoxadiazole, benzodioxan, benzimidazole, oxadiazole, pyrazole, pyridine and pyridine N-oxide;
   R⁹ is chosen from H, (C₁-C₆)alkyl, halo(C₁-C₆)alkyl, (C₁-C₆)alkoxycarbonyl, carboxy(C₁-C₆)alkyl, (C₁-C₆)alkoxycarboxy(C₁-C₆)alkyl;
   R¹⁰ is H, (C₁-C₆)alkyl, or taken together, or
   R⁹ and R¹⁰ together form a heterocycle optionally substituted with (C₁-C₆)alkyl;
   p is 0 or 1,
   q is 0, 1 or 2,
   R¹¹ is linear (C₁-C₆)alkyl,
   R¹² is H or (C₁-C₆)alkyl; or
   two adjacent substituents together form an optionally substituted fused heterocyclic ring.
19. A compound or salt of formula Ia according to item 1, wherein R^{1a} is chosen from substituted phenyl; substituted five membered heteroaryls selected from thiazoles, thiadiazoles, pyrazoles, oxadiazole, isoxazoles, triazoles, imidazoles, thiophenes, tetrazoles and oxazoles; substituted six membered heteroaryls selected from pyridines, pyrimidines, pyridazinones, pyrimidinone, pyridinone, pyrazines and diazines; substituted 5-and 6- membered non-aryl heterocyclics selected from tetrahydrothiophenes, piperazine, oxazolidinones, imidazolidinones, morpholines, piperidines, pyrrolidinones, pyrrolidinediones, pyrrolidines, piperidinones, piperidinediones and trioxa-bicyclo[2.2.2]octanes; substituted fused bicycles selected from benzoxazolones, indoles, isoindolinediones, 2H-pyrrolopyridinediones, purines, indolinediones, triazolopyridinones, benzimidazoles, benzoxadiazoles, quinolines and quinolones and substituted heterocyclylheterocycles selected from azetidinylpyrimidines, pyrrolidinylpyrimidines, piperidinylpyrimidines, azetidinylpyridines, pyrrolidinylpyridines and piperidinylpyridines; wherein the substituents are chosen independently from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂, -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH and 5-tetrazolyl;
   R³ is chosen from -CH₃, -CH₂CH₃, -CF₃, -CHF₂ and -CH₂F;
   R⁵ is chosen from H, -F, -OH, -CH₃, -OCH₃, -CF₃, -CN, -NH₂ and -C≡CH;
   R² is
      (a) phenyl and R⁷ is chosen from H, halogen, nitro, acetyl, hydroxyethyl, -NH₂, - SCH₃, methoxycarbonyl, -SOCH₃, -SO₂CH₃ -OCH₃, -OCF₃, -CN, -CF₃, -CH₂OCH₃ or
      (b) benzoxadiazole, benzodioxole, 2,2-difluorobenzodioxole, benzoxadiazole, benzodioxan, benzimidazole, oxadiazole, pyrazole, pyridine and pyridine N-oxide;
   R⁹ is chosen from H, (C₁-C₆)alkyl, halo(C₁-C₆)alkyl, (C₁-C₆)alkoxycarbonyl, carboxy(C₁-C₆)alkyl, (C₁-C₆)alkoxycarboxy(C₁-C₆)alkyl;
   R¹⁰ is H, (C₁-C₆)alkyl, or taken together, or
   R⁹ and R¹⁰ together form a heterocycle optionally substituted with (C₁-C₆)alkyl;
   p is 0 or 1,
   q is 0, 1 or 2,
   R¹¹ is linear (C₁-C₆)alkyl,
   R¹² is H or (C₁-C₆)alkyl; or two adjacent substituents together form an optionally substituted fused heterocyclic ring.
20. A compound or salt according to item 1 of formula 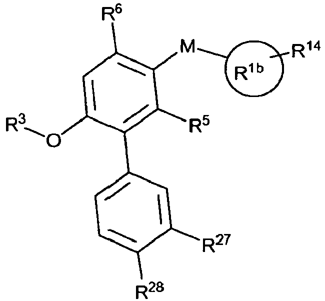 wherein
   R^{1b} is phenyl, five-membered heteroaryl, six-membered heteroaryl, 4-7 membered non-aryl heterocycle or fused bicycle;
   R¹⁴ is chosen from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂, -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH, 5-tetrazolyl and monocyclic heterocycle substituted with any of the foregoing;
   R²⁷ is chosen from hydrogen, halogen, nitro, cyano, halo(C₁-C₆)alkyl, hydroxy, (C₁-C₆)alkoxy, (C₁-C₆)oxaalkyl, carboxy, (C₁-C₆)alkoxycarbonyl, aminocarbonyl (-CONH₂), (C₁-C₆)alkylaminocarbonyl, acyl, hydroxy(C₁-C₆)alkyl, halo(C₁-C₆)alkoxy, amino(C₁-C₆)alkyl, ,amino, (C₁-C₆)alkylamino, di[(C₁-C₆)alkyl]amino, mercapto, (C₁-C₆)alkylthio, (C₁-C₆)alkylsulfinyl, (C₁-C₆)alkylsulfonyl, (C₁-C₆)alkylsulfonamido, acylamino, amidino, phenyl, benzyl, hetercyclyl, phenoxy, benzyloxy, and heteroaryloxy;
   R²⁸ is chosen from H and F, or
   R²⁷ together with R²⁸ forms a five-membered ring.
21. A compound or salt according to item 1 of formula 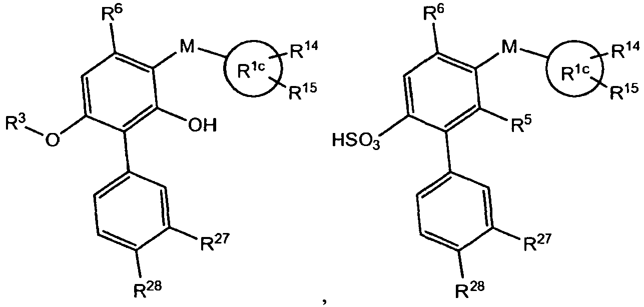 or 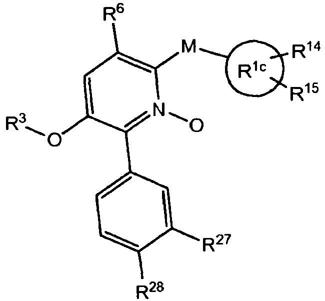 wherein
   R^{1c} is phenyl, five-membered heteroaryl, six-membered heteroaryl, 4-7 membered non-aryl heterocycle or fused bicycle;
   R¹⁴ is chosen from H, -CH₂NHC(=O)NH₂, -NHC(=O)NH₂, -NHC(=O)NHEt, -CH₃, -CH₂CF₃, -CH₂NHC(=O)CH₃, -NHCH₃, -NHEt, -NH(tBoc), -CHO, -NHC(=O)NHCH₂CH₂Cl, -NHSO₂NH₂, -NHEt, -N(CH₃)₂, -NH₂, -COOH, -C(=O)NH₂, -CH₂C(=O)NH₂, -CH₂COOH, -CH₂COOEt, -CN, -OCH₃, -OC(=O)NH₂, -NH(CH₃)C(=O)NH₂, halogen, -CH₂NHC(=O)OEt, -NHSO₂CH₃, -N(SO₂CH₃)₂ -NHC(=O)OCH₃, -OH, -CH₂NHC(=O)N(CH₃)₂, -CH₂NH₂, -CH₂OH, -CH₂CH₂OH, -SO₂NH₂, -NHC(=O)NHCH₂COOH, -CH₂NHCHO, -NHC(=O)NHCH₂COOEt, -COOCH₃, , -COOEt, -NHC(=O)NH(CH₂)₃COOEt, -NHC(=O)NH(CH₂)₂COOEt, -OCH(CH₃)COOH, -SCH₂COOH, -NH(Et)C(=O)OEt, -NHC(=O)NH(CH₂)₂COOH, -CH₂NHSO₂CH₃ -OEt, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc), -OCH₂CH₂N(CH₃)₂, -OCH₂CH₂OCH₃, 3'-nitro-6-methoxybiphenyl-3-ylmethyl, tetrahydroimidazol-2-on-1-yl, 3-methyltetrahydroimidazol-2-one-1-yl, pyrazol- 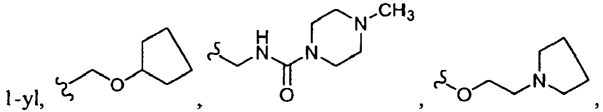 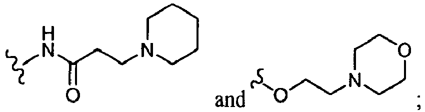
   R¹⁵ is chosen from H, NO₂, OH, NH₂, and -NHSO₂NH₂; or
   R¹⁵ together with R¹⁴ forms methylene dioxy;
   R²⁷ is chosen from hydrogen, halogen, nitro, cyano, halo(C₁-C₆)alkyl, hydroxy, (C₁-C₆)alkoxy, (C₁-C₆)oxaalkyl, carboxy, (C₁-C₆)alkoxycarbonyl, aminocarbonyl (-CONH₂), (C₁-C₆)alkylaminocarbonyl, acyl, hydroxy(C₁-C₆)alkyl, halo(C₁-C₆)alkoxy, amino(C₁-C₆)alkyl, ,amino, (C₁-C₆)alkylamino, di[(C₁-C₆)alkyl]amino, mercapto, (C₁-C₆)alkylthio, (C₁-C₆)alkylsulfinyl, (C₁-C₆)alkylsulfonyl, (C₁-C₆)alkylsulfonamido, acylamino, amidino, phenyl, benzyl, hetercyclyl, phenoxy, benzyloxy, and heteroaryloxy;
   R²⁸ is chosen from H and F, or
   R²⁷ together with R²⁸ forms a five-membered ring.
22. A compound or salt according to item 20 or 21 wherein R²⁷ and R²⁸ represent a fused heterocycle at 3- and 4- positions so that the residue formed from R²⁷ and R²⁸ together with the phenyl to which they are attached is chosen from: 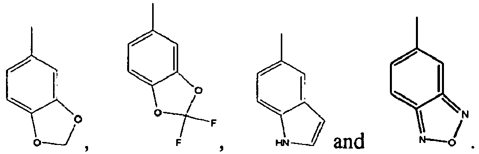
23. A compound or salt according to item 20 or 21 wherein R²⁷ is chosen from halogen, nitro, acetyl, hydroxyethyl, amino, methylthio, trifluoromethyl, methoxymethyl, methoxycarbonyl, trifluoromethoxy, cyano and 1,3,4-thiadiazol-2-yl, or taken together R⁷ and R⁸ are methylenedioxy or difluoromethylenedioxy.
24. A compound or salt according to item 23 wherein R^{1b} or R^{1c} is chosen from a benzene ring, a triazole, a pyridine or pyridine-N-oxide, a pyrazole, a tetrahydrothiophene, an imidazole, a pyrimidine, a thiadiazole, and an imidazopyridine.
25. A compound or salt according to any of items 1-3 wherein R⁵ is fluoro, H, CN or OH.
26. A compound or salt according to any of items 1-3 wherein R³ is methyl or fluoromethyl.
27. A compound or salt according to item 1 of formula: 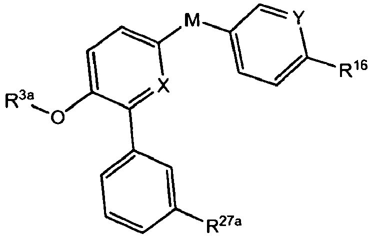 wherein
   R^{3a} is methyl, fluorinated methyl or HSO₃;
   Y is CH, CF or N→O;
   R^{27a} is chosen from halogen, cyano, acetyl, methylthio, nitro and trifluoromethyl; and
   R¹⁶ is chosen from -NR¹⁷C(=O)NR¹⁸R¹⁹ and 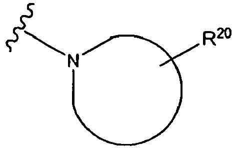
   wherein 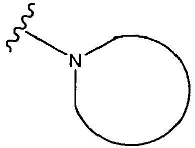 is a 4-7 membered ring heterocycle attached through its nitrogen;
   R¹⁷, and R¹⁸ are independently chosen from H, (C₁-C₆)alkyl and halo(C₁-C₆)alkyl;
   R¹⁹ is chosen from H, (C₁-C₆)alkyl, halo(C₁-C₆)alkyl, -[(C₁-C₆)alkyl]COOH, and -[(C₁-C₆)alkyl]COO(C₁-C₆)alkyl; and
   R²⁰ is chosen from a carboxylic acid, a carboxamide, a carboxylic ester, a primary, secondary or tertiary alcohol and a primary, secondary or tertiary amine.
28. A salt of a compound of any of items 1-3 wherein the salt is a pharmaceutically acceptable salt.
29. A pharmaceutical composition comprising a pharmaceutically acceptable carrier and a compound or pharmaceutically acceptable salt according to any of items 1-3.
30. A pharmaceutical composition comprising
   (a) a pharmaceutically acceptable carrier;
   (b) a compound or pharmaceutically acceptable salt according to any of items 1-3; and
   (c) a second agent chosen from cholinesterase inhibitors, NMDA antagonists, calpain inhibitors and antioxidants.
31. A pharmaceutical composition according to item 30 wherein said second agent is chosen from tacrine, huperzine, donepezil, lanicemine, remacemide, neramexane, memantine, vitamin E and coenzyme Q10.
32. A method for the treatment or prophylaxis of a disease or condition mediated by peripheral phosphodiesterase-4 comprising administering to a mammal a therapeutically effective amount of a compound according to any of items 1-3.
33. A method according to item 32 wherein said disease or condition is chosen from stroke, myocardial infarct, and cardiovascular inflammatory conditions.
34. A method according to item 32 wherein said disease or condition is cancer.
35. A method according to item 32 wherein said disease or condition is chosen from asthma and COPD.
36. A method for treating or preventing bone loss comprising administering to a mammal a therapeutically effective amount of a compound according to any of items 1-3.
37. A method for treating bladder inflammation, bladder overactivity and pain arising from bladder inflammation comprising administering to a mammal a therapeutically effective amount of a compound according to any of items 1-3.
38. Use of a compound or salt according to any of items 1-3 for the treatment, prevention or amelioration of a disorder responsive to inhibition of peripheral phosphodiesterase-4 in a subject in need thereof.
39. Use according to item 38 wherein said disorder is chosen from stroke, myocardial infarct, and cardiovascular inflammatory conditions.
40. Use according to item 38 wherein said disorder is cancer.
41. Use according to item 38 wherein said disorder is chosen from asthma and COPD.
42. Use of a compound or salt according to any of items 1-3 for the treatment or prevention of bone loss.
43. Use of a compound or salt according to any of items 1-3 for the treatment, prevention or amelioration of bladder inflammation, bladder overactivity and pain arising from bladder inflammation.
44. Use of a compound or salt of any of items 1-3 in the manufacture of a medicament for the treatment, prevention or amelioration of a disorder is chosen from stroke, myocardial infarct, cardiovascular inflammatory conditions, cancer. asthma, COPD, bone loss, bladder inflammation, bladder overactivity or pain arising from bladder inflammation.

## Claims

1. A compound of formula Ia, Ib, Ic or Id: 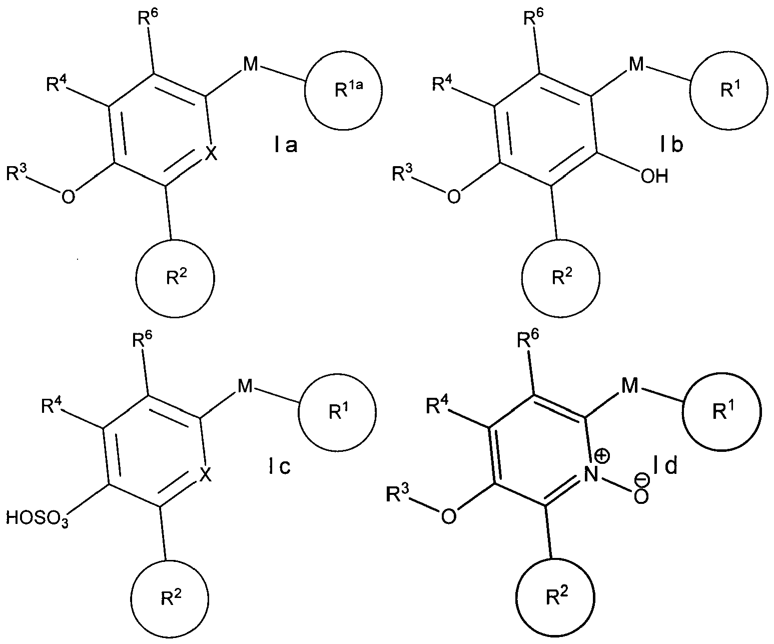 or salt thereof, in particular a pharmaceutically acceptable salt thereof, wherein
R¹ is an optionally substituted carbocycle or optionally substituted heterocycle of three or fewer rings;
R^{1a} is chosen from
(a) a residue chosen from 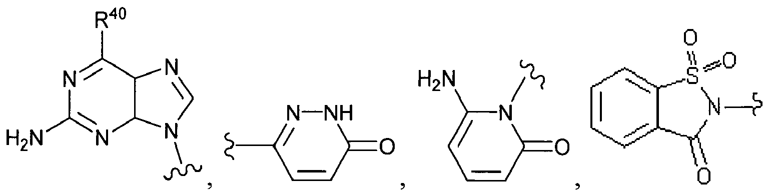 and 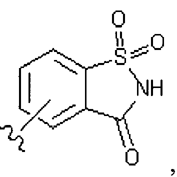 , wherein R⁴⁰ is chosen from H, halogen, OH, NH₂ and CH₃;
(b) a substituted heterocycle of three or fewer rings or substituted carbocycle of three or fewer rings; and
(c) a heterocycle that is itself substituted with a heterocycle carrying a further substituent;
wherein substituents on the heterocycle or carbocycle are chosen from carboxy, alkoxycarbonyl, carboxyalkylcarbonylamino, carboxyalkyl, carboxyalkoxy, carboxyalkylthio, carboxyalkylaminocarbonylamino, guanidino, the residue of an amino acid and the residue of an N-methylated amino acid;
wherein the term "a residue of an amino acid" refers to an amino acid etc. minus the functional groups that are considered part of the bond to the parent structure, or
(d) when the compound or salt is a compound or salt of formula Ia R^{1a} may also be chosen from
i) a substituted heterocycle of three or fewer rings;
ii) a monocyclic heterocycle attached to a substituted monocyclic heterocycle; and
iii)a substituted carbocycle of three or fewer rings
wherein substituents on the heterocycle or carbocycle are chosen from -COOH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂, -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH and 5-tetrazolyl.
R² is is chosen from optionally substituted heterocycle;
R³ is chosen from H, -C(=O)NH₂, -(C₁-C₆)alkyl, halo(C₁-C₆)alkyl, -(C₁-C₆)alkyl-R³⁰, -(C₂-C₆)alkyl-R³¹, and saturated 4- or 5-membered heterocycle optionally substituted with methyl;
R³⁰ is chosen from -C(=O)NH₂ and 4- or 5-membered heterocycle optionally substituted with methyl;
R³¹ is chosen from (C₁-C₄)alkoxy, amino, hydroxy, (C₁-C₆)alkylamino and di(C₁-C₆)alkylamino;
R⁴ is chosen from H and F;
R⁶ is chosen from H, (C₁-C₆)alkyl and halogen;
X is N, N→O, or C-R⁵;
R⁵ is chosen from H, halogen, OH, (C₁-C₆)alkyl, (C₁-C₆)alkoxy, CF₃, CN, NH₂, CH₂OH, CH₂NH₂ and C≡CH; and
M is chosen from direct bond, -C(R²⁰)(R²¹)-, -O-, -NR²²-, -S(O)ₙ-, -C(=O)-, -C(R²⁰)(R²¹)C(R²⁰)(R²¹)-, -C(R²⁰)=C(R²¹)-, -C(R²⁰)(R²¹)-O-, -C(R²⁰)(R²¹)-NR²²-, - C(R²⁰)(R²¹)-S(O)ₙ-, -C(R²⁰)(R²¹)-C(=O)-, -O-C(R²⁰)(R²¹)-, -NR²²-C(R²⁰)(R²¹)-, -S(O)ₙ-C(R²⁰)(R²¹)-, -C(=O)-C(R²⁰)(R²¹)- and 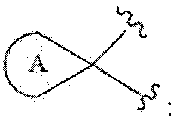 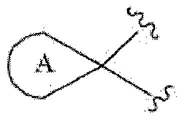 is a five or six-membered ring optionally substituted with methyl:
n is zero, one or two; and
R²⁰, R²¹ and R²² are selected independently in each occurrence from H and (C₁-C₄)alkyl. ; and
R²⁰, R²¹ and R²² are selected independently in each occurrence from H and (C₁-C₄)alkyl,
and wherein "optionally substituted carbocycle" or "optionally substituted heterocycle" refers with regard to R¹ and R² to the optional replacement of one or more hydrogen atoms of R¹ and/or R² with halogen, haloalkyl, alkyl, acyl, alkoxyalkyl, hydroxyalkyl, carbonyl (i.e. oxo), phenyl, heteroaryl, benzenesulfonyl, hydroxy, alkoxy, haloalkoxy, oxaalkyl, carboxy, alkoxycarbonyl [-C(=O)O-alkyl], alkoxycarbonylamino [ -NHC(=O)O-alkyl], alkoxycarbonylaminoalkyl [-alkyl-NHC(=O)O-alkyl], carboxyalkylcarbonylamino [-NHC(=O)-alkyl-COOH], carboxamido [-C(=O)NH₂], aminocarbonyloxy [-OC(=O)NH₂], alkylaminocarbonyl [-C(=O)NH-alkyl], dialkylaminocarbonyl [-C(=O)N(alkyl)₂], aminocarbonylalkyl [-alkyl-C(=O)NH₂], cyano, acetoxy, nitro, amino, alkylamino, dialkylamino, aminoalkyl, (alkyl)(aryl)aminoalkyl, alkylaminoalkyl (including cycloalkylaminoalkyl), dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, benzyloxy, residues of amino acids, amino acid amides, protected residues of aminoacids and their amides, and N-methylated amino acids and amino acid amides, and wherein
for the purpose of R¹, the substituents alkyl, acyl, alkoxyalkyl, hydroxyloweralkyl, phenyl, heteroaryl, benzenesulfonyl, loweralkoxy, haloalkoxy, oxaalkyl, alkoxycarbonyl, alkoxycarbonylamino, carboxamido, alkylaminocarbonyl, amino, alkylamino, (alkyl)(aryl)aminoalkyl, alkylaminoalkyl, heterocyclylalkoxy, alkylthio, sulfonylamino, alkylsulfinyl, alkylsulfonyl, acylaminoalkyl, acylaminoalkoxy, acylamino, amidino, aryl, benzyl, heterocyclyl, heterocyclylalkyl, heterocyclylalkoxy, phenoxy, benzyloxy, heteroaryloxy, heterocyclylamino, oxaalkyl, aminosulfonyl, amidino, guanidino, ureido, benzyloxyphenyl, and benzyloxy may be further substituted with one or two substituents from the list of substituents given above for R¹ and/or R², and
wherein the term "a residue of an amino acid, amino acid amide" refers to an amino acid etc. minus the functional groups that are considered part of the bond to the parent structure, and
wherein the term "alkyl" is intended to include linear, branched, or cyclic hydrocarbon structures and combinations thereof.

2. A compound or salt of formula Ia or Ic according to claim 1 wherein X is N, N→O or CR⁵.

3. A compound or salt according to any of claims 1 or 2 wherein R¹ or R^{1a} is a substituted phenyl, in particular a phenyl substituted with a substituent chosen from halogen, haloalkyl, alkyl, acyl, alkoxyalkyl, hydroxyalkyl, carbonyl, phenyl, heteroaryl, benzenesulfonyl, alkoxy, haloalkoxy, oxaalkyl, carboxy, alkoxycarbonyl, alkoxycarbonylalkyl, alkoxycarbonylamino, carboxyalkyl, carboxyalkoxy, carboxyalkylthio, alkoxycarbonylaminoalkyl, carboxyalkylcarbonylamino, carboxamido, aminocarbonyloxy, alkylaminocarbonyl, dialkylaminocarbonyl, aminocarbonylalkyl, cyano, acetoxy, nitro, amino, alkylamino, dialkylamino, aminoalkyl, (alkyl)(aryl)aminoalkyl, alkylaminoalkyl, dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, benzyloxy, the residues of amino acids, amino acid amides, protected residues of aminoacids, protected residues of amino acid amides, N-methylated amino acids, N-methylated amino acid amides, -CH₃, -CH₂CF₃, -CF₃, -CHO, -COOH, -CN, halogen, -OEt, -C(=O)NH₂, -C(=O)NHEt, -C(=O)NMe₂ -COOCH₃, -COOEt, -CH₂NHC(=O)NH₂, -CH(CH₃)NHC(=O)NH₂, -CH₂NHC(=O)H, -CH₂NHC(=O)CH₃, -CH₂C(=O)NH₂, -CH₂COOH, -CH₂COOEt, -CH₂NHC(=O)OEt, -CH₂NHC(=O)O-C₆H₅, -CH₂NHC(=O)C(=O)NH₂, -CH₂NHC(=O)NHEt, -C(CH₃)₂OH -CH₂NHC(=O)N(CH₃)₂, -CH₂NHC(=O)NHCH₃, -CH₂NH₂, -CH(CH₃)NH₂, -C(CH₃)₂NH₂, -CH₂OH, -CH₂CH₂OH, -CH₂NHSO₂CH₃ -CH₂OC(=O)NHEt, -OCH₃, -OC(=O)NH₂, -OCH₂CH₂N(CH₃)₂, -OCH₂CH₂OCH₃, -OCH(CH₃)COOH, -SCH₂COOH, -NHC(=O)NH₂, -NHC(=O)NHEt, -NHCH₃, -NHEt, -NH(tBoc), -NHCH₂COOH, -N(CH₃)CH₂COOH, -NHC(=O)NHCH₂CH₂Cl, -NHSO₂NH₂, -NHEt, -N(CH₃)₂, -NH₂, , -NH(CH₃)C(=O)NH₂, -NHSO₂CH₃-N(SO₂CH₃)₂ -NHC(=O)OCH₃, -NHC(=O)OtBu, -NHC(=O)CH₃, -SO₂NH₂, -NHC(=O)CH₂CH₂COOH, -NHC(=O)NHCH₂COOH, -CH₂NHCHO, -NHC(=O)NHCH₂COOEt, -NHC(=O)NH(CH₂)₃COOEt, -NHC(=O)NH(CH₂)₂COOEt, -N(CH₃)CH₂CH₂OH, -NHC(=O)OEt, -N(Et)C(=O)OEt, -NHC(=O)NH(CH₂)₂COOH, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc),

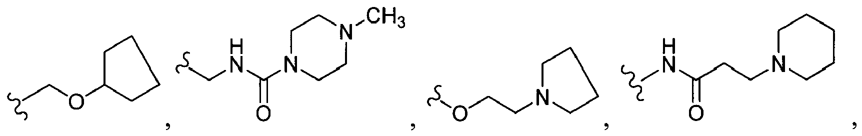

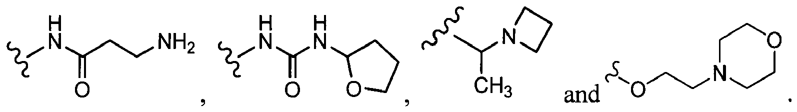

4. A compound or salt according to any of claims 1 or 2 wherein R¹ or R^{1a} is an optionally substituted heterocycle chosen from
i) pyrazole, pyrrole, indole, quinoline, isoquinoline, tetrahydroisoquinoline, benzofuran, benzodioxan, benzodioxole, morpholine, thiazole, pyridine, pyridine N-oxide, pyrimidine, thiophene, furan, oxazole, oxazoline, oxazolidine, isoxazole, dioxane, azetidine, piperazine, piperidine, pyrrolidine, pyridazine, azepine, pyrazolidine, imidazole, imidazoline, imidazolidine, purine, imidazolopyridine, pyrazine, thiazolidine, isothiazole, 1,2-thiazine-1,1-dioxide, 2,6,7-trioxabicyclo[2.2.2]octane, quinuclidine, isothiazolidine, benzimidazole, thiadiazole, benzopyran, benzothiazole, benzotriazole, benzoxazole, benzoxadiazole, tetrahydrofuran, tetrahydropyran, benzothiophene, thiamorpholine, thiamorpholine sulfoxide, thiamorpholine sulfone, oxadiazole, triazole, tetrazole, isoindole, pyrrolopyridine, triazolopyridine;
ii) the dihydro and tetrahydro congeners of i), and
iii) isoxazolidine,
in particular wherein said heterocycle of i), ii) or iii) is a heterocycle substituted with a substituent chosen from halogen, haloalkyl, alkyl, acyl, alkoxyalkyl, hydroxyalkyl, carbonyl, phenyl, heteroaryl, benzenesulfonyl, hydroxy, alkoxy, haloalkoxy, oxaalkyl, carboxy, alkoxycarbonyl, alkoxycarbonylamino, alkoxycarbonylaminoalkyl, carboxyalkyl, carboxyalkoxy, carboxyalkylthio, carboxyalkylcarbonylamino, carboxamido, aminocarbonyloxy, alkylaminocarbonyl, dialkylaminocarbonyl, aminocarbonylalkyl, cyano, acetoxy, nitro, amino, alkylamino, dialkylamino, aminoalkyl, (alkyl)(aryl)aminoalkyl, alkylaminoalkyl, dialkylaminoalkyl, dialkylaminoalkoxy, alkyl(hydroxyalkyl)amino, heterocyclylalkoxy, mercapto, alkylthio, alkylsulfonyl, alkylsulfonylamino, alkylsulfinyl, alkylsulfonyl, arylthio, arylsulfonyl, arylsulfonylamino, arylsulfinyl, arylsulfonyl, acylaminoalkyl, acylaminoalkoxy, acylamino, amidino, aryl, benzyl, heterocyclyl, heterocyclylalkyl, phenoxy, benzyloxy, heteroaryloxy, heterocyclylamino, hydroxyimino, alkoxyimino, oxaalkyl, aminosulfonyl, trityl, amidino, guanidino, ureido, -NHC(=O)NHalkyl, -NHC(=O)NH-heterocyclyl, -alkyl-NHC(=O)N(alkyl)₂, heterocyclylalkylcarbonylamino, benzyloxyphenyl, benzyloxy, the residues of amino acids, amino acid amides, protected residues of aminoacids, protected residues of amino acid amides, N-methylated amino acids, N-methylated amino acid amides, -CH₃, -CH₂CF₃, -CF₃, -CHO, -COOH, -CN, halogen, -OH, , -OEt, -C(=O)NH₂, -C(=O)NHEt, -C(=O)NMe₂ -COOCH₃, -COOEt, -CH₂NHC(=O)NH₂, -CH(CH₃)NHC(=O)NH₂, -CH₂NHC(=O)H, -CH₂NHC(=O)CH₃, -CH₂C(=O)NH₂, -CH₂COOH, -CH₂COOEt, -CH₂NHC(=O)OEt, -CH₂NHC(=O)O-C₆H₅, -CH₂NHC(=O)C(=O)NH₂, -CH₂NHC(=O)NHEt, -C(CH₃)₂OH, -CH₂NHC(=O)N(CH₃)₂, -CH₂NHC(=O)NHCH₃, -CH₂NH₂, -CH(CH₃)NH₂, -C(CH₃)₂NH₂, -CH₂OH, -CH₂CH₂OH, -CH₂NHSO₂CH₃ -CH₂OC(=O)NHEt, -OCH₃, -OC(=O)NH₂, -OCH₂CH₂N(CH₃)₂, -OCH₂CH₂OCH₃, -OCH(CH₃)COOH, -SCH₂COOH, -NHC(=O)NH₂, -NHC(=O)NHEt, -NHCH₃, -NHEt, -NH(tBoc), -NHCH₂COOH, -N(CH₃)CH₂COOH, -NHC(=O)NHCH₂CH₂Cl, -NHSO₂NH₂, -NHEt, -N(CH₃)₂, -NH₂, , -NH(CH₃)C(=O)NH₂, -NHSO₂CH₃-N(SO₂CH₃)₂ -NHC(=O)OCH₃, -NHC(=O)OtBu, -NHC(=O)CH₃, -SO₂NH₂, -NHC(=O)CH₂CH₂COOH, -NHC(=O)NHCH₂COOH, -CH₂NHCHO, -NHC(=O)NHCH₂COOEt, -NHC(=O)NH(CH₂)₃COOEt, -NHC(=O)NH(CH₂)₂COOEt, -N(CH₃)CH₂CH₂OH, -NHC(=O)OEt, -N(Et)C(=O)OEt, -NHC(=O)NH(CH₂)₂COOH, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc),

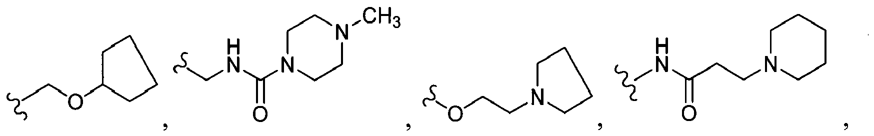

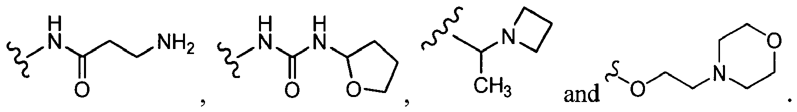

5. A compound or salt according to claim 1 wherein R² is an optionally substituted monocyclic unsaturated heterocycle, unsubstituted bicyclic unsaturated heterocycle or a fluoro-substituted bicyclic unsaturated heterocycle.

6. A compound or salt according to claim 5 wherein R² is chosen from 6-chloropyridine, and pyridine N-oxide.

7. A compound or salt of formula Ib, Ic or Id according to claim 1, wherein R¹ is chosen from optionally substituted phenyls; optionally substituted five membered heteroaryls selected from thiazoles, thiadiazoles, pyrazoles, oxadiazole, isoxazoles, triazoles, imidazoles, thiophenes, tetrazoles and oxazoles; optionally substituted six membered hereroaryls selected from pyridines, pyrimidines, pyridazinones, pyrimidinone, pyridinone, pyrazines and diazines; optionally substituted 5-and 6-membered non-aryl heterocyclics selected from tetrahydrothiophenes, piperazine, oxazolidinones, imidazolidinones, morpholines, piperidines, pyrrolidinones, pyrrolidinediones, pyrrolidines, piperidinones, piperidinediones and trioxa-bicyclo[2.2.2]octanes; and optionally substituted fused bicycles selected from benzoxazolones, indoles, isoindolinediones, 2H-pyrrolopyridinediones, purines, indolinediones, triazolopyridinones, benzimidazoles, benzoxadiazoles, quinolines and quinolones; wherein the substituents are chosen independently from hydrogen, halogen, halo(C₁-C₆)alkyl, hydroxyl, (C₁-C₆)alkoxy, carboxy, (C₁-C₆)alkoxycarbonyl, aminocarbonyl (-CONH₂), (C₁-C₆)alkylaminocarbonyl, cyano, carbonyl (oxo), acyl, hydroxy(C₁-C₆)alkyl, halo(C₁-C₆)alkoxy, amino(C₁-C₆)alkyl, (C₁-C₆)alkoxy(C₁-C₆)alkyl, nitro, amino, (C₁-C₆)alkylamino, di[(C₁-C₆)alkyl]amino, mercapto, (C₁-C₆)alkylthio, sulfoxide, sulfone, sulfonate, sulfonimide, acylamino, amidino, phenyl, benzyl, heteroaryl, phenoxy, benzyloxy, heteroaryloxy, aminocarbonyl(C₁-C₆)alkyl, (C₁-C₆)alkoxycarbonyl(C₁-C₆)alkyl, carboxy(C₁-C₆)alkyl, formylamino(C₁-C₆)alkyl, carboxy(C₁-C₆)alkylamino, -(CH₂)ₚ-NR¹²CO-(CH₂)_{q}-NR⁹R¹⁰, -NHSO₂R¹¹,-OCH₂CH₂NR⁹R¹⁰ -NHSO₂NR⁹R¹⁰, -SO₂NR⁹R¹⁰, -(CH₂)ₚ-NHCOR⁹, OCONR⁹R¹⁰ and NR¹²COOR¹¹;
R³ is chosen from -CH₃, -CH₂CH₃, -CF₃, -CHF₂ and -CH₂F;
R⁵ is chosen from H, -F, -OH, -CH₃, -OCH₃, -CF₃, -CN, -NH₂ and -C≡CH;
R² is benzoxadiazole, benzodioxole, 2,2-difluorobenzodioxole, benzoxadiazole, benzodioxan, benzimidazole, oxadiazole, pyrazole, pyridine and pyridine N-oxide;
R⁹ is chosen from H, (C₁-C₆)alkyl, halo(C₁-C₆)alkyl, (C₁-C₆)alkoxycarbonyl, carboxy(C₁-C₆)alkyl, (C₁-C₆)alkoxycarboxy(C₁-C₆)alkyl;
R¹⁰ is H, (C₁-C₆)alkyl, or taken together, or
R⁹ and R¹⁰ together form a heterocycle optionally substituted with (C₁-C₆)alkyl;
p is 0 or 1,
q is 0, 1 or 2,
R¹¹ is linear (C₁-C₆)alkyl,
R¹² is H or (C₁-C₆)alkyl; or
two adjacent substituents together form an optionally substituted fused heterocyclic ring.

8. A compound of formula Ia: 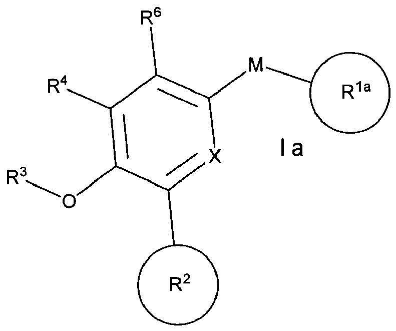 or salt thereof, wherein
R⁴ is chosen from H and F;
R⁶ is chosen from H, (C₁-C₆)alkyl and halogen;
X is N, N→O, or C-R⁵;
M is chosen from direct bond, -C(R²⁰)(R²¹)-, -O-, -NR²²-, S(O)ₙ-, -C(=O)-, -C(R²⁰)(R²¹)C(R²⁰)(R²¹)-, -C(R²⁰)=C(R²¹)-, -C(R²⁰)(R²¹)-O-, -C(R²⁰)(R²¹)-NR²²-, - C(R²⁰)(R²¹)-S(O)ₙ-, -O-C(R²⁰)(R²¹)-, -NR²²-C(R²⁰)(R²¹)-, -S(O)ₙ-C(R²⁰)(R²¹)-, -C(=O)-C(R²⁰)(R²¹)- and 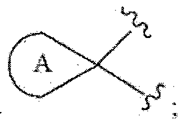 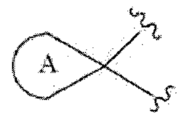 is a five or six-membered ring optionally subtituted with methyl:
n is zero, one or two; and
R²⁰, R²¹ and R²² are selected independently in each occurrence from H and (C₁-C₄)alkyl, and
R^{1a} is chosen from substituted phenyl; substituted five membered heteroaryls selected from thiazoles, thiadiazoles, pyrazoles, oxadiazole, isoxazoles, triazoles, imidazoles, thiophenes, tetrazoles and oxazoles; substituted six membered heteroaryls selected from pyridines, pyrimidines, pyridazinones, pyrimidinone, pyridinone, pyrazines and diazines; substituted 5-and 6- membered non-aryl heterocyclics selected from tetrahydrothiophenes, piperazine, oxazolidinones, imidazolidinones, morpholines, piperidines, pyrrolidinones, pyrrolidinediones, pyrrolidines, piperidinones, piperidinediones and trioxa-bicyclo[2.2.2]octanes; substituted fused bicycles selected from benzoxazolones, indoles, isoindolinediones, 2H-pyrrolopyridinediones, purines, indolinediones, triazolopyridinones, benzimidazoles, benzoxadiazoles, quinolines and quinolones and substituted heterocyclylheterocycles selected from azetidinylpyrimidines, pyrrolidinylpyrimidines, piperidinylpyrimidines, azetidinylpyridines, pyrrolidinylpyridines and piperidinylpyridines; wherein the substituents are chosen independently from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂, -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH and 5-tetrazolyl;
R³ is chosen from -CH₃, -CH₂CH₃, -CF₃, -CHF₂ and -CH₂F;
R⁵ is chosen from H, -F, -OH, -CH₃, -OCH₃, -CF₃, -CN, -NH₂ and -C≡CH;
R² is benzoxadiazole, benzodioxole, 2,2-difluorobenzodioxole, benzoxadiazole, benzodioxan, benzimidazole, oxadiazole, pyrazole, pyridine and pyridine N-oxide;
R⁹ is chosen from H, (C₁-C₆)alkyl, halo(C₁-C₆)alkyl, (C₁-C₆)alkoxycarbonyl, carboxy(C₁-C₆)alkyl, (C₁-C₆)alkoxycarboxy(C₁-C₆)alkyl;
R¹⁰ is H, (C₁-C₆)alkyl, or taken together, or
R⁹ and R¹⁰ together form a heterocycle optionally substituted with (C₁-C₆)alkyl;
p is 0 or 1,
q is 0, 1 or 2,
R¹¹ is linear (C₁-C₆)alkyl,
R¹² is H or (C₁-C₆)alkyl; or two adjacent substituents together form an optionally substituted fused heterocyclic ring,
wherein the term "alkyl" is intended to include linear, branched, or cyclic hydrocarbon structures and combinations thereof, and
with the proviso that when R³ is methyl, R² is phenyl and R^{1a} is hydroxyphenyl then M cannot be -C(=O)-.

9. A compound or salt according to claim 1 of formula 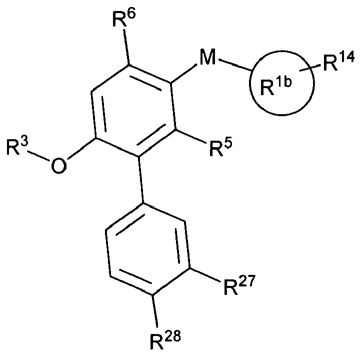 wherein
R^{1b} is phenyl, five-membered heteroaryl, six-membered heteroaryl, 4-7 membered non-aryl heterocycle or fused bicycle;
R¹⁴ is chosen from -COOH, -OH, -COOCH₃, -COOEt, -CH₂COOH, -CH₂COOEt, -CH₂NHC(=O)OEt, -CH₂NHC(=O)C(=O)NH₂, -OCH(CH₃)COOH, -SCH₂COOH, -NHCH₂COOH, -N(CH₃)CH₂COOH, -NHSO₂NH₂, -NHC(=O)CH₂CH₂COOH, -NHC(=O)NHCH₂COOH, -NHC(=O)NHCH₂COOEt, -NHC(=O)NH(CH₂)₃COOEt, -NHC(=O)NH(CH₂)₂COOEt, -NHC(=O)NH(CH₂)₂COOH, -NHC(=O)NH(CH₂)₃COOH, 5-tetrazolyl and monocyclic heterocycle substituted with any of the foregoing;
R²⁷ together with R²⁸ forms a five-membered heterocyclic ring,
or a compound or salt according to claim 1 of formula 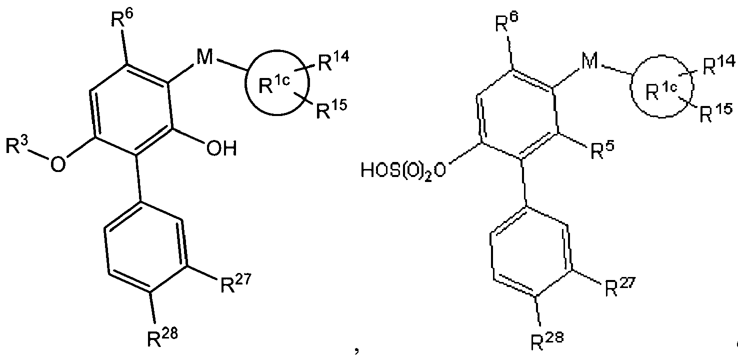 or 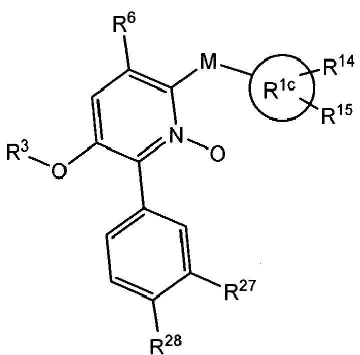 wherein
R^{1c} is phenyl, five-membered heteroaryl, six-membered heteroaryl, 4-7 membered non-aryl heterocycle or fused bicycle;
R¹⁴ is chosen from H, -CH₂NHC(=O)NH₂, -NHC(=O)NH₂, -NHC(=O)NHEt, -CH₃, -CH₂CF₃, -CH₂NHC(=O)CH₃, -NHCH₃, -NHEt, -NH(tBoc), -CHO, -NHC(=O)NHCH₂CH₂Cl, -NHSO₂NH₂, -NHEt, -N(CH₃)₂, -NH₂, -COOH, -C(=O)NH₂, -CH₂C(=O)NH₂, -CH₂COOH, -CH₂COOEt, -CN, -OCH₃, -OC(=O)NH₂, -NH(CH₃)C(=O)NH₂, halogen, -CH₂NHC(=O)OEt, -NHSO₂CH₃ -N(SO₂CH₃)₂ -NHC(=O)OCH₃, -OH, -CH₂NHC(=O)N(CH₃)₂, -CH₂NH₂, -CH₂OH, -CH₂CH₂OH, -SO₂NH₂, -NHC(=O)NHCH₂COOH, -CH₂NHCHO, -NHC(=O)NHCH₂COOEt, -COOCH₃,, -COOEt, -NHC(=O)NH(CH₂)₃COOEt, -NHC(=O)NH(CH₂)₂COOEt, -OCH(CH₃)COOH, -SCH₂COOH, -NH(Et)C(=O)OEt, -NHC(=O)NH(CH₂)₂COOH, -CH₂NHSO₂CH₃ -OEt, -NHC(=O)CH₂N(CH₃)₂, -NHC(=O)NH(CH₂)₃COOH, -NHC(=O)CH₂NH₂, -NHC(=O)CH₂CH₂NH₂, -NHC(=O)CH₂NH(tBoc), -OCH₂CH₂N(CH₃)₂, -OCH₂CH₂OCH₃, 3'-nitro-6-methoxybiphenyl-3-ylmethyl, tetrahydroimidazol-2-on-1-yl, 3-methyltetrahydroimidazol-2-one-1-yl, pyrazol- 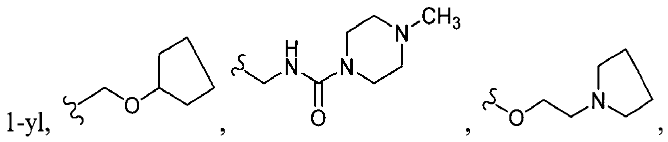 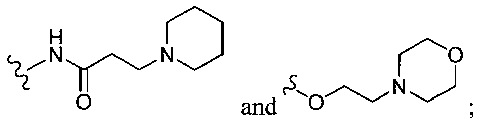
R¹⁵ is chosen from H, NO₂, OH, NH₂, and -NHSO₂NH₂; or
R¹⁵ together with R¹⁴ forms methylene dioxy;
R²⁷ together with R²⁸ forms a five-membered heterocyclic ring.

10. A compound or salt according to claim 9 wherein R²⁷ and R²⁸ represent a fused heterocycle at 3- and 4- positions so that the residue formed from R²⁷ and R²⁸ together with the phenyl to which they are attached is chosen from: 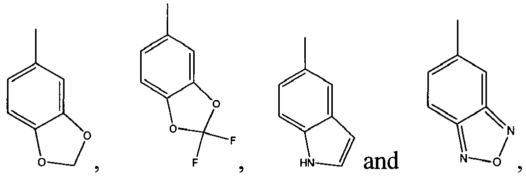 or wherein R²⁷ is chosen from halogen, nitro, acetyl, hydroxyethyl, amino, methylthio, trifluoromethyl, methoxymethyl, methoxycarbonyl, trifluoromethoxy, cyano and 1,3,4-thiadiazol-2-yl, or taken together R⁷ and R⁸ are methylenedioxy or difluoromethylenedioxy.

11. A compound or salt according to claim 10 wherein R²⁷ is chosen from halogen, nitro, acetyl, hydroxyethyl, amino, methylthio, trifluoromethyl, methoxymethyl, methoxycarbonyl, trifluoromethoxy, cyano and 1,3,4-thiadiazol-2-yl, or taken together R⁷ and R⁸ are methylenedioxy or difluoromethylenedioxy and wherein R^{1b} or R^{1c} is chosen from a benzene ring, a triazole, a pyridine or pyridine-N-oxide, a pyrazole, a tetrahydrothiophene, an imidazole, a pyrimidine, a thiadiazole, and an imidazopyridine.

12. A compound or salt according to any of claims 1 or 2 wherein R⁵ is fluoro, H, CN or OH or wherein R³ is methyl or fluoromethyl.

13. A compound or salt according to claim 1 of formula: 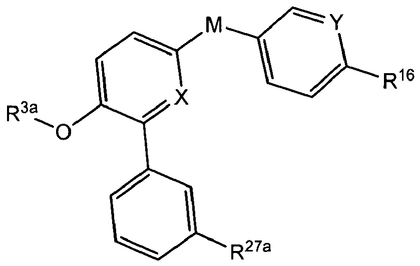 wherein
R^{3a} is methyl, fluorinated methyl or HSO₃;
Y is CH, CF or N→O;
R^{27a} is chosen from
R¹⁶ is chosen from -NR¹⁷C(=O)NR¹⁸R¹⁹ and 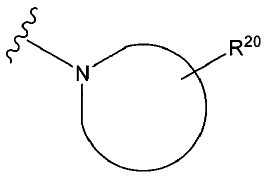 wherein 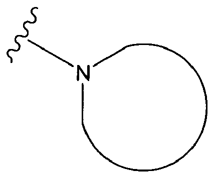 is a 4-7 membered ring heterocycle attached through its nitrogen;
R¹⁷, and R¹⁸ are independently chosen from H, (C₁-C₆)alkyl and halo(C₁-C₆)alkyl;
R¹⁹ is chosen from H, (C₁-C₆)alkyl, halo(C₁-C₆)alkyl, -[(C₁-C₆)alkyl]COOH, and -[(C₁-C₆)alkyl]COO(C₁-C₆)alkyl; and
R²⁰ is chosen from a carboxylic acid, a carboxamide, a carboxylic ester, a primary, secondary or tertiary alcohol and a primary, secondary or tertiary amine.

14. Compound or salt according to claim 1 wherein the compound or salt is selected from the group listed in any one of Tables 1 to 4.

15. A pharmaceutical composition comprising
(a) a pharmaceutically acceptable carrier;
(b) a compound or pharmaceutically acceptable salt according to any of claims 1 or 2; and optionally
(c) a second agent chosen from cholinesterase inhibitors, NMDA antagonists, calpain inhibitors and antioxidants, in particular wherein said second agent is chosen from tacrine, huperzine, donepezil, lanicemine, remacemide, neramexane, memantine, vitamin E and coenzyme Q10.

16. Compound or salt of any of claims 1 or 2 for use in a method of treatment, in particular in a method for the treatment, prevention or amelioration of a disorder is chosen from stroke, myocardial infarct, cardiovascular inflammatory conditions, cancer. asthma, COPD, bone loss, bladder inflammation, bladder overactivity or pain arising from bladder inflammation.
